(19) Europäisches Patentamt
European Patent Office
Office européen des brevets

(11) EP 3 426 211 B1

(12) EUROPEAN PATENT SPECIFICATION

(45) Date of publication and mention of the grant of the patent:
**21.04.2021 Bulletin 2021/16**

(21) Application number: **17711548.2**

(22) Date of filing: **09.03.2017**

(51) Int Cl.:
*A61F 13/511* (2006.01) *A61F 13/512* (2006.01)
*A61F 13/514* (2006.01) *A61F 13/15* (2006.01)

(86) International application number:
**PCT/US2017/021483**

(87) International publication number:
**WO 2017/156208 (14.09.2017 Gazette 2017/37)**

# (54) ABSORBENT ARTICLES

SAUGFÄHIGE ARTIKEL

ARTICLES ABSORBANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2016 US 201662305655 P**

(43) Date of publication of application:
**16.01.2019 Bulletin 2019/03**

(73) Proprietor: **The Procter & Gamble Company**
**Cincinnati, OH 45202 (US)**

(72) Inventors:
- **HAMMONS, John, Lee**
**Cincinnati**
**Ohio 45202 (US)**
- **ARORA, Kelyn, Anne**
**Cincinnati**
**Ohio 45202 (US)**
- **MOSS, Stephanie, Niezgoda**
**Cincinnati**
**Ohio 45202 (US)**
- **AVILES, Misael, Omar**
**Cincinnati**
**Ohio 45202 (US)**
- **ISELE, Olaf, Erik Alexander**
**Cincinnati**
**Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**WO-A1-00/74620** **WO-A1-2014/145804**
**US-A1- 2005 245 157** **US-A1- 2006 189 956**
**US-A1- 2007 048 498** **US-A1- 2013 022 784**
**US-A1- 2014 234 575** **US-B1- 7 102 054**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 426 211 B1

## Description

### FIELD OF THE INVENTION

The disclosure herein relates generally to material webs and articles incorporating material webs.

### BACKGROUND OF THE INVENTION

Nonwoven webs have been used in a myriad of disposable absorbent articles over the years. For example, in some particular absorbent articles, e.g. diapers and feminine hygiene pads, nonwovens may be utilized as a topsheet, backsheet, or some other feature of these particular absorbent articles.

Unfortunately, the requirements for absorbent articles may be disparate depending on use. For example, a nonwoven web used as a topsheet for baby diapers may not be suitable for adult incontinence products. Similarly, a nonwoven web suitable as a topsheet for adult incontinence products may not be suitable for feminine hygiene pads.

Additionally, requirements for nonwoven webs in disposable absorbent articles may vary by geography. For example, in one geography an absorbent article with a soft topsheet may be a factor which is foremost in consumer's minds. In another geography, absorbent articles which minimize the amount of rewet may be foremost in consumer's minds. In yet another geography, the speed of acquisition of liquid insults may be foremost in consumer's minds.

It would be beneficial for a material web to address one or more of the above concerns. It would also be beneficial to have a process which facilitated the production of material webs capable of addressing one or more of the above concerns.

### SUMMARY OF THE INVENTION

The invention is defined in the set of appended claims.

Disclosed herein are material webs, which may include spunbond, meltblown and combinations thereof, which can be used in disposable absorbent articles. Some exemplary uses include topsheet, acquisition layer or overwrap for a tampon. The material webs of the present invention, when utilized for example as a topsheet of a feminine hygiene article or other absorbent article, can provide a soft feel to the user and can provide quick acquisition of menstrual and/or urine insults. Other benefits and configurations in these and other disposable absorbent articles are discussed hereinafter.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:

Figure 1 is a schematic representation of a cross section of a material web of the present invention.
Figure 2 is a schematic representation of a process for making a spunmelt nonwoven web of the present invention.
Figures 3A-3C are schematic illustrations of cross sections of bi-component filaments for use with the present invention.
Figure 4A is an illustration of an exemplary straight filament.
Figure 4B is an illustration of an exemplary curled filament.
Figure 5A is a schematic representation of a material web of the present invention shown in plan view.
Figure 5B is a schematic representation of the material web of Figure 5A shown in cross section along line 5B-5B.
Figure 5C is a schematic representation of the material web of Figure 5A shown in cross-section along line 5C-5C.
Figure 5D is a schematic representation of another form of the material web of Figure 5A shown in cross-section.
Figure 5E is a schematic representation of another form of the material web of Figure 5A shown in cross-section.
Figures 6A-6E are schematic representations of tunnel tufts on material webs of the present invention.
Figures 7A-7D are schematic representations of filled tufts on material webs of the present invention.
Figure 8A is a plan view photomicrograph showing one side of a material web having three-dimensional discontinuity formed therein in accordance with the present disclosure.
Figure 8B is a plan view photomicrograph showing the other side of the material web of Figure 8A, with the openings.
Figure 8C is a perspective view of a discontinuity in a two layer material web in accordance with the present disclosure.
Figure 8D is a schematic view of a nested tuft in accordance with the present disclosure.
Figure 9A-9D are schematic representations of corrugations and grooves on material webs of the present invention.
Figures 10-14 are schematic illustrations of disposable absorbent articles comprising a plurality of zones in accordance with the present invention.

Figures 15A-15B are SEM photos of a first plurality of filaments of a first stratum and a second plurality of filaments of a second stratum, respectively.

Figure 15C is an SEM photo of a material web constructed in accordance with the present invention.

Figure 16A is a photo of a material web comprising apertures, wherein the material web is constructed in accordance with the present invention.

Figure 16B is a photo of a nonwoven laminate comprising a hydrophobic first layer and a hydrophilic second layer.

Figure 17A is a photo of a material web comprising tunnel tufts, wherein the material web is constructed in accordance with the present invention.

Figure 17B is a photo of a nonwoven laminate comprising a hydrophobic first layer and a hydrophilic second layer.

Figure 18 is a depiction of a coordinate system for the material webs of the present invention.

Figures 19-32 are photographs of material webs comprising patterned apertures in accordance with the present invention.

Figure 33 shows a plan view of a feminine hygiene pad constructed in accordance with the present disclosure.

Figure 34 shows a plan view of a diaper constructed in accordance with the present disclosure.

Figure 35 shows a cross section of the diaper of Figure 34 taken along lines 35-35.

Figure 36 shows a cross section of the diaper of Figure 35 in an expanded state.

Figure 37 is an isometric view of an exemplary material web with corrugations therein constructed in accordance with the present disclosure.

Figure 38 is an isometric view of an exemplary material web with corrugations therein constructed in accordance with the present disclosure.

Figure 39 is an isometric view of an exemplary material web with corrugations therein constructed in accordance with the present disclosure.

Figure 40 is a cross-sectional view of a material web of material in a three strata configuration in accordance with the present disclosure.

Figure 41 is a perspective view of the web of material of Figure 40 with various portions of nonwoven component strata cut away to show the composition of each nonwoven component stratum in accordance with the present disclosure.

Figure 42 is a cross-sectional view of a material web in a four stratum configuration in accordance with the present disclosure.

Figure 43 is a perspective view of the material web of Figure 42 with various strata of material web cut away to show the composition of each nonwoven stratum in accordance with the present disclosure.

Figure 44 is a schematic representation of a cross section of a material web of the present invention.

## DETAILED DESCRIPTION OF THE INVENTION

As used herein "disposable absorbent article" or "absorbent article" shall be used in reference to articles such as diapers, training pants, diaper pants, refastenable pants, adult incontinence pads, adult incontinence pants, feminine hygiene pads, tampons, and pessary devices. The term "disposable article" shall be used in reference to articles such as facemasks. For ease of discussion, the terms "disposable absorbent article" or "absorbent article" will be used; however, the material webs of the present invention may equally be utilized in facemasks unless otherwise specified.

As used herein "hydrophilic" and "hydrophobic" have meanings well established in the art with respect to the contact angle of a referenced liquid on the surface of a material. Thus, a material having a liquid (water) contact angle of greater than about 90 degrees is considered hydrophobic, and a material having a liquid (water) contact angle of less than about 90 degrees is considered hydrophilic. Compositions which are hydrophobic, will increase the contact angle of water on the surface of a material while compositions which are hydrophilic will decrease the contact angle of water on the surface of a material. Notwithstanding the foregoing, reference to relative hydrophobicity or hydrophilicity between material(s) and/or composition(s) does not imply that the material or composition are hydrophobic or hydrophilic. For example, a composition may be more hydrophobic than a material. In such a case neither the composition nor the material may be hydrophobic; however, the contact angle of water droplets on the composition is greater than that of water droplets on the material. As another example, a composition may be more hydrophilic than a material. In such a case, neither the composition nor the material may be hydrophilic; however, the contact angle with respect to water droplets exhibited by the composition may be less than that exhibited by the material.

As used herein, "spunbond filaments" refers to small diameter filaments which are formed by extruding molten thermoplastic material as filaments from a plurality of fine capillaries of a spinneret with the diameter of the extruded filaments then being rapidly reduced. Spunbond filaments are generally not tacky when they are deposited on a collecting surface. Spunbond filaments are generally continuous and have average diameters (from a sample of at least 10 measurements) larger than 7 microns, and more particularly, between about 8 and 40 microns.

The term "filament" refers to any type of artificial continuous strand produced through a spinning process, a

meltblowing process, a melt fibrillation or film fibrillation process, or an electrospinning production process, or any other suitable process to make filaments. The term "continuous" within the context of filaments are distinguishable from staple length fibers in that staple length fibers are cut to a specific target length. In contrast, "continuous filaments" are not cut to a predetermined length, instead, they can break at random lengths but are usually much longer than staple length fibers.

By "substantially randomly oriented" it is meant that, due to processing conditions of laying down multiple filaments onto a collecting surface (e.g. a moving foraminous belt with vacuum suction underneath) for forming of a nonwoven web, those filaments are touching down and tipping over onto the collecting surface following turbulent, chaotic, random movements so that the direction of a section of a filament can go into any direction on a 360° circle - as laydown direction. The laydown orientation may be more common in the machine direction (MD) than the cross direction (CD), or vice-versa as can be analyzed via a histogram of fiber orientation distribution.

Each of the material webs comprises at least two strata. As used herein, the term "strata" and "stratum" refer to the layered regions which make up a unitary structure, which in the case of the present invention is the material web. The combination of strata of the material web is not an assembly or laminate of preformed layers forming a multilayered structure. Rather the material web of the present invention is constructed by assembling the strata in an integral manner as described herein. In some forms, where adjacent strata are indistinguishable, the adjacent strata may be considered to be a stratum.

The ideas and techniques described herein for forming the material webs of the present invention can be applied to spunbond filaments and/or fine fiber / nanofiber webs with multiple strata; which in turn may be comprised within a spunmelt web. Continuous and discontinuous fiber spinning technologies of molten materials and typically of thermoplastics are commonly referred to as spunmelt technologies. Spunmelt technologies may comprise both the meltblowing process and spunbonding processes. A spunbonding process comprises supplying a molten polymer, which is then extruded under pressure through a large number of orifices in a plate known as a spinneret or die. The resulting continuous fibers are quenched and drawn by any of a number of methods, such as slot draw systems, attenuator guns, or Godet rolls, for example. In the spunlaying or spunbonding process, the continuous fibers are collected as a loose web upon a moving foraminous surface, such as a wire mesh conveyor belt, for example. When more than one spinneret is used in line for forming a multi-strata web, the subsequent nonwoven component strata are collected upon the uppermost surface of the previously formed nonwoven component strata.

As used herein, "fine fibers" and "nanofibers" shall be used synonymously and shall refer to filaments or fibers which have a diameters of less than about 8 microns. For example, meltblown filaments can have a diameter between 2 to 8 microns while other filament making methods can product sub-micron diameter filaments as discussed hereafter.

Methods to produce fine fibers or nanofibers comprise melt fibrillation and electrospinning. Melt fibrillation is a general class of making fibers defined in that one or more polymers are molten and are extruded into many possible configurations (e.g., co-extrusion, homogeneous or bi-component films or filaments) and then fibrillated or fiberized into filaments. Meltblowing is one such specific method (as described herein).

The meltblowing process is related to the spunbonding process for forming a stratum of a nonwoven material, wherein, a molten polymer is extruded under pressure through orifices in a spinneret or a die. High velocity gas impinges upon and attenuates the fibers as they exit the die. The energy of this step is such that the formed fibers are greatly reduced in diameter and are fractured so that micro-fibers of indeterminate length are produced. This differs from the spunbonding process where the continuity of the fibers are generally preserved. Often meltblown nonwoven structures are added to spunbond nonwoven structures to form spunbond, meltblown ("SM") webs or spunbond, meltblown, spunbond ("SMS") webs, which are strong webs with some barrier properties. Coaxial meltblown is known in the art and is considered a form of meltblowing.

Melt film fibrillation is another method that may be used to produce nanofibers, i.e. submicron fibers. A melt film is produced from the melt and then a fluid is used to form fibers from the melt film. Examples of this method comprise U.S. Patent Nos. 6,315,806, 5,183,670, and 4,536,361, to Torobin et al., and U.S. Patent Nos. 6,382,526, 6,520,425, and 6,695,992, to Reneker et al. and assigned to the University of Akron, and U.S. Patent Nos. 8,395,016; 8,487,156; 7,291 300; 7,989,369; and 7,576,019. The process according to Torobin uses one or an array of co-annular nozzles to form a tube of film which is fibrillated by high velocity air flowing inside this annular film. Other melt film fibrillation methods and systems are described in the U.S. Pat. Publ. No. 2008/0093778, to Johnson, et al., published on April 24, 2008, U.S. Pat. No. 7,628,941, to Krause et al., and U.S. Pat. Publ. No. 2009/0295020, to Krause, et al., published on December 3, 2009 and provide uniform and narrow fiber distribution, reduced or minimal fiber defects such as unfiberized polymer melt (generally called "shots"), fly, and dust, for example. These methods and systems further provide uniform nonwoven webs for absorbent hygiene articles.

Electrospinning is another commonly used method of producing sub-micron fibers. In this method, typically, a polymer is dissolved in a solvent and placed in a chamber sealed at one end with a small opening in a necked down portion at the other end. A high voltage potential is then applied between the polymer solution and a collector near the open end of the chamber. The production rates of this process are very slow and fibers are typically produced in small quantities. Another spinning technique for producing sub-micron fibers is solution or flash spinning which utilizes a solvent.

So, in the context of the material webs of the present invention, a first nonwoven stratum are integrally formed with a second nonwoven stratum. However, material webs of the present invention are not limited to nonwovens. Additionally, the material webs of the present invention may comprise a film strata in conjunction with a nonwoven strata described above.

An exemplary web is shown in Figure 1. As shown in Figure 1, material webs 10 of the present invention have a machine direction (MD) (perpendicular to the plane of the sheet showing Figure 1), a cross machine direction (CD), and a Z direction (thickness direction), as is commonly known in the art of web manufacture. As shown, the material web 10 comprises at least a first stratum 20 and a second stratum 30. The material web 10 further comprises a first surface 50 and a second surface 52. As discussed herein, the first stratum 20 and the second stratum 30 are integrally formed. For example, the first stratum 20 and the second stratum 30 may be integrally formed via a spunmelt, melt blowing, or electrospinning processes described herein.

Additionally, in some forms of the present invention, each of the first stratum 20 and the second stratum 30 comprise a plurality of randomly oriented filaments. For example, the first stratum 20 may comprise a first plurality of randomly oriented filaments, and the second stratum 30 may comprise a second plurality of randomly oriented filaments. For ease of visualization, a delineation 54 is shown between the first stratum 20 and second stratum 30; however, as the first stratum 20 and second stratum 30 are integrally formed, as described herein, a delineation between adjacent strata may not be so easily detectable. But, as noted previously, in some forms, the first stratum 20 or the second stratum 30 may comprise a film.

For the material webs 10 of the present invention, the first stratum 20 is different than the second stratum 30. As shown in Figure 1, such a configuration creates a Z-direction characteristic difference that is measurable as disclosed herein. In the creation of a Z-direction characteristic difference, the first stratum 20 may differ from the second stratum 30 in a myriad of ways. Some suitable examples include surface energy, thickness, filament diameter, filament cross-sectional area, filament cross-sectional shape, filament cross sectional configuration with multiple polymers (such as for example "bico"), filament curl, and/or filament composition, softness, coefficient of friction, extensibility and/or color. Each of the foregoing represent a characteristic of the filaments of the strata or of the strata itself. And each is discussed hereafter in additional detail.

Each of these variables can impact the performance attributes of absorbent articles in various ways. For example, acquisition speed, reduction of rewet, creation of barrier properties, better conformance of the product, increase in softness, etc.

Additionally, the material webs 10 of the present invention, may comprise an MD and/or CD characteristic difference that is measurable as disclosed herein. In the creation of the MD and/or CD characteristic differences, the first stratum 20 and/or second stratum 30 may comprise a myriad of features. For example, apertures, bond sites, embossments tunnel tufts, filled tufts, nested tufts, outer tufts, and corrugations can provide an MD and/or CD characteristic difference.

## Material web - Z-direction characteristic differences

The modification of strata characteristics, as noted above, can create Z-direction characteristic differences in the material web 10 which can enhance certain properties of the material webs 10. For example, acquisition time, rewet, permeability, softness, masking, resiliency, and capillarity are some of the properties which can be modified based upon the differences in the first plurality of filaments and the second plurality of filaments. The differences between the first stratum 10 and the second stratum 30 are discussed hereafter along with the benefits in the properties of the material web.

Referring now to Figures 1 and 2, a material web of the present invention may be produced via a spunbond process comprising multiple spinbeams 255, 257. In some forms, the first spinbeam 255 may deposit a first plurality of filaments 261 onto a belt. The second spinbeam 257 may deposit a second plurality of filaments 263 onto the belts over the top of the first plurality of filaments 261. And, as noted previously, the second plurality of filaments may be configured differently than the first plurality of filaments such that the first stratum 20 is different than the second stratum 30.

Forms of the present invention are contemplated where additional spinbeams are provided to provide additional strata with additional filaments. Accordingly, material webs of the present invention comprise a third stratum and may comprise fourth stratum and so on. And, the strata of the material web may be configured such that at least two of the strata are different. Additionally, forms of the present invention are contemplated where processes for the material webs may allow for the inclusion of one or more nanofiber strata, e.g. one or more meltblown strata, one or more melt fibrillation strata, and/or one or more electrospun strata.

Additionally, forms of the present invention are contemplated where the first stratum 20 is created in a first step and subsequently processed. Subsequently, the second stratum 30 may be deposited onto the first stratum 20. For example, the first stratum may be provided to an aperturing process (described herein) and subsequently, the second stratum may be integrally formed on the first stratum via the processes described herein. As another example, the first stratum may comprise a film. The first stratum may be subjected to an aperturing process (described herein) and

subsequently, the second stratum may be integrally formed on the first stratum via the processes described herein. As another example, the first stratum may comprise a nonwoven which is subjected to an aperturing process (described herein), and subsequently, the second stratum which comprises a film is integrally formed on the first stratum, e.g. extruded onto the first nonwoven stratum. And, as yet another example, the first stratum and the second stratum may be integrally formed without any intermediate processing of the first stratum or second stratum.

Surface Energy

One of the ways to create a Z-direction characteristic difference in the material webs of the present invention is to utilize differing surface energies for the first stratum 20 and/or second stratum 30 (and/or of any additional strata). In general, nonwoven strata which have a high surface energy may be considered to be more hydrophilic than nonwoven strata which have a low surface energy. That said, in some forms of the present invention, the first stratum 20 may be more phobic than the second stratum 30. Accordingly, in some forms, the first stratum 20 may have a lower surface energy than the second stratum 30.

The increased phobicity of the first stratum 20 (relative to the second or any other stratum) may be achieved in a variety of ways. For example, the first plurality of filaments may comprise a composition which is more phobic than that of the second plurality of filaments. In one specific example, the first plurality of filaments may comprise polyethylene while the second plurality of filaments comprise polyethylene terephthalate. In general, polyethylene and polypropylene are more phobic than polylactic acid, polyethylene terephthalate and nylon. The first plurality of filaments and/or second plurality of filaments may use any suitable combination of these compositions.

In another example, the first plurality of filaments and/or second plurality of filaments may comprise a melt additive. In one specific example, the first plurality of filaments may comprise a phobic melt additive added directly or as master batch to the polymer melt during spinning of the first plurality of filaments. Such a melt-additive could comprise for example lipid esters or polysiloxanes. For those forms where the additive is melt blended into the filaments, the additive can bloom to the surface of the filaments and create a film covering a portion of the external surface of the filament and/or can create fibrils, flakes, particles, and/or other surface features. In conjunction with the phobic melt additive or independent therefrom, the second plurality of filaments may comprise a philic melt additive. In another example, the first plurality of filaments may comprise a phobic melt additive at a first weight percent while the second plurality of filaments may comprise a phobic melt additive at a second weight percent. The first weight percent may be greater than the second weight percent such that the first stratum 20 is more phobic than the second stratum 30. In yet another example, the first plurality of filaments may comprise a philic melt additive at a first weight percent and the second plurality of filaments may comprise a philic melt additive at a second weight percent. In such forms, the second weight percent may be greater than the first weight percent such that the second stratum 30 is more philic than the first stratum 20. In yet another example, the first plurality of filaments may comprise a first phobic melt additive while the second plurality of filaments comprise a second phobic melt additive. In such forms, the first phobic melt additive may render the first plurality of filaments more phobic than the second plurality of filaments or vice versa. In yet another example, the first plurality of filaments may comprise a first philic melt additive while the second plurality of filaments comprise a second philic melt additive. In such form, the first philic melt additive may render the first plurality of filaments more philic than the second plurality of filaments or vice versa.

For those forms where melt additives are provided to the first plurality of filaments and/or the second plurality of filaments, the melt additive may preferably form between about 0.11 percent by weight to about 20 percent by weight of the first stratum 20 and/or second stratum 30. In some forms, the melt additives are more preferably less than about 15 percent by weight, even more preferably less than about 10 percent by weight, and most preferably less than about 8 percent by weight, specifically including any values within these ranges or any ranges created thereby.

Any suitable phobic melt additive may be utilized. Examples of phobic melt additives include fatty acids and fatty acid derivatives. The fatty acids may originate from vegetable, animal, and/or synthetic sources. Some fatty acids may range from a C8 fatty acid to a C30 fatty acid, or from a C12 fatty acid to a C22 fatty acid. In other forms, a substantially saturated fatty acid may be used, particularly when saturation arises as a result of hydrogenation of fatty acid precursor. Examples of fatty acid derivatives include fatty alcohols, fatty acid esters, and fatty acid amides. Suitable fatty alcohols (R-OH) include those derived from C12-C28 fatty acids.

Suitable fatty acid esters include those fatty acid esters derived from a mixture of C12-C28 fatty acids and short chain (C1-C8, preferably C1-C3) monohydric alcohols preferably from a mixture of C12-C22 saturated fatty acids and short chain (C1-C8, preferably C1-C3) monohydric alcohols.. The hydrophobic melt additive may comprise a mixture of mono, di, and/or tri-fatty acid esters. An example includes fatty acid ester with glycerol as the backbone as illustrated in [1].

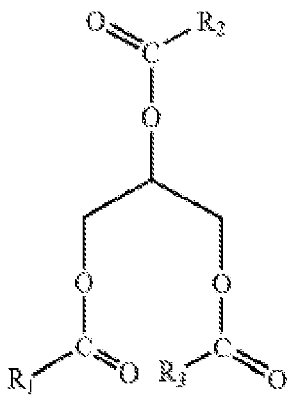
[1]

where R1, R2 , and R3 each is an alkyl ester having carbon atoms ranging from 11 to 29. In some forms, the glycerol derived fatty acid ester has at least one alkyl chain, at least two, or three chains to a glycerol, to form a mono, di, or triglyceride. Suitable examples of triglycerides include glycerol thibehenate, glycerol tristearate, glycerol tripalmitate, and glycerol trimyristate, and mixtures thereof. In the case of triglycerides and diglycerides, the alkyl chains could be the same length, or different length. Example includes a triglyceride with one alkyl C18 chain and two C16 alkyl chain, or two C18 alkyl chains and one C16 chain. Preferred triglycerides include alkyl chains derived from C14-C22 fatty acids.

Suitable fatty acid amides include those derives from a mixture of C12-C28 fatty acids (saturated or unsaturated) and primary or secondary amines. A suitable example of a primary fatty acid amide includes those derived from a fatty acid and ammonia as illustrated in [2].

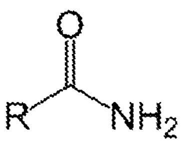
[2]

where R has a number of carbon atoms ranging from 11 to 27. In at least one other form, the fatty acids may range from a C16 fatty acid to a C22 fatty acid. Some suitable examples include erucamide, oleamide and behanamide. Other suitable hydrophobic melt additives include hydrophobic silicones. Additional suitable phobic melt additives are disclosed in U.S. Patent Application Serial No. 14/849630 and U.S. Patent Application Serial No. 14/933028. Another suitable phobic melt additive is available from Techmer PM in Clinton, TN under the trade name PPM17000 High Load Hydrophobic. One specific example of a phobic melt additive is glycerol tristearate. As used herein, glycerol tristearate is defined as a mixture of long-chained triglycerides containing predominately C18 and C16 saturated alkyl chain lengths. Additionally there could be varying degrees of unsaturation and cis to trans unsaturated bond configurations. The alkyl chain lengths could range from about C10 to about C22. The degrees of unsaturation typically will range from 0 to about 3 double bonds per alkyl chain. The ratio of cis to trans unsaturated bond configurations can range from about 1:100 to about 100:1. Other suitable examples for use with polypropylene and/or polyethylene, a triglyceride which contains either stearic acid or palmic acid or both as the fatty acid components, or a mixture of such triglycerides. Other suitable hydrophobic melt additives may comprise erucamide or polysiloxanes.

Any suitable philic additive can be used. Some suitable examples include those available from Techmer PM, Clinton, TN sold under the trade name of Techmer PPM15560; TPM12713, PPM19913, PPM 19441, PPM19914, , PPM112221 (for polypropylene), PM19668, PM112222 (for polyethylene). Additional examples are available from Polyvel Inc. located in Hammonton, NJ, sold under the trade name of Polyvel VW351 PP Wetting Agent (for polypropylene); from Goulston Technologies Inc. located in Monroe, NC sold under the trade name Hydrosorb 1001; as well as those philic additives disclosed in US Patent Application Publication No. 2012/0077886 and U.S. Patent Nos. 5,969,026 and U.S. Patent No. 4,578,414.

Nucleating agents may be included along with the melt additives. Nucleating agents can help to drive more or faster blooming of either a philic or phobic melt additive. Thus it would create a characteristic difference in the Z-Direction even when the same phobic or philic melt-additive is used for all of the strata: the nucleating agent when added to one or less than all of the strata will produce a more intensive philic or phobic effect or contact angle effect (depending on the type of additive in those strata) than the stratum or strata with the same philic or phobic melt-additive but that doesn't (or don't) contain the nucleating agent. Suitable nucleating agents may be comprised of a nonitol, a trisamide and/or a

sorbitol-based nucleating agent. Specific but non-limiting examples are: organic nucleation agents such as Millad NX 8000 or (in its new trade name) NX UltraClear GP110B from the Milliken company. An example of an effective inorganic nucleating agent is $CaCO_3$, or other and especially nano-clay or nano-scale mineral molecules.

For those forms where the first plurality of filaments comprise a hydrophobic melt additive, the material web may be incorporated into a disposable absorbent article as a topsheet or overwrap in the case of a tampon. While conventional wisdom would typically advise against a hydrophobic topsheet, material webs of the present invention may comprise apertures which allow for rapid acquisition of liquid insults. In such forms, hydrophobic topsheets can provide a clean dry surface against a wearer’s skin. Additionally, the hydrophobic treatment in the first plurality of filaments may reduce liquid rewet. Examples of the material webs of the present invention comprising hydrophobic and hydrophilic melt additives are provided in the "EXAMPLES" section of this specification.

And, while conventional wisdom may promote post-filament-production enhancement of hydrophobicity / hydrophilicity, e.g. topical application, applications of such compositions may be cause additional strife. For example, many topically applied treatments can migrate to other structures within an absorbent article. Or, for the material webs of the present invention, post- - production treatment may migrate from the first stratum 20 to the second stratum 30 or vice versa during application. Such migration could disturb the desired surface energy difference between the first stratum 20 and the second stratum 30.

However, forms are contemplated where the first stratum 20 is produced and subsequently treated with a surface energy modifying composition. Subsequently, the second stratum 30 is formed onto the first stratum 20.

Filament Diameter or Cross-sectional Area

Another way to create Z-direction characteristic difference in the material webs of the present invention is to utilize differing filament sizes in the first plurality of filaments versus the second plurality of filaments in the first stratum 20 and second stratum 30, respectively. The term "filament size", refers to the cross-sectional dimension, diameter or area of the filament; for a circular-round cross-sectional shape the cross-sectional dimension is the diameter and the area is a circle, but there can be more complicated cross-sectional shapes. The first stratum 20 and second stratum 30 may comprise filament size differences in addition to or independent from the surface energy differences discussed above.

In some forms, the first plurality of filaments may comprise a first size while the second plurality of filaments comprise a second size. The first size may be different than the second size. In some forms, the first size may be greater than the second size. The first plurality of filaments and the second plurality of filaments may comprise any suitable size. In some forms, the first plurality of filaments and the second plurality of filaments can have an average size in the range of about 8 microns to about 40 microns, or a filament titer in the range from 0.5 to 10 denier, specifically including all values within these ranges and any ranges created thereby.

Generally, nonwoven webs with larger filaments increase permeability. The increase in permeability can provide quicker fluid penetration or transfer or acquisition times which can be a desirable quality. However, the increase in permeability may unfortunately increase the potential for liquid rewet.

In contrast, nonwoven webs with smaller filaments typically have lower permeability but higher capillarity. The lower permeability can mean slower fluid acquisition times; however, the higher capillarity can reduce the likelihood of rewet which can be desirable.

For those forms of the present invention where the material web 10 is utilized as a topsheet, a larger filament size in the first stratum 20 can mean higher permeability - quicker fluid acquisition and lower capillarity. And, a smaller filament size in the second stratum 30 can mean lower permeability but higher capillarity - reducing the likelihood of rewet. Where the material webs of the present invention comprise additional strata, the additional strata can further enhance the capillarity / permeability of the material web. For example, the third stratum may comprise filament sizes which are smaller than the second stratum and the fourth stratum may comprise smaller filament sizes than the third stratum. Accordingly, each of the subsequent strata may have increased capillarity. In such forms, a capillary gradient can be configured where the capillarity increases for those strata nearer the absorbent core.

Filament Cross Sectional Shape

Still another way to create Z-direction characteristic difference in the material webs of the present invention is to utilize differing filament cross-sectional shapes. The first plurality of filaments and/or second plurality of filaments may comprise any suitable cross-sectional shape. In some forms, the first plurality of filaments may comprise a shape that is different than that of the second plurality of filaments. Still in other forms, the first plurality of filaments may comprise a plurality of shapes and at least one of the shapes of the first plurality of filaments is different than the shape of the second plurality of filaments. Similarly, the second plurality of filaments may comprise a plurality of shapes.

The first plurality of filaments and/or the second plurality of filaments may comprise a non-round filaments. (Round meaning typically circular and solid without cavities or hollow sections.) As used herein, the term "non-round

filaments" describes filaments having a non-round cross-section, and includes "shaped filaments" and "capillary channel filaments." Such filaments can be solid or hollow, and they can be tri-lobal, delta-shaped, and can be filaments having capillary channels on their outer surfaces. The capillary channels can be of various cross-sectional shapes such as "U-shaped", "H-shaped", "C-shaped" and "V-shaped". One practical capillary channel filament is T-401, designated as 4DG filament available from Filament Innovation Technologies, Johnson City, TN. T-401 filament is a polyethylene terephthalate (PET polyester). Other suitable shapes include round, round hollow, or ribbon.

The cross sectional shape of the first plurality of filaments and/or second plurality of filaments may be varied in conjunction with or independently of the surface energy and filament diameter / cross-sectional area differences discussed above.

Generally, non-round filaments have increased capillarity / wicking potential than their round filament counterparts due to their higher surface area. That said, non-round filaments may not readily give up the fluid which is disposed thereon. As such, non-round filaments may not be beneficial for the purposes of masking / rewet in a wearer-facing surface of a material web. Instead, the non-round filaments may perform much better if provided subjacent to the wearer-facing surface of an absorbent article. Additionally, non-round filaments have greater wicking ability, higher capillary suction and provide more resiliency than their round filaments counterparts. Each of these traits may provide more benefit if provided in a stratum which is closer to an absorbent core than a stratum which comprises a portion of the wearer-facing surface of an absorbent article.

In some specific forms, the first stratum 20 may comprise round filaments while the second stratum 30 comprises non-round filaments. Forms of the present invention are contemplated where the first stratum 30 and/or second stratum 40 have mixed filament shapes. For example, the first stratum 20 may comprise a higher percentage of round filaments than does the second stratum 30. And for those forms of the present invention which comprise additional strata, the third stratum may comprise non-round filaments and/or may comprise a lower percentage of round filaments than the second stratum 30. And, if a fourth stratum is provided, the fourth stratum may similarly comprise non-round filaments and/or may comprise a lower percentage of round filaments than the second stratum 30.

Additional forms are contemplated where the first stratum 20 and the second stratum 30 each comprise round filaments. The third stratum may comprise non-round filaments. In other forms, the third stratum may comprise round filaments and a fourth stratum may comprise non-round filaments.

## Filament Cross-Sectional Configuration

Still another way to create Z-direction characteristic differences in the material webs of the present invention is to utilize differing filament cross-sectional configurations. For example, the filaments of the first stratum and/or the second stratum can be mono-component, bi-component, and/or bi-constituent. As used herein, the term "mono-component" filament refers to a filament formed from one extruder using one or more polymers. This is not meant to exclude filaments formed from one polymer to which small amounts of additives have been added for coloration, antistatic properties, lubrication, hydrophilicity, etc.

As used herein, the term "bi-component filaments" refers to filaments which have been formed from at least two different polymers extruded from separate extruders but spun together to form one filament. Bi-component filaments are also sometimes referred to as conjugate filaments or multi-component filaments. The polymers are arranged in substantially constantly positioned distinct zones across the cross-section of the bi-component filaments and extend continuously along the length of the bi-component filaments. The configuration of such a bi-component filament may be, for example, a sheath/core arrangement wherein one polymer is surrounded by another, or may be a side-by-side arrangement, a pie arrangement, or an "islands-in-the-sea" arrangement. Some suitable examples of bi-component filament configurations are shown in Figures 3A-3C. For example, filaments of the material webs of the present invention may comprise filaments having a cross section 300 which comprises a first component 300A and a second component 300B arranged in a side by side configuration. As shown, a delineation 302 between the first component 300A and 300B may be easily discernable depending on the compositions of the first component 300A and the second component 300B. In some forms, the first component 300A and the second component 300B may be present in a filament in about equal proportion, e.g. 50/50. However, in some forms, the ratio of the first component 300A to the second component 300B may vary. As such, the delineation 302 may be offset more proximal to one side of the filament. Ratios of the compositions are discussed hereafter.

As another example, material webs of the present invention may comprise bi-component filaments having a cross-section 310 which comprises a first component 310A and a second component 310B in an eccentric sheath-core configuration. And with this configuration, the core, i.e. second component 310B may be tangent to an edge of the filament as shown in Figure 3B or may be offset from the edge of the filament. In one specific example of the sheath-core configuration, the first component 310A may be concentric with the second component 310B.

Another example of a bi-component filament cross-section that may be utilized in the present invention is shown with regard to Figure 3C. As shown, filaments having a tri-lobal cross-section 320 may be utilized. The tri-lobal cross

section 320 comprises a first component 320A and a second component 320B, where the second component 320B is one of the lobes of the tri-lobal cross section. As shown the first component 320A comprises about one-third of the filament cross section 320. In some forms, the delineation 302 may be shifted where the first composition comprises more or less of the cross section 320.

Similar configurations are contemplated with all of the potential filament cross-sections discussed herein. Namely, the bi-component filament cross-sections may comprise a first component and a second component in any of the cross-sectional shapes discussed herein. And, in some forms, depending on the compositions utilized, a third component, fourth component, etc. may be provided for multi-component filaments.

Bi-component filaments may comprise two different resins, e.g. a first resin and a second resin. The resins may have different polymer compositions, melt flow rates, molecular weights, branching, viscosity, crystallinity, rate of crystallization, and/or molecular weight distributions. Ratios of the two different polymers may be about 50/50, preferably about 60/40, more preferably about 70/30, or most preferably about 80/20, or any ratio within these ratios. The ratio may be selected to control the amount of curl, strength of the nonwoven strata, softness, bonding or the like.

As used herein, the term "bi-constituent filaments" refers to filaments which have been formed from at least two polymers extruded from the same extruder as a blend. Bi-constituent filaments do not have the various polymer components arranged in relatively constantly positioned distinct zones across the cross-sectional area of the filament and the various polymers are usually not continuous along the entire length of the filament, instead usually forming fibrils which start and end at random. Bi-constituent filaments are sometimes also referred to as multi-constituent filaments. In one specific example, a bi-component filament may comprise a multi-constituent components.

Further details regarding bi-component or multi-component filaments and methods of making the same may be found in U.S. Patent Application Publ. No. 2009/0104831, published on April 23, 2009, U.S. Pat No. 8,226,625, issued on July 24, 2012, U.S. Pat. No. 8,231,595, issued on July 31, 2012, U.S. Pat. No. 8,388,594, issued on March 5, 2013, and U.S. Pat. No. 8,226,626, issued on July 24, 2012.

In some forms, the first plurality of filaments may be mono-component while the second plurality of filaments are bi-component or vice-versa. In some forms, the first plurality of filaments may be bi-component while the second plurality of filaments are multi-component having at least three components or vice versa. Still in other forms, the first plurality of filament may be mono-component while the second plurality of filaments are multi-component having at least three components or vice versa.

The material webs of the present invention may utilize the filament cross-sectional configuration variation independently or in conjunction with the surface energy, filament size, and/or filament cross sectional shape variations discussed heretofore. And, for those forms comprising third stratum and, in some forms, fourth stratum, the filament cross-sectional configuration between at least two strata may be different.

## Filament Curl

Still another way to create Z-direction characteristic differences in the material webs of the present invention is to utilize curled filaments in the first stratum 20 and/or second stratum 30. In some forms of the present invention, the first stratum 20 and/or the second stratum 30 may comprise curly filaments. For example, the second plurality of continuous filaments may comprise non curled filaments - straight filaments while the first plurality of continuous filaments are curled. Examples of a straight filament and a curled filament are shown in Figures 4A and 4B, respectively. In some forms, the first plurality of filaments and the second plurality of filaments may each comprise curled filaments. In such forms, the first plurality of filaments may comprise more curl than the second plurality of filaments.

As used herein, "curled filament" refers to bi-component filaments which may be configured in a side-by-side, core-eccentric sheath or other suitable configuration. The selection of suitable resin combinations and bi-component filament configuration can lead to a helical crimp or curl generated in the filament. The curl may occur spontaneously during the spinning or laydown process, or on its own after web formation. In some forms, a nonwoven web may require an additional step (e.g. heating or mechanical deformation) to induce the filaments to curl. Some exemplary suitable resin combinations for achieving curled filaments are discussed herein.

The incorporation of a curled filaments into the first stratum 20 and/or second stratum 30 is believed to provide advantages over conventional material webs particularly when used in the disposable absorbent article context. For example, where the first plurality of filaments and/or the second plurality of filaments are curled, higher permeability and/or loft may be achieved versus conventional nonwoven webs which do not include curled filaments. And, nonwoven webs comprising curled filaments are typically perceived as softer by users.

Additionally, material webs comprising curled filaments may facilitate some additional processing. One example includes mechanical processes which manipulate material webs creating three dimensional or apertured structures. For example, filament materials that are not extensible can break, stretch, thin, or tear when subjected to such mechanical processes. However, where curled filaments are utilized, the need for extensible filament materials is assuaged to some extent. During processing of curled filaments, rather than breaking, stretching, and/or thinning, the curled filaments tend

to uncurl. As such, filament materials which would ordinarily not be suited for such mechanical processing, may be suitable if configured as curled filaments. And, material webs comprising curled filaments generally exhibit better elastic recovery from mechanical processing than other material webs. As a specific example, polypropylene and poly-lactic acid based filaments would typically not withstand the mechanical processing needed for the creation of three dimensional or aperture structures on a nonwoven web; however, when configured as a curled filament, such filaments may withstand such mechanical processing.

Still another benefit of utilizing curled filaments in material webs is with regard to tensile elongation. Some material webs utilizing curled filaments may comprise better tensile elongation than conventional nonwoven webs. In one specific example, material webs comprising curled filaments comprising polypropylene / polypropylene bi-component filaments may exhibit a higher tensile elongation than a conventional nonwoven web comprising filaments comprising polypropylene mono-component filaments.

Yet another benefit of the curled filaments of the present invention is with regard to tensile strength ratio between the MD and CD. Material webs of the present invention utilizing continuous curled filaments typically exhibit a tensile strength ratio between the MD and CD that is generally more balanced than the tensile strength ratio between the MD and CD for carded curled fiber material webs. In general, curled fiber carded material webs have a much higher tensile strength in the MD as the fibers are typically combed to be aligned in the MD direction.

Yet another benefit to the utilization of curled filaments in the material webs of the present invention is with regard to bond strength. In some forms, particularly where the filaments comprise bi-component polypropylene / polypropylene, better bond strength can be achieved which makes the material web more abrasion resistant.

Even still, more benefits of the utilization of curled filaments in the material webs of the present invention include compatibility with like chemistries. For example, curled filaments which are bi-component comprising polypropylene / polypropylene may be thermally joined (bonded) to subjacent materials in a disposable absorbent article which are polypropylene based. Also, the cost associated with polypropylene / polypropylene filaments can be less than the cost associated with other bi-component filaments. And, polypropylene / polypropylene filaments or filaments comprising two different polyesters may be recyclable versus bi-component filaments comprising polyethylene / polypropylene.

The material webs of the present invention may utilize curled filaments in the first stratum 20 and/or second stratum 30 to create Z-direction characteristic differences in the material web. The utilization of curled filaments in the first stratum 20 and/or second stratum 30 may be in conjunction with the surface energy variations, filament size variations, filament cross-sectional shape variations, and/or filament cross-sectional configurations or independent of the foregoing. And, for those forms comprising third stratum and, in some forms, fourth stratum, the third stratum and/or fourth stratum may comprise curled filaments or may comprise any other filament described herein.

Strata Composition

Still another way to create Z-direction characteristic difference in the material webs of the present invention is via the utilization of varying strata compositions. The first plurality of filaments and the second plurality of filaments may comprise any suitable composition. Some suitable thermoplastic polymers include polymers that melt and then, upon cooling, crystallize or harden, but can be re-melted upon further heating. Suitable thermoplastic polymers used herein can have a melting temperature (also referred to as solidification temperature) from about 60°C to about 300°C, from about 80°C to about 250°C, or from 100°C to 215°C. And, the molecular weight of the thermoplastic polymer should be sufficiently high to enable entanglement between polymer molecules and yet low enough to be melt spinnable.

The thermoplastic polymers can be derived from any suitable material including renewable resources (including bio-based, agricultural and recycled materials), fossil minerals and oils, and/or biodegradable materials. One suitable example of a thermoplastic polymer derived from renewable resources is SHA7260 High Density Polyethylene from Braskem in Philadelphia, PA.

Other suitable examples of thermoplastic polymers include polyolefins, polyesters, polyamides, copolymers thereof, and combinations thereof. Some exemplary polyolefins include polyethylene or copolymers thereof, including low density, high density, linear low density, or ultra-low density polyethylenes such that the polyethylene density ranges between 0.90 grams per cubic centimeter to 0.97 grams per cubic centimeter, between 0.92 and 0.95 grams per cubic centimeter or any values within these ranges or any ranges within these values. The density of the polyethylene may be determined by the amount and type of branching and depends on the polymerization technology and co-monomer type. Polypropylene and/or polypropylene copolymers, including atactic polypropylene; isotactic polypropylene, syndiotactic polypropylene, and combination thereof, "hereafter propylene polymers" can also be used. Polypropylene copolymers, especially ethylene can be used to lower the melting temperature and improve properties. These polypropylene polymers can be produced using metallocene and Ziegler-Natta catalyst systems. These polypropylene and polyethylene compositions can be combined together to optimize end-use properties. Polybutylene is also a useful polyolefin and may be used in some embodiments. Other suitable polymers include polyamides or copolymers thereof, such as Nylon 6, Nylon 11, Nylon 12, Nylon 46, Nylon 66; polyesters or copolymers thereof, such as maleic anhydride polypropylene

copolymer, polyethylene terephthalate; olefin carboxylic acid copolymers such as ethylene/acrylic acid copolymer, ethylene/maleic acid copolymer, ethylene/methacrylic acid copolymer, ethylene/vinyl acetate copolymers or combinations thereof; poly-lactic acid; polyacrylates, polymethacrylates, and their copolymers such as poly(methyl methacrylates).

Non-limiting examples of suitable commercially available polypropylene or polypropylene copolymers include Basell Prof ax PH-835 (a 35 melt flow rate Ziegler-Natta isotactic polypropylene from Lyondell-Basell), Basell Metocene MF-650W (a 500 melt flow rate metallocene isotactic polypropylene from Lyondell-Basell), Moplen , HP2833, HP462R and S, HP551R, HP552N, HP552R, HP553R, HP561R, HP563S, HP567P, HP568S, RP3231, Polybond 3200 (a 250 melt flow rate maleic anhydride polypropylene copolymer from Crompton), Exxon Achieve 3854 (a 25 melt flow rate metallocene isotactic polypropylene from Exxon-Mobil Chemical), Mosten NB425 (a 25 melt flow rate Ziegler-Natta isotactic polypropylene from Unipetrol), Danimer 27510 (a polyhydroxyalkanoate polypropylene from Danimer Scientific LLC), , , Achieve 3155 (a 35 melt flow rate Ziegler-Natta isotactic polypropylene from Exxon Mobil),

The thermoplastic polymer component can be a single polymer species as described above or a blend of two or more thermoplastic polymers as described above, e.g. two different polypropylene resins. As an example, the constituent filaments of the first stratum can be comprised of polymers such as polypropylene and blends of polypropylene and polyethylene. The material webs may comprise filaments selected from polypropylene, polypropylene / polyethylene blends, and polyethylene / polyethylene terephthalate blends. In some forms, the material webs may comprise filaments selected from cellulose rayon, cotton, other hydrophilic filament materials, or combinations thereof. The filaments can also comprise a super absorbent material such as polyacrylate or any combination of suitable materials.

In some forms, the thermoplastic polymer can be selected from the group consisting of polypropylene, polyethylene, polypropylene co-polymer, polyethylene co-polymer, polyethylene terephthalate, polybutylene terephthalate, polylactic acid, polyhydroxyalkanoates, polyamide-6, polyamide-6,6, and combinations thereof. The polymer can be polypropylene based, polyethylene based, polyhydroxyalkanoate based polymer systems, copolymers and combinations thereof.

Biodegradable thermoplastic polymers also are contemplated for use herein. Biodegradable materials are susceptible to being assimilated by microorganisms, such as molds, fungi, and bacteria when the biodegradable material is buried in the ground or otherwise contacts the microorganisms (including contact under environmental conditions conducive to the growth of the microorganisms). Suitable biodegradable polymers also include those biodegradable materials which are environmentally-degradable using aerobic or anaerobic digestion procedures, or by virtue of being exposed to environmental elements such as sunlight, rain, moisture, wind, temperature, and the like. The biodegradable thermoplastic polymers can be used individually or as a combination of biodegradable or non-biodegradable polymers. Biodegradable polymers include polyesters containing aliphatic components. Among the polyesters are ester polycondensates containing aliphatic constituents and poly(hydroxycarboxylic) acid. The ester polycondensates include diacids/diol aliphatic polyesters such as polybutylene succinate, polybutylene succinate co-adipate, aliphatic/aromatic polyesters such as terpolymers made of butylenes diol, adipic acid and terephthalic acid. The poly(hydroxycarboxylic) acids include lactic acid based homopolymers and copolymers, polyhydroxybutyrate (PHB), or other polyhydroxyalkanoate homopolymers and copolymers. Such polyhydroxyalkanoates include copolymers of PHB with higher chain length monomers, such as $C_6$-$C_{12}$, and higher, polyhydroxyalkanaotes, such as those disclosed in U.S. Patent Nos. RE 36,548 and 5,990,271.

An example of a suitable commercially available polylactic acid is NATUREWORKS from Cargill Dow™ sold under the trade names 6202D, 6100D, 6252D and 6752D and 6302D and LACEA from Mitsui Chemical. An example of a suitable commercially available diacid/diol aliphatic polyester is the polybutylene succinate/adipate copolymers sold as BIONOLLE 1000 and BIONOLLE 3000 from the Showa High Polymer Company, Ltd. (Tokyo, Japan). An example of a suitable commercially available aliphatic/aromatic copolyester is the poly(tetramethylene adipate-co-terephthalate) sold as EASTAR BIO Copolyester from Eastman Chemical or ECOFLEX from BASF.

Polypropylene can have a melt flow index of greater than 5 g/10 min, as measured by ASTM D-1238, used for measuring polypropylene. Other contemplated melt flow indices for polypropylene include greater than 10 g/10 min, greater than 20 g/10 min, or about 5 g/10 min to about 50 g/10 min.

In some forms, the first plurality of filaments and/or the second plurality of filaments may comprise elastomeric filaments. Elastic or elastomeric filaments can be stretched at least about 50% and return to within 10% of their original dimension. In some forms, the first plurality of filaments can be comprised of polymers such as polypropylene and blends of polypropylene and polyethylene. In some embodiments, the second plurality of filaments can be comprised of polymers such as polypropylene, polypropylene / polyethylene blends, and polyethylene / polyethylene terephthalate blends. Some suitable examples of elastomers suitable for creating filaments are sold under the trade name Vistamaxx™ 2330, 6202 from Exxon™, and 7050; G1643, MD6705, DM1648 from Kraton™; Elastollan™ B 95 A 11N000, 2280A, EB 60D11 from BASF™; and Infuse™ 9817 and 9900 from Dow™.

Some specific examples of compositions for curled filaments which can be used in the material webs of the present invention include polyethylene / polypropylene side-by-side bi-component filaments. Another example, is a polypropylene / polyethylene bi-component filament where the polyethylene is configured as a sheath and the polypropylene is configured as a core within the sheath. Still another example, is a polypropylene / polypropylene bi-component filament where two different propylene polymers are configured in a side-by-side configuration. Still another example, is polypropylene / poly-lactic acid bi-component filament. Still another example is polyethylene / poly-lactic acid bi-component filament. For the bi-component filaments of polyethylene / poly-lactic acid, such filaments may be produced from renewable resources. For example, both the polyethylene and polylactic acid may be bio sourced.

In some forms, a composition of the first plurality of filaments may be different than a composition of the second plurality of filaments. For those forms comprising additional strata, e.g. third stratum and fourth stratum, the additional strata may comprise the composition of the first stratum 20 or the second stratum 30. In some forms however, the third stratum and fourth stratum may comprise filament compositions which are different from the first stratum and/or the second stratum.

Forms of the present invention are contemplated where the first plurality of filaments and/or the second plurality of filaments comprise agents in addition to their constituent chemistry. For example, suitable additives include additives for coloration, antistatic properties, coefficient of friction, lubrication, hydrophilicity, and the like and combinations thereof. These additives, for example titanium dioxide for coloration, are generally present in an amount less than about 5 weight percent and more typically about 2 weight percent.

In one specific example, the first plurality of filaments may comprise a constituent chemistry which provides for a first color for the first stratum 20 while the second plurality of filaments may comprise a constituent chemistry which provides for a second color of the second stratum 30. The first color and the second color may be different. Such color differentiation may be beneficial in providing a masking benefit for liquid insults in an absorbent article.

For those forms of the present invention where one of the first strata 20 or the second strata 30 comprise a film, any suitable material may be utilized. Some suitable examples are described in U.S. Pat. No. 3,929,135, entitled "Absorptive Structures Having Tapered Capillaries", which issued to Thompson on Dec. 30, 1975; U.S. Pat. No. 4,324,246 entitled "Disposable Absorbent Article Having A Stain Resistant Topsheet", which issued to Mullane and Smith on Apr. 13, 1982; U.S. Pat. No. 4,342,314 entitled "Resilient Plastic Web Exhibiting Fiber-Like Properties", which issued to Radel and Thompson on Aug. 3, 1982; and U.S. Pat. No. 4,463,045 entitled "Macroscopically Expanded Three-Dimensional Plastic Web Exhibiting Non-Glossy Visible Surface and Cloth-Like Tactile Impression", which issued to Ahr, Lewis, Mullane, and Ouellette on Jul. 31, 1984. Additionaly exemplary films are discussed in U.S. Patent Nos. 7,410,683; 8,440,286 and 8,697,218.

The material webs of the present invention may utilize the strata composition variation independently or in conjunction with the surface energy, filament size, filament cross-sectional shape, filament cross-sectional configuration, and/or curled filament variations discussed heretofore. And, for those forms comprising third stratum and, in some forms, fourth stratum, the filament composition between at least two strata may be different.

<u>Softness / Coefficient of Friction Reduction</u>

As noted previously, the material webs of the present invention may comprise a plurality of nonwoven strata. The addition of a melt additive to the thermoplastic polymers listed herein can provide a Z-direction characteristic difference with regard to the softness of one or more of the strata. For example, the first plurality of filaments of the first strata 20 may comprise a melt additive that reduces the coefficient of friction of the filaments which can lead to an increase in the perception of softness by a user. The second plurality of filaments of the second strata 30 may not comprise this same melt additive or may not comprise a melt additive with regard to reducing the coefficient of friction among the second plurality of filaments.

The melt additive provided for softness is preferably a fast bloom slip agent, and can be a hydrocarbon having one or more functional groups selected from hydroxide, aryls and substituted aryls, halogens, alkoxys, carboxylates, esters, carbon unsaturation, acrylates, oxygen, nitrogen, carboxyl, sulfate and phosphate. In one particular form, the slip agent is a salt derivative of an aromatic or aliphatic hydrocarbon oil, notably metal salts of fatty acids, including metal salts of carboxylic, sulfuric, and phosphoric aliphatic saturated or unsaturated acid having a chain length of 7 to 26 carbon atoms, preferably 10 to 22 carbon atoms. Examples of suitable fatty acids include the monocarboxylic acids lauric acid, stearic acid, succinic acid, stearyl lactic acid, lactic acid, phthalic acid, benzoic acid, hydroxystearic acid, ricinoleic acid, naphthenic acid, oleic acid, palmitic acid, erucic acid, and the like, and the corresponding sulfuric and phosphoric acids. Suitable metals include Li, Na, Mg, Ca, Sr, Ba, Zn, Cd, Al, Sn, Pb and so forth. Representative salts include, for example, magnesium stearate, calcium stearate, sodium stearate, zinc stearate, calcium oleate, zinc oleate, magnesium oleate and so on, and the corresponding metal higher alkyl sulfates and metal esters of higher alkyl phosphoric acids.

In other forms, the slip agent is a non-ionic functionalized compound. Suitable functionalized compounds include: (a) esters, amides, alcohols and acids of oils including aromatic or aliphatic hydrocarbon oils, for example, mineral oils, naphthenic oils, paraffinic oils; natural oils such as castor, corn, cottonseed, olive, rapeseed, soybean, sunflower, other vegetable and animal oils, and so on. Representative functionalized derivatives of these oils include, for example, polyol

esters of monocarboxylic acids such as glycerol monostearate, pentaerythritol monooleate, and the like, saturated and unsaturated fatty acid amides or ethylenebis(amides), such as oleamide, erucamide, linoleamide, and mixtures thereof, glycols, polyether polyols like Carbowax, and adipic acid, sebacic acid, and the like; (b) waxes, such as carnauba wax, microcrystalline wax, polyolefin waxes, for example polyethylene waxes; (c) fluoro-containing polymers such as polytetrafluoroethylene, fluorine oils, fluorine waxes and so forth; and (d) silicon compounds such as silanes and silicone polymers, including silicone oils, polydimethylsiloxane, amino-modified polydimethylsiloxane, and so on.

The fatty amides useful in the present invention are represented by the formula:

$$RC(O)NHR^1$$

where R is a saturated or unsaturated alkyl group having of from 7 to 26 carbon atoms, preferably 10 to 22 carbon atoms, and $R^1$ is independently hydrogen or a saturated or unsaturated alkyl group having from 7 to 26 carbon atoms, preferably 10 to 22 carbon atoms. Compounds according to this structure include for example, palmitamide, stearamide, arachidamide, behenamide, oleamide, erucamide, linoleamide, stearyl stearamide, palmityl palmitamide, stearyl arachidamide and mixtures thereof.

The ethylenebis(amides) useful in the present invention are represented by the formula:

$$RC(O)NHCH_2CH_2NHC(O)R$$

where each R is independently is a saturated or unsaturated alkyl group having of from 7 to 26 carbon atoms, preferably 10 to 22 carbon atoms. Compounds according to this structure include for example, stearamidoethylstearamide, stearamidoethylpalmitamide, palmitamidoethylstearamide, ethylenebisstearamide, ethylenebisoleamide, stearylerucamide, erucamidoethylerucamide, oleamidoethyloleamide, erucamidoethyloleamide, oleamidoethylerucamide, stearamidoethylerucamide, erucamidocthylpalmitamide, palmitamidoethyloleamide and mixtures thereof.

Commercially available examples of fatty amides include Ampacet 10061 which comprises 5 percent of a 50:50 mixture of the primary amides of erucic and stearic acids in polyethylene; Elvax 3170 which comprises a similar blend of the amides of erucic and stearic acids in a blend of 18 percent vinyl acetate resin and 82 percent polyethylene. These slip agents are available from DuPont. Slip agents also are available from Croda Universal, including Crodamide OR (an oleamide), Crodamide SR (a stearamide), Crodamide ER (an erucamide), and Crodamide BR (a behenamide); and from Crompton, including Kemamide S (a stearamide), Kemamide B (a behenamide), Kemamide O (an oleamide), Kemamide E (an erucamide), and Kemamide (an N,N'-ethylenebisstearamide). Other commercially available slip agents include Erucamid ER erucamide.

Other suitable melt additives for softness / reduction of the coefficient of friction include erucamide, stearamide, oleamide, and silicones e.g. polydimethylsiloxane. Some specific examples include Crodamide™ slip & anti-block agents from Croda™, and Slip BOPP from Ampacet™. Some additional specific examples of softness / reduction of the coefficient of friction melt additives specifically tailored for polypropylene are from Techmer™ and sold under the trade names, PPM16368, PPM16141, PPM11790, PPM15710, PPM111767, PPM111771, and PPM12484. Some specific examples specifically tailored for polyethylene are from Techmer™ and sold under the trade name PM111765, PM111770, and PM111768.

The material webs of the present invention may utilize the softness melt additive variation independently or in conjunction with the surface energy, filament size, filament cross-sectional shape, filament cross-sectional configuration, and/or curled filament variations discussed heretofore. And, for those forms comprising third stratum and, in some forms, fourth stratum, the filament composition between at least two strata may be different.

Additionally, some softness melt additives may provide a softness benefit as well as a surface energy modification benefit. For example, fatty amides also provide a hydrophobic benefit. These melt additives are listed herein under hydrophobic melt additives.

Thickness

Still another way to create Z-direction characteristic differences in the material webs of the present invention is via the variation of thickness of the first stratum 20 versus the thickness of the second stratum 30. For example, the first plurality of filaments may comprise curled filaments, as discussed previously. In such forms, it may be beneficial to create the first stratum 20 with a greater thickness than that of the second stratum 30 such that liquid insults may be better masked because of the increased distance from the first surface 50 of the material web. The thickness of each stratum and the overall material web can be adjusted by varying at least one of the basis weights of the strata, the filament size, the filament shape, filament curl, or any other suitable process. In other forms, the second stratum 30 may have a greater thickness than that of the first stratum 20. A method of measuring thicknesses is provided below.

In some forms, the thickness of the strata may not be readily discernable without much analysis. For example,

where the difference between the first stratum 20 and the second stratum 30 are solely with regard to surface energy, visual analysis may not be sufficient to determine the delineation 54 between the first stratum 20 and the second stratum 30. However, for other forms, for example, where the difference is with regard to filament cross-sectional shape, filament size, filament cross-sectional configuration, and/or curled filaments, visual examination - as described herein - can provide the thickness of the first stratum 20 / second stratum 30. Principally we can change the thickness by either changing the number of filaments (the basis weight of one of the strata) or by changing the porosity (i.e. the void volume fraction).

For those forms of the present invention comprising third stratum, the third stratum may comprise a thickness which is different than the thickness of the first stratum 20 and/or the thickness of the second stratum 30.

Permeability, Capillarity, Acquisition, Rewet

Each of the foregoing strata characteristics can impact the properties of their respective strata, e.g. permeability, porosity, capillarity, acquisition, rewet softness, masking, and/or visual distinction, e.g. color difference. Many of these properties present tradeoffs. For example, quick acquisition time of liquid insults can lead to potential rewet problems. These tradeoffs are discussed in additional detail in the "EXAMPLES" section of this specification.

**Material web - MD and/or CD characteristic difference**

In addition to the Z-direction characteristic differences through the strata which can be created via the modification of the strata, e.g. surface energy, filament size, filament cross-sectional shape, filament cross-sectional configuration, filament curl, filament composition, softness / coefficient of friction reduction, and/or thickness of the strata, material webs 10 of the present invention may additionally comprise deliberate MD/CD characteristic differences within each stratum. The MD/CD characteristic differences discussed hereafter may be utilized in conjunction with or independently of the Z-direction characteristic differences discussed heretofore. The utilization of MD/CD characteristic differences can similarly impact properties of the material webs like permeability, softness, acquisition, rewet, masking, and/or visual distinction, e.g. color differences.

The MD/CD characteristic differences can be created via the creation of discrete discontinuities in the material webs of the present invention. These discrete discontinuities can vary the property in localized areas of each of the strata. For example, the first surface 50 and second surface 52 of the material webs 10 of the present invention are generally considered as planar. Discontinuities are disruptions to the planar surface - either the first surface 50 and/or the second surface 52. Some exemplary discontinuities include apertures, bond sites, embossing, tunnel tufts, filled tufts, nested tufts, corrugations, and/or grooves.

Apertures

Referring to Figures 5A and 5B, one way to create an MD and/or CD characteristic differences is through the utilization of apertures. A laminate 100 comprising the material web 10 and a material layer 170, e.g. secondary topsheet or acquisition layer is shown. In one example, material webs 10 of the present invention may further comprise apertures 125 which extend from the first surface 50 to the second surface 52 of the material web 10. As shown, the material web 10 may comprise a plurality of apertures 125. And as shown, the apertures 125 while extending through the material web 10 may not extend through a material layer 170. The material layer 170 may be any suitable layer from a disposable absorbent article, e.g. secondary topsheet, acquisition layer, distribution layer, combinations thereof, etc.

In another form, the material layer 170 may be an additional stratum integrally formed with the material web 10 where the apertures 125 have been created with an ablating process such as for example a laser-based material removal step that precisely removes small regions of the filament material (in an intentional pattern) to a certain depth such as one or two strata. Forms of the present invention are contemplated where the apertures extend only through the first stratum 20 and not through the second stratum 30 via an ablation process, or vice versa. For example, discrete portions of the first stratum 20 may be ablated to form apertures therethrough. And, where the second stratum comprises a color difference than the second stratum color would be more visible through the discrete portions. While outside of the discrete portions, the second color would appear different.

The apertures 125 can increase the permeability of the material web 10 and also decrease acquisition time. The apertures 125 may be any suitable size. For example, apertures 125 may have an Effective Aperture AREA in the range of about 0.1 $mm^2$ to about 15 $mm^2$, preferably from about 0.3 $mm^2$ to about 10 $mm^2$, more preferably from about 0.5 $mm^2$ to about 8 $mm^2$, or most preferably from about 1.0 $mm^2$ to about 5 $mm^2$, specifically including all 0.05 $mm^2$ increments within the specified ranges and all ranges formed therein or thereby. All Effective Aperture Areas are determined using the Aperture Test described herein. Effective Aperture Area is discussed in further detail in U.S. Patent Application Serial Nos. 14/933,028; 14/933,017; and 14/933,001.

The apertures 125 may be produced by any suitable method. For example, in some forms, each of the apertures 125 may be surrounded, at least in part, by a melt lip 135. In some forms of the present invention, the first stratum 20 and the second stratum 30 may be joined about the periphery of each of the plurality of apertures 125 via the melt lip 135. For example, melt lips 135 may be created, in part, by melting / fusing filaments of the first stratum 20 and second stratum 30. During the melting / fusing, the melted filament material can form bonds with surrounding filaments of the first stratum 20 and the second stratum 30, thereby forming a thin film like area.

The thin film like areas may be subsequently broken. The breaking apart of the thin film like areas forms the aperture 125 and the melt lip 135. Generally, to break apart the melted areas, the material web 10 is stretched in the CD direction. This stretching causes a portion of the thin film like areas to break apart and form apertures 125. A remaining portion of the film like area remains unbroken forming the melt lip 135. Additionally, during the aperturing process, the material web 10 is generally under tension in the MD direction. This process is further described in in U.S. Patent Nos. 5,658,639; 5,628,097; 5,916,661; 7,917,985; and U.S. Patent Application Publication No. 2003/0021951 and U.S. Patent Application Serial Nos. 14/933,028; 14/933,017; and 14/933,001. Additional processes for aperturing material webs are described in U.S. Patent Nos. 8,679,391 and 8,158,043, and U.S. Patent Application Publication Nos. 2001/0024940 and 2012/0282436. Other methods for aperturing material webs are provided in U.S. Patent Nos. 3,566,726; 4,634,440; and 4,780,352.

For those forms of the present invention which include third stratum and optionally fourth stratum, the apertures 125 may be formed in the resultant material web. The apertures in such forms, may extend from the first surface through the second surface of the resultant material web.

Forms of the present invention are contemplated where apertures are provided to the material webs of the present invention in a pattern or a plurality thereof. For example, an array of apertures may be provided to the material webs of the present invention. Some exemplary patterns are disclosed with regard to Figures 19-32.

Referring to Figures 19-32, material webs 1000 of the present invention may comprise an array of apertures comprising a plurality of patterns 1110A and 1110B with continuous or semi-continuous land areas. As shown, a first pattern 1110A may comprise a first plurality of apertures which are oriented in a direction which is generally parallel to a machine direction 1675 as well as a second plurality of apertures which are oriented at multiple angles with respect to the machine direction. Similarly, a second pattern 1110B may comprise a third plurality of apertures which are oriented at multiple angles with respect to the machine direction 1675 as well as a fourth plurality of apertures which are generally parallel to the machine direction 1675. As shown, the apertures of the first pattern 1110A and/or the second pattern 1110B may be of different lengths, different angles with respect to the machine direction 1675, and/or different Effective Aperture AREAs. Effective Aperture Areas are discussed hereafter.

Additionally, at least one or a plurality of apertures in the first pattern 1110A may be substantially enclosed by the second pattern 1110B, e.g. third plurality of apertures and fourth plurality of apertures. For example, the second pattern may form a quilt like pattern, e.g. diamond shaped boundaries or any other suitable shape, with the first pattern disposed within the second pattern thereby forming a unit. The combination of the first pattern and the second pattern may repeat so that there are a plurality of units. Additionally, the first pattern within the second pattern may be different from one unit to the next. Additional patterns may be utilized. The apertures angled with respect to the machine direction 1675 are believed to aid in fluid acquisition/distribution. For example, fluid moving along the patterned web 1164 in the machine direction 1675 may be diverted, in part, because of the angled apertures.

Still referring to Figures 18-32, as noted previously, the first pattern 1110A and/or the second pattern 1110B may comprise a plurality of apertures of which at least a portion are angled with respect to the machine direction 1675 at a first angle 1680 and another portion are angled with respect to the machine direction at a second angle 1682. The first angle 1680 and the second angle 1682 may be different from one another. In some forms, the second angle 1682 may be the mirror image of the first angle 1680. For example, the first angle may be about 30 degrees from an axis parallel to the machine direction 1675 while a second angle is -30 degrees from the axis parallel to the machine direction 1675. Similarly, the first pattern 1110A and/or the second pattern 1110B may comprise a plurality of apertures which are oriented generally parallel to the machine direction 1675. Apertures which are oriented generally parallel to the machine direction 1675 generally have a lower aspect ratio (discussed hereafter) and larger Effective Aperture AREA (described hereafter) as opposed to those apertures which are angled with respect to the machine direction 1675. It is believed that those apertures with increased Effective Aperture AREA allow for quicker fluid acquisitions time. While any suitable angle may be utilized, as discussed hereafter, once the first angle 1680 and the second angle 1682 are increased beyond 45 degrees from the machine direction 1675 (-45 in the case of the second angle 1682), the forces of the cross-direction 1677 stretching act more along a long axis of the aperture than perpendicular thereto. So, apertures which are angled more than 45 degrees with respect to the machine direction 1675 (- 45 degrees in the case of the second angle 1682) typically comprise less Effective Aperture AREA than those which are angled to a lesser extent with respect to the machine direction 1675.

As stated previously, the angled apertures are believed to provide additional fluid handling benefits for the patterned web 1164. In some forms, greater than about 10 percent of the apertures are angled with respect to the

machine direction 1675. Additional forms are contemplated where greater than about 20 percent, greater than about 30 percent, greater than about 40 percent, greater than about 50 percent, greater than about 60 percent, greater than about 70 percent, greater than about 80 percent and/or less than 100 percent, less than about 95 percent, less than about 90 percent, less than about 85 percent of the apertures are angled with respect to the machine direction 1675 including any number or any ranges encompassed by the foregoing values.

Referring to Figures 19-32, the population density of apertures may be greater nearer a centerline 1690 of the material web 1000. For example, interaperture distance between adjacent apertures near the centerline 1690 may be a first distance while interaperture distance between adjacent apertures further away from the centerline 1690 may be a second distance. The first distance may be less than the second distance. As an example, interaperture distance between adjacent apertures can be about 1 mm. As such, the first distance may be about 1 mm while the second distance may be about 5 mm or greater. Additional forms are contemplated where the interaperture distance between adjacent apertures increases with increasing distance from the centerline. Interaperture distances are discussed further hereafter.

Additionally, in some instances, apertures nearer the centerline 1690 may be angled at the first angle 1680 while apertures further from the centerline 1690 are positioned at the second angle 1682. The first angle 1680 may be greater than the second angle 1682 with respect to the centerline 1690. For, example, the apertures further from the centerline 1690 may be oriented such that they are generally parallel to the centerline 1690 while the apertures positioned closer to the centerline 1690 are angled with respect to the centerline 1690. In some forms, the angle at which apertures are positioned relative to the centerline 1690 may decrease as the distance from the centerline 1690 increases. For example, a first aperture adjacent the centerline 1690 may be oriented at a first angle of 30 degrees with respect to the centerline 1690, while a second aperture 1 mm from the centerline 1690 may be oriented at 20 degrees from the centerline. The apertures positioned furthest away from the centerline 1690 may be generally parallel to the centerline 1690. Additional configurations are contemplated where apertures near the centerline 1690 are angled to a lesser extent than those further from the centerline 1690. In some embodiments, the apertures near the centerline 1690 may be generally parallel to the centerline 1690 while the apertures further from the centerline 1690 are angled with respect to the centerline 1690. Feret angles of apertures are discussed further hereafter.

As stated previously the lengths of the apertures may vary as well. In conjunction with being angled as disclosed above or independently therefrom, in some embodiments, the apertures adjacent the centerline 1690 may be longer than those which are further away from the centerline 1690. Similarly, the size of the apertures may vary. Variances in aperture size (Effective Aperture AREA) may be employed in conjunction with the variation of aperture angle and/or the variation in aperture length, or variances in aperture size may be employed independently of the variation of aperture angle and/or variation in aperture length. For those forms where aperture size may vary, larger apertures may be positioned adjacent the centerline 1690 while apertures having a smaller Effective Aperture AREA are positioned further away from the centerline 1690. For example, apertures adjacent the centerline 1690 may have an Effective Aperture AREA of 15 square millimeters while apertures further away from the centerline may have less Effective Aperture AREA, e.g. 1.0 square mm. Any of the values / ranges of Effective Aperture AREA provided herein may be utilized for configuring the Effective Aperture AREA variance described above.

As mentioned previously, the angle of orientation of the aperture can impact the fluid handling capabilities of the material web 1000. Moreover, length of the aperture, width of the aperture, Effective Aperture AREA, spacing between apertures, as well as aperture density can similarly impact fluid handling. However, many of length of apertures, width of apertures, angle of orientation, spacing and density can have competing / negative impacts on the other variables. As stated previously, apertures which are at a greater angle to the machine direction 1675 tend to open less and therefore have less Effective Aperture AREA than apertures which are either parallel to the machine direction 1675 or which have a smaller angle with respect to the machine direction 1675. Similarly, angled apertures which are too closely spaced together tend to open less and therefore have less Effective Aperture AREA. As such, Interaperture distance between adjacent angled apertures may be increased over that which is between apertures which are generally oriented parallel to the machine direction 1675. Additional details of such forms are discussed further in U.S. Patent Application Serial Nos. 14/933,028; 14/933,017; and 14/933,001.

Additional processes for forming apertures in the material webs of the present invention are contemplated. Some additional processes for forming apertures are disclosed in U.S. Patent Nos. 8,679,391 and 8,158,043, and U.S. Patent Application Publication Nos. 2001/0024940 and 2012/0282436. Other methods for aperturing webs are provided in U.S. Patent Nos. 3,566,726; 4,634,440; and 4,780,352.

Referring back to Figures 5A and 5B, the material webs 10 of the present invention may comprise apertures as described above (including patterned apertures) along with the Z-direction characteristic differences described heretofore. Or the apertures described herein may be used independently thereof. Generally, apertures increase permeability. However, the introduction of apertures in a topsheet can also increase the likelihood of rewet.

As noted previously, forms of the present invention are contemplated where the apertures extend through the first stratum 20 but not through the second stratum 30 or vice versa. Such material webs can be obtained, for example, by forming the first stratum 20 and forming apertures therein. Subsequently, the second stratum 30 may be formed on

the first stratum 20 as described herein or vice versa.

Bond Sites

Yet another way to create an MD and/or CD characteristic difference in the material web 10 is via the utilization of bond sites. Referring now to Figures 5A, 5C, and 5D, the material web 10 may further comprise a plurality of bond sites 175. The bond sites 175 can join the first stratum 20, the second stratum 30, and a material layer 171. The material layer 171 can be a secondary topsheet or a distribution layer where the material web 10 forms a portion of a topsheet of an absorbent article.

In some forms, the bond sites 175 may be formed when the material web 10 and material layer 171, e.g. a secondary topsheet or acquisition layer, are passed through a nip between a pair of counter-rotating rolls exerting so much pressure that the filament materials are deformed into flat topography and usually leading to the filaments in these bond sites to become attached to one another. At least one of the rolls comprises nubs which compress the material web 10 at the bond site 175. In some forms of the present invention, the nubs may engage the first surface 50 of the first stratum 20 and compress the material web 10 and the material layer 171. The compression can be in the negative Z-direction and may generally thin the material of the first stratum 20, the second stratum 30, and the material layer 171 which make up the bond site 175. This compression, which can be coupled with the application of heat in some forms, can cause the constituent material of the first stratum 20, the second stratum 30, and the material layer 171 at the bond site 175 to fuse together.

Referring to Figure 5D, alternate forms of bond sites 175 may exist. Recall that the formation of the bonds may be derived from a pair of rolls, one of which comprises nubs. Forms of the present invention are contemplated, where each of the pair of rolls comprises nubs and as the nubs engage the material web 10 and material layer 171, a first nub from a first roll may compress the material web 10 and material layer 171 in the negative Z-direction, and a second nub from a second roll may compress the material web 10 and the material layer 171 in the positive Z-direction. In such forms of the present invention, the resulting bond sites 175 may have depressions the first surface 50 of the material web 10 and a second surface 55 of the laminate 100. And, for those forms where there are additional strata are subjected to the bonding process, the constituent material of the additional strata become intimately connected and adhered to the constituent material of the first stratum 20 and the second stratum 30 and the material layer 171. The bonding of the constituent material of the first stratum 20, second stratum 30, material layer 171 and additional strata can create a thin film like area on opposing sides of the laminate 100.

Bond sites 175 of the present invention may be any suitable shape, essentially any geometric 2-dimensional shape that can be drawn. Some suitable shapes include circular, elliptical, rectangular, diamond, heart, star, clover (3 leaf, 4 leaf), bowtie shapes and combinations thereof. In some forms, the bond sites 175 may comprise a plurality of shapes. Suitable bond shapes are discussed in U.S. Patent Application Serial No. 14/933,017.

The bond sites 175 can impact the softness of the material web 10 as well as its resiliency. For example, where adjacent bond sites 175 are spaced apart (center-to-center) by more than 4mm or between about 10 mm to about 12 mm, a soft feeling may be achieved. Conversely, adjacent bond sites 175 may not provide a soft feel if center to center spacing is less than about 4 mm. Such spacings and distinctions between "soft" and "not soft" also depends on the bond shape or combination of shapes. Bond sites 175 may be used in conjunction with the apertures 125 or independently thereof. Processes for bonding are generally known in the art, including thermal and high pressure bonding. Additionally, forms of the present invention are contemplated where the bond sites are provided in patterns. Details of such forms are discussed in further detail in U.S. Patent Application Serial Nos. 14/933,028; 14/933,017; and 14/933,001.

The strata of the present invention may be bonded together via primary bond sites. Typically, the primary bond sites are thermal point bonds fusing or compressing all strata of the material web together in discrete areas forming film-like discrete primary bond sites. The bond sites 175 discussed herein exclude primary bond sites.

The material webs 10 of the present invention may comprise bond sites 175 as described above (including patterned bond sites) along with the Z-direction characteristic differences and/or apertures described heretofore. Or the apertures described herein may be applied independently thereof. In one specific example, where the first stratum 20 comprises a first color and the second stratum 30 comprises a second color different from the first color, the bond site 175 can effectively shift the second color as seen through the bond site 175 such that the second color as seen through the first stratum 20 is different than the color seen via the bond site 175. The same effect may be achieved where the first stratum 20 and the second stratum 30 comprise the same color but where the material layer 171 comprises a color different than that of the first stratum 20 and second stratum 30. Such color effects are disclosed in addition detail in U.S. Patent Application Serial No. 14/933,001.

Embossments

Still another way to create an MD and/or CD characteristic difference in the material webs 10 of the present

invention is via the utilization of embossments. Referring to Figure 5E, unlike bond sites 175 (shown in Figures 5A, 5C, and 5D), embossments 180 typically do not cause the actual bonding of the constituent material of the first stratum 20, the second stratum 30, and the material layer 170 e.g. secondary topsheet or acquisition layer via melting. Instead, embossments 180 tend to visibly and permanently compress the first stratum 20, the second stratum 30, and the material layer 170. For those forms of the present invention which comprise additional strata, the embossment 180 may comprise the first stratum 20, the second stratum 30, the material layer 170 and the additional strata. In some forms, the embossment 180 may be limited to the material web 10 or additional layers if present.

Embossments 180 can provide an acquisition gradient in an absorbent article. For example, where the material web 10 forms a portion of a topsheet of an absorbent article, the embossment 180 may not readily receive a liquid insult. Instead, the embossment 180 may act as a fluid highway which can distribute the insult to multiple areas of an absorbent core in the absorbent article.

Embossments 180 may be used in conjunction with apertures 125, bond sites, 175, and/or any of the Z-direction characteristic differences disclosed herein, or may be utilized independently thereof. Generally, embossments, decrease permeability in the area of the embossment but decrease the likelihood of rewet in an absorbent article.

Forms of the present invention are contemplated where the first stratum 20 or second stratum 30 is embossed prior to the formation of the second stratum 30 or first stratum 20 thereon, respectively. Forms are also contemplated where the material web comprises at least a third stratum in addition to the first stratum 20 and the second stratum 30. In such forms, the first and second strata may be embossed prior to the formation of the third strata thereon.

## Tunnel Tufts

Still another way to create a characteristic difference in the MD and/or CD is via the utilization of tunnel tufts. Referring to Figure 6A, material webs of the present invention may comprise tunnel tufts 270. As shown, in some forms, some of the second plurality of filaments of the second stratum 30 may extend in the positive Z-direction beyond the first surface 50 to form tunnel tufts 270. And, a corresponding opening 285 may be created in the second surface 52 of the material web 10.

The tunnel tuft 270 may be created when localized areas of constituent material of the first stratum 20 and the second stratum 30 are urged in the positive Z-direction such that material of the first stratum 20 and/or second stratum 30 may be disposed superjacent to the first surface 50 of the material web 10. The disposition of the second plurality of filaments of the second stratum 30 may form the tunnel tuft 270. And, as shown in Figure 6A, in some forms, the disposition of the first plurality of filaments in the first stratum 20 may cause at least some of the first plurality of filaments to break under the urging in the Z-direction. In such forms, the tunnel tufts 270 may extend through ends 245 of the first plurality of filaments. However, as shown in Figure 6B, the disposition of the first plurality of filaments of the first stratum 20 may, create an outer tuft 230. In some forms, the outer tuft 230 may form a cap over the tunnel tuft 270.

In some forms, material webs 10 of the present invention may comprise a plurality of tunnel tufts 270 for which there are no corresponding outer tufts 230 and/or similarly may comprise a plurality of tunnel tufts 270 each of which are disposed within a corresponding outer tuft 230.

Additional arrangements of tunnel tufts are provided with respect to Figures 6C-6D. As shown, the tunnel tuft 270 and/or outer tuft 230 may extend beyond the second surface 52 of the material web 10. However, instead being urged in the positive Z-direction, urging of the material of the first stratum 20 and the second stratum 30 may be in the negative Z-direction. And, similar to Figure 6A, some of the second plurality of filaments of the second stratum 30 may break as shown in Figure 6C or may form the outer tuft 230 as shown in Figure 6D.

Figures 6A-6E illustrate tunnel tufts 270 which may be formed with material webs comprising extensible filaments. The tunnel tufts 270 and outer tufts 230 disclosed herein comprise a plurality of looped filaments that are substantially aligned such that each of the tunnel tufts 270 and outer tufts 230 have a distinct linear orientation and a longitudinal axis L of the tuft, e.g. 270, 230. By "aligned", it is meant that looped filaments are all generally oriented such that, if viewed in plan view, each of the looped filaments has a significant vector component parallel to a transverse axis and can have a major vector component parallel to the transverse axis. The transverse axis T is generally orthogonal to longitudinal axis in the MD-CD plane and the longitudinal axis is generally parallel to the MD.

Another characteristic of the tunnel tufts 270 and outer tufts 230 shown in Figures 6A-6E - formed with extensible non-curled filaments -- can be their generally open structure characterized by open void area 633 defined interiorly of the tunnel tuft 270. The term "void area" is not meant to refer to an area completely free of any filaments. The void area 633 of tunnel tufts 270 may comprise a first void space opening and a second void space opening. Rather, the term is meant as a general description of the general appearance of tunnel tuft 270. Therefore, it may be that in some tunnel tufts 270 a non-looped filaments or a plurality of loose non-looped filaments may be present in the void area 633. By "open" void area is meant that the two longitudinal ends of tunnel tuft 270 are generally open and free of filaments, such that the tunnel tuft 270 can form something like a "tunnel" structure in an uncompressed state, as shown in Figures 6A-6D.

Regarding Figure 6E, tunnel tuft can comprise a plurality of looped filaments that are substantially aligned such

that each of the tunnel tufts have a distinct linear orientation and a longitudinal axis L. By "looped" filaments it is meant to refer to filaments of the tufts that are integral with and begin and end in the nonwoven stratum in which they begin but extend generally outwardly in the Z-direction (or negative Z-direction) from the first surface or second surface of the respective stratum. By "aligned", it is meant that looped filaments are all generally oriented such that, if viewed in plan view, each of the looped filaments has a significant vector component parallel to a transverse axis and can have a major vector component parallel to the transverse axis. The transverse axis T is generally orthogonal to longitudinal axis in the MD-CD plane and the longitudinal axis is generally parallel to the MD.

The extension and/or urging of the first plurality of filaments and the second plurality of filaments, as shown in Figures 6A-6D, can be accompanied by a general reduction in filament cross sectional dimension (e.g., diameter for round filaments) due to plastic deformation of the filaments and Poisson's ratio effects.

Tunnel tufts 270 and/or outer tufts 230 can provide a masking benefit for liquid insults in a disposable absorbent article. Additionally, tunnel tufts 270 and/or outer tufts 230 can provide a softness benefit as well. Tunnel tufts 270 and/or outer tufts 230 can be provided to the material web in any suitable configuration. Forms are contemplated where the tunnel tufts 270 and/or outer tufts 230 area arranged in zones and/or patterns. Such zones and patterns are described in additional detail in U.S. Patent Application Serial No. 14/933,017.

Tunnel tufts 270 and outer tufts 230 are discussed in additional detail, including methods of making, in U.S. Patent Nos. 7,172,801; 7,838,099; 7,754,050; 7,682,686; 7,410,683; 7,507,459; 7,553,532; 7,718,243; 7,648,752; 7,732,657; 7,789,994; 8,728,049; and 8,153,226.

The tunnel tufts 270 and/or outer tufts 230 may be used in conjunction with the apertures, bond sites, embossments to create an MD and/or CD characteristic difference and/or with any of the Z-direction characteristic differences described herein. Or, the tunnel tufts 270 and/or outer tufts 230 may be utilized independently thereof.

Forms of the present invention are contemplated where the material web 10 of the present invention comprising tunnel tufts and/or outer tufts is utilized in an absorbent article as a topsheet. In such forms, the tunnel tufts and/or outer tufts may form a portion of a wearer-facing surface of the absorbent article - tufts oriented in the positive Z-direction. In other forms where the material web 10 is a topsheet, the material web 10 along with a subjacent layer of the absorbent article, e.g. acquisition layer, secondary topsheet, may comprise the tunnel tufts and/or outer tufts described herein. In such forms, the subjacent layer may form the outer tufts and the tufts may be oriented in the negative Z-direction and positioned on a garment-facing side of the material web 10.

Forms of the present invention are contemplated where the first stratum 20 or second stratum 30 is provided with tunnel tufts prior to the formation of the second stratum 30 or first stratum 20 thereon, respectively. Forms are also contemplated where the material web comprises at least a third stratum in addition to the first stratum 20 and the second stratum 30. In such forms, the first and second strata may be provided with tunnel tufts prior to the formation of the third strata thereon.

## Filled Tufts

Another way to create characteristic differences in the MD and/or CD is with the utilization of filled tufts. In contrast to the tunnel tufts 270 shown in Figures 6A-6E, material webs of the present invention comprising curled filaments either in the first plurality of filaments or the second plurality of filaments form very different discontinuities - filled tufts - than those shown in Figures 6A-6E. Shown in Figures 7A-7E are schematic representations of the material web 10 comprising filled tufts.

The material web 10 shown in Figures 7A-7D comprise at least one stratum which comprises curled filaments. As shown in Figure 7A, the second stratum 30 comprises a plurality of curled filaments. As ends 245 of some of the first plurality of filaments of the first stratum 20 are shown, the first stratum 20 may not comprise curled filaments. During the localized urging of the material web 10 in the positive Z-direction, at least some of the first plurality of filaments may break thereby creating ends 245. As shown the second plurality of filaments of the second stratum 30 form a filled tuft 370 which comprises a plurality of filaments which fill the filled tuft 370. The filled tuft 370 may extend through the first stratum 20. In some forms, as shown in Figure 7B, first plurality of filaments may form an outer tuft 330 which covers the filled tuft 370. And, as shown in Figures 7C and 7D, the first plurality of filaments of the first stratum 20 may in some forms, form the filled tuft 370. In some forms, the second plurality of filaments of the second stratum 30 may form the outer tuft 330. In such forms, the material web 10 is subjected to localized urging in the negative Z-direction.

As noted previously regarding "Curled Filaments", the first plurality of filaments may be curled and/or the second plurality of filaments may be curled. For those forms where the material web 10 comprises additional strata, the constituent filaments of the additional strata may comprise curled filaments.

In contrast to the tunnel tufts 270 (shown in Figures 6A-6E), filled tufts 370, are substantially filled with looped filaments and/or non-looped filaments. Additionally, unlike the alignment of filaments with the transverse axis shown in Figure 6E, the curled filaments of the filled tufts 370 can appear more random with regard to the transverse axis T. And, in contrast to the tunnel tufts 270, shown in Figures 6A-6E, it has been found that for the filled tufts 370, the constituent

filaments quite often uncoil from their curly state rather than become stretched and thinned.

The filled tufts 370 can be beneficial for those forms where the second plurality of filaments form the filled tuft 370 and where the first plurality of filaments (at least a portion thereof) break upon the localized Z-direction urging. For example, if the first plurality of filaments do not create a corresponding outer tuft 330, liquid insults can have easy access to the second plurality of filaments of the filled tuft 370. And, if the second plurality of filaments are hydrophilic -- either from a filament composition standpoint and/or melt additive standpoint, the filled tuft 370 will provide additional surface area for the liquid to contact. Similarly, even in those forms where a corresponding outer tuft 330 exists, the filled tuft 370 may still provide great liquid handling properties.

Additionally, where the material webs of the present invention comprise at least one stratum comprising curled filaments, the resultant material web has a higher caliper for a given basis weight. This higher caliper in turn delivers consumer benefits of comfort due to cushiony softness, faster absorbency due to higher permeability, and improved masking. Additional benefits may include less red marking, higher breathability and resiliency.

Forms of the present invention are contemplated where the material web 10 comprising filled tufts and/or outer tufts is utilized in an absorbent article as a topsheet. In such forms, the filled tufts and/or outer tufts may form a portion of a wearer-facing surface of the absorbent article - tufts oriented in the positive Z-direction toward a wearer of the article. In other forms where the material web 10 is a topsheet, the material web 10 along with a subjacent layer of the absorbent article, e.g. acquisition layer, secondary topsheet, may comprise the filled tufts and/or outer tufts described herein. In such forms, the subjacent layer may form the outer tufts and the tufts may be oriented in the negative Z-direction and positioned on a garment-facing side of the material web 10.

Methods of making filled tufts 270 and outer tufts 330 are discussed in U.S. Patent Nos. 7,172,801; 7,838,099; 7,754,050; 7,682,686; 7,410,683; 7,507,459; 7,553,532; 7,718,243; 7,648,752; 7,732,657; 7,789,994; 8,728,049; and 8,153,226. Filled tufts 370 and corresponding outer tufts 330 are discussed in additional detail in U.S. Patent Application Serial No. 14/933,028.

The filled tufts 370 and/or outer tufts 330 may be used in conjunction with the apertures, bond sites, embossments to create an MD and/or CD characteristic differences and/or any of the Z-direction characteristic differences described herein. Or, the filled tufts 370 and/or outer tufts 330 may be utilized independently thereof.

Forms are contemplated where the material web comprises at least a third stratum in addition to the first stratum 20 and the second stratum 30. In such forms, the first and second strata may be provided with filled tufts prior to the formation of the third strata thereon.

## Nested Tufts

Yet another way to create an MD and/or CD characteristic difference is via the utilization of nested tufts. Referring now to Figures 8A-8D, examples of material webs 10 comprising nested tufts 632 are shown. As noted heretofore, the material web 10 has the first surface 50, the opposing second surface 52, and a thickness T therebetween (the thickness being shown in Figure 8D). Figure 8A shows the first surface 50 of the material web 10 with nested tufts 632 that extend outward (out of the plane of the sheet comprising Figure 8A) from the first surface 50 of the material web 10. As shown, the material web 10 may comprise a generally planar first region 640 and a plurality of discrete integral second regions 642 which comprise nested tufts 632.

As shown, the nested tufts 632 may have a width, W, that varies from one end 660 to the opposing end 660 when the nested tufts 632 are viewed in plan view. As shown, the width W may be generally parallel to a transverse axis TA. The width W may vary with the widest portion of the nested tufts 632 in the middle of the nested tufts 632, and the width of the nested tufts 632 decreasing at the ends 660 of the nested tufts 632. In other cases, the nested tufts 632 could be wider at one or both ends 60 than in the middle of the nested tufts 632. In still other cases, nested tufts 632 can be formed that have substantially the same width from one end of the nested tufts 632 to the other end of the nested tufts 632. If the width of the nested tufts 632 varies along the length of the nested tufts 632, the portion of the nested tufts 632 where the width is the greatest is used in determining the aspect ratio of the nested tufts 632.

Similarly, the nested tufts 632 may have a length L which is generally parallel to a longitudinal axis LA. When the nested tufts 632 have a length L that is greater or less than their width W, the length of the nested tufts 632 may be oriented in any suitable direction relative to the material web 100. For example, the length of the nested tufts 632 (that is, the longitudinal axis, LA, of the nested tufts 632) may be oriented in the MD, the CD, or any desired orientation between the MD and the CD. As shown, the transverse axis TA is generally orthogonal to the longitudinal axis LA in the MD-CD plane. In some forms, as shown, the longitudinal axis LA is parallel to the MD. In some forms, all the spaced apart nested tufts 632 may have generally parallel longitudinal axes LA.

Figure 8B shows the second surface 52 of the material web 10 such as that shown in Figure 8A, having nested tufts 632 formed therein, with the nested tufts 632 being oriented into the sheet showing Figure 8B. The second surface 52 may comprise a plurality of base openings 644. In some forms, the base openings 644 may not be in the form of an aperture or a through-hole. The base openings 644 may instead appear as depressions. In some forms, the base

openings 644 may open into the interior of the nested tuft 632.

Referring to Figures 8A, 8C and 8D, the nested tufts 632 may have any suitable shape when viewed from the side. Suitable shapes include those in which there is a distal portion or "cap" with an enlarged dimension and a narrower portion at the base when viewed from at least one side. The term "cap" is analogous to the cap portion of a mushroom. (The cap does not need to resemble that of any particular type of mushroom. In addition, the nested tufts 632 may, but need not, have a mushroom-like stem portion.) In some cases, the nested tufts 632 may be referred to as having a bulbous shape when viewed from the end 660. The term "bulbous", as used herein, is intended to refer to the configuration of the nested tufts 632 as having a cap 652 with an enlarged dimension and a narrower portion at the base when viewed from at least one side (particularly when viewing from one of the shorter ends 660) of the nested tufts 632. The term "bulbous" is not limited to nested tufts 632 that have a circular or round plan view configuration that is joined to a columnar portion. The bulbous shape, in the form shown (where the longitudinal axis LA of the nested tufts 632 is oriented in the machine direction), may be most apparent if a section is taken along the transverse axis TA of the nested tufts 632 (that is, in the cross-machine direction). The bulbous shape may be less apparent if the nested tufts 632 is viewed along the length (or longitudinal axis LA) of the nested tufts 632.

Referring to Figures 8A-8D, as discussed herein, the material web 10 of the present invention comprises multiple strata, and as shown, the individual strata can be designated 630A, 630B, etc. As shown, the nested tufts 632 may comprise: a base 650 proximate the first surface 50 of the material web 10; an opposed enlarged distal portion or cap portion, or "cap" 652, that extends to a distal end 654; side walls (or "sides") 656; an interior 658; and a pair of ends 660. The "base" 650 of the nested tufts 632 comprises the narrowest portion of the nested tufts 632 when viewed from one of the ends of the nested tufts 632. The term "cap" does not imply any particular shape, other than it comprises the wider portion of the nested tufts 632 that includes and is adjacent to the distal end 654 of the nested tufts 632. The side walls 656 have an inside surface and an outside surface. The side walls 656 transition into, and may comprise part of the cap 652. Therefore, it is not necessary to precisely define where the side walls 656 end and the cap 652 begins. The cap 652 will have a maximum interior width, $W_1$, between the inside surfaces of the opposing side walls 656. The cap 652 will also have a maximum exterior width W between the outside surfaces of the opposing side walls 656. The ends 660 of the nested tufts 632 are the portions of the nested tufts 632 that are spaced furthest apart along the longitudinal axis, L, of the nested tufts 632.

Still referring to Figures 8A-8D, the narrowest portion of the nested tufts 632 defines the base opening 644. The base opening 644 has a width $W_O$. The base opening 644 may be located (in the Z-direction) between a plane defined by the second surface 52 of the material web 10 and the distal end 654 of the nested tuft 632. The material web 10 may have an opening in the second surface 52 that transitions into the base opening 644 (and vice versa), and is the same size as, or larger than the base opening 644. The base opening 644 will, however, generally be discussed more frequently herein since its size will often be more visually apparent to the consumer in those embodiments where the material web 10 is placed in an article with the base openings 644 visible to the consumer. It should be understood that in certain forms of the present invention, base openings 644 face outward (for example, toward a consumer and away from the absorbent core in an absorbent article), it may be desirable for the base openings 644 not to be covered and/or closed off by another web.

The nested tufts 632 have a depth D measured from the second surface 30 of the material web 100 to the interior of the nested tufts 632 at the distal end 654 of the nested tufts 632. The nested tufts 632 have a height H measured from the second surface 30 of the material web 100 to the exterior of the nested tuft 632 at the distal end 654. In most cases the height H of the nested tufts 632 will be greater than the thickness T of the first region 640. The relationship between the various portions of the nested tufts 632 may be such that as shown in Figure 6H, when viewed from the end, the maximum interior width $W_I$ of the cap 652 of the nested tufts 632 is wider than the width, Wo, of the base opening 644.

The nested tufts 632 may, in some cases, be formed from looped filaments (which may be continuous) that are pushed outward so that they extend away from the first surface 50 in the Z-direction or away from the second surface 52 in the negative Z-direction. The nested tufts 632 will typically comprise more than one looped filament. In some cases, the nested tufts 632 may be formed from looped filaments and at least some broken filaments. In addition, in the case of some types of nonwoven materials (such as carded materials, which are comprised of shorter filaments), the nested tufts 632 may be formed from loops comprising multiple discontinuous filaments. Multiple discontinuous filaments in the form of a loop are described in U.S. Patent Application Serial No. 14/844,459. The looped filaments may be: aligned (that is, oriented in substantially the same direction); not be aligned; or, the filaments may be aligned in some locations within the protrusions 32, and not aligned in other parts of the protrusions.

In some forms, the filaments in at least part of the nested tufts 632 may remain substantially randomly oriented (rather than aligned), similar to their orientation in the precursor web(s). For example, in some cases, the filaments may remain substantially randomly oriented in the cap of the nested tufts 632, but be more aligned in the side walls such that the filaments extend in the Z-direction (positive or negative depending on the orientation of the nested tuft 632) from the base of the protrusions to the cap. In addition, the alignment of filaments can vary between strata, and can also vary

between different portions of a given nested tufts 632 within the same stratum.

Where the precursor web comprises a nonwoven material, the nested tufts 632 may comprise a plurality filaments that at least substantially surround the sides of the nested tufts 632. This means that there are multiple filaments that extend (e.g., in the positive or negative Z-direction) from the base 650 of the nested tufts 632 to the distal end 654 of the nested tufts 632, and contribute to form a portion of the sides 656 and cap 652 of a nested tufts 632. In some cases, the filaments may be substantially aligned with each other in the Z-direction in the sides 656 of the nested tufts 632. The phrase "substantially surround", thus, does not require that each individual filament be wrapped in the X-Y plane substantially or completely around the sides of the nested tufts 632. If the filaments are located completely around the sides of the nested tufts 632, this would mean that the filaments are located 360° around the nested tufts 632. The nested tufts 632 may be free of large openings at their ends 660. In some cases, the nested tufts 632 may have an opening at only one of their ends, such as at their trailing end.

In some forms, similar-shaped looped filaments may be formed in each stratum of multiple stratum nonwoven materials, including in the stratum 630A that is spaced furthest from the discrete male forming elements during the process of forming the nested tufts 632 therein, and in the stratum 630B that is closest to the male forming elements during the process. In the nested tufts 632, portions of one stratum such as 630B may fit within the other stratum, such as 630A. These strata may be referred to as forming a "nested" structure in the nested tufts 632. Formation of a nested structure may require the use of two (or more) highly extensible nonwoven precursor webs. In the case of two strata materials, nested structures may form two complete loops, or (as shown in some of the following drawing figures) two incomplete loops of filaments.

The nested tufts 632 may have certain additional characteristics. As shown in Figures 8C and 8D, the nested tufts 632 may be substantially hollow. As used herein, the term "substantially hollow" refers to structures which the nested tufts 632 are substantially free of filaments in interior of nested tuft. The term "substantially hollow", does not, however, require that the interior of the nested tuft must be completely free of filaments. Thus, there can be some filaments inside the nested tufts 632. "Substantially hollow" nested tufts are distinguishable from filled three-dimensional structures, such as those made by laying down filaments, such as by airlaying or carding filaments onto a forming structure with recesses therein.

The side walls 656 of the nested tufts 632 can have any suitable configuration. The configuration of the side walls 656, when viewed from the end of the nested tuft such as in 8C, can be linear or curvilinear, or the side walls can be formed by a combination of linear and curvilinear portions. The curvilinear portions can be concave, convex, or combinations of both. For example, the side walls 656 may comprise portions that are curvilinear concave inwardly near the base of the nested tuft and convex outwardly near the cap of the nested tuft. The sidewalls 656 and the area around the base opening 644 of the nested tuft may have significantly lower concentration of filaments per given area (which may be evidence of a lower basis weight or lower opacity) than the portions of the first region 640. The nested tufts 632 may also have thinned filaments in the sidewalls 656. The filament thinning, if present, will be apparent in the form of necked regions in the filaments. Thus, the filaments may have a first cross-sectional area when they are in the undeformed precursor material 102, and a second cross-sectional area in the side walls 656 of the nested tufts 632 of the deformed material web 10, wherein the first cross-sectional area is greater than the second cross-sectional area. The side walls 656 may also comprise some broken filaments as well. In some forms, the side walls 656 may comprise greater than or equal to about 30%, alternatively greater than or equal to about 50% broken filaments.

In some forms, the distal end 654 of the nested tufts 632 may be comprised of original basis weight, non-thinned, and non-broken filaments. If the base opening 644 faces upward, the distal end 654 will be at the bottom of the depression that is formed by the nested tuft. The distal end 654 will be free from apertures formed completely through the distal end. Thus, the nonwoven materials may be nonapertured. The term "apertures", as used herein, refers to holes formed in the nonwovens after the formation of the nonwovens, and does not include the pores typically present in nonwovens. The term "apertures" also does not refer to irregular breaks (or interruptions) in the nonwoven material(s) resulting from localized tearing of the material(s) during the process of forming nested tufts therein, which breaks may be due to variability in the precursor material(s). The distal end 654 may have relatively greater filament concentration in comparison to the remaining portions of the structure that forms the protrusions. The filament concentration can be measured by viewing the sample under a microscope and counting the number of filaments within an area.

The nested tufts 632 may be of any suitable shape. Since the nested tufts 632 are three-dimensional, describing their shape depends on the angle from which they are viewed. When viewed from above (that is, perpendicular to the plane of the web, or plan view) such as in Figure 8A, suitable shapes include, but are not limited to: circular, diamond-shaped, rounded diamond-shaped, U.S. football-shaped, oval-shaped, clover-shaped, heart-shaped, triangle-shaped, teardrop shaped, and elliptical-shaped. In other cases, the nested tufts 632 may be non-circular. The nested tufts 632 may have similar plan view dimensions in all directions, or the nested tufts 632 may be longer in one dimension than another. That is, the nested tufts 632 may have different length and width dimensions. If the nested tufts 632 have a different length than width, the longer dimension will be referred to as the length of the nested tufts 632. The nested tufts 632 may, thus, have a ratio of length to width, or an aspect ratio. The aspect ratios can range from about 1:1 to

about 10:1.

In some forms, the length of the cap 652 may be in a range from about 1.5 mm to about 10 mm. In some forms, the width of the cap (measured where the width is the greatest) may be in a range from about 1.5 mm to about 5 mm. The cap portion of the protrusions may have a plan view surface area of at least about 3 $mm^2$. In some embodiments, the protrusions may have a pre-compression height H that is in a range from about 1 mm to about 10 mm, alternatively from about 1 mm to about 6 mm. In some embodiments, the protrusions may have a post-compression height H that is in a range from about 0.5 mm to about 6 mm, alternatively from about 0.5 mm to about 1.5 mm. In some embodiments, the protrusions may have a depth D, in an uncompressed state that is in a range from about 0.5 mm to about 9 mm, alternatively from about 0.5 mm to about 5 mm. In some forms, the protrusions may have a depth D, after compression that is in a range from about 0.25 mm to about 5 mm, alternatively from about 0.25 mm to about 1 mm.

Methods of forming nested tufts are disclosed in U.S. Patent Application Publication No. 2016/0074256. For the material webs of the present invention, the first stratum may be incorporated into an absorbent article as, for example, an acquisition stratum and the second stratum may be a topsheet of the absorbent article. Each of the first stratum and the second stratum may form nested tufts which fit into one another. Additional forms are contemplated where the material webs of the present invention comprise multiple strata and form the topsheet and are subsequently processed with an acquisition layer.

Forms are contemplated where the material web comprises at least a third stratum in addition to the first stratum 20 and the second stratum 30. In such forms, the first and second strata may be provided with nested tufts prior to the formation of the third strata thereon.

Hybrid Tufts

The material web 10 shown in Figure 44 comprises hybrid tufts 770 each having a corresponding opening 285. Through ablation or other very carefully applied process, material of the first stratum 20 may be removed such that material of the second stratum 30 is exposed through the ends 245 of the material of the first stratum 20. Or, as described previously, the first stratum 20 may be subjected to a process which forms apertures in the first stratum 20. Subsequently, the second stratum 30 may be formed on the first stratum 20.

Additionally, in some forms, material of the second stratum 30 may be urged in the positive Z-direction such that a distal end of the hybrid tuft 770 is generally co-planar with the first surface 50 of the material web 10.

In such forms, the material web 10 may form a portion of a topsheet of an absorbent article. The first stratum 20 may be more hydrophobic than the second stratum.

Outer Tufts, Tunnel Tufts, Filled Tufts, Nested Tufts, Hybrid tufts

Tunnel tufts, filled tufts, outer tufts, nested tufts, and hybrid tufts of material webs of the present invention are thought to mask or partially mask fluid that is collected by the material web remaining in the capillaries between filaments of the tunnel, filled tufts, outer, or nested tufts, as well as masking the liquid that is absorbed in the absorbent layers (which are discolored by the liquids in an undesirable way) under this structure comprising these characteristic differences. Such material webs employed in an absorbent article such as a wipe, a sanitary napkin, a tampon, or a diaper can be appealing to the user (or caregiver) in that potentially unsightly fluids retained in the capillaries between filaments of the various tufts will be obscured or partially obscured from the viewer. The tufts may cover or partially cover interstices in which fluids can be held. Such a feature can make material webs appear less soiled. An additional benefit of the tufts described herein is the soft feel created by the tufts.

Outer, tunnel, filled tufts, nested tufts, hybrid tufts may be spaced apart from adjacent tufts. Each of the spaced apart tufts have generally parallel longitudinal axes L. The number of tufts per unit area of a material web of the present invention, i.e., the area density of tufts and/or caps, can be varied from one tuft per unit area, e.g., square centimeter to as high as 100 tufts per square centimeter. There can be at least 10, or at least 20 tufts per square centimeter, depending on the end use. In general, the area density need not be uniform across the entire area of material webs of the present invention, and, in some embodiments, tufts can be only in certain regions of material webs of the present invention, such as in regions having predetermined shapes, such as lines, stripes, bands, circles, and the like.

The outer, tunnel, filled, nested tufts, and hybrid tufts can impact permeability in zones in the MD and/or CD directions. So certain areas of the material web, particularly where the tufts are disposed, may experience higher permeability as well as have a different texture than the generally planar first surface and/or second surface. Corrugations and grooves, discussed hereafter, may similarly impact the material webs of the present invention regarding permeability and texture.

Additionally, where the material webs of the present invention are incorporated into absorbent articles, the tufts described herein and/or corrugations and grooves may be formed in the material web as well as additional layers of the absorbent article. For example, where the material webs of the present invention are utilized to form a portion of the

topsheet of an absorbent article, the tufts and/or corrugations and grooves may be formed in a subjacent fluid handling layer between the topsheet and an absorbent core in addition to being formed in the material web. In one specific example, the tufts and/or corrugations and grooves may be formed in the material web in conjunction with an acquisition layer. In another specific example, the tufts and/or corrugations and grooves may be formed in the material web in conjunction with a secondary topsheet. In such forms, the fluid handling layer may form the outer tuft which covers the tuft created by the material web. In contrast, forms are contemplated where the fluid handling layer forms the tunnel tuft while the material web forms the outer tuft or simply forms a discontinuity through which the tunnel tuft extends.

The tunnel tufts, filled tufts, outer tufts, hybrid tufts, and/or nested tufts can be used in conjunction with apertures, bond sites, embossments, and/or any of the Z-direction characteristic differences disclosed herein, or may be used independently therefrom.

Corrugations

Yet another way to create characteristic differences in the MD and/or CD is via the utilization of corrugations. The nonwoven web 10 of the present invention may comprise corrugations which on the first surface 50 and the second surface 52. Some exemplary corrugations are shown in Figures 9A-9D. As shown, the material web 10 of the present invention may comprise corrugations 670 and grooves 675 disposed between adjacent corrugations 670. The corrugations 670 can extend in a direction generally parallel to the MD or generally parallel to the CD. The corrugations 670 and/or grooves 675 may comprise any suitable shape. For example, as shown, the corrugations 670 may have an arcuate shape. As another example, the corrugations 670 may comprise a triangular shape. Additionally, examples are contemplated where a material web constructed in accordance with the present invention comprises at least one corrugation having an arcuate shape and one ridge comprising a triangular shape.

The utilization of corrugations 670 may provide softness benefits to the material web 10. Additionally, the material web 10 may have higher permeability in the corrugations 670. Additional details regarding corrugations 670, including suitable processes for forming corrugations 670 can be found in U.S. Patent Nos. 6,458,447; 7,270,861; 8,502,013; 7,954,213; 7,625,363; 8,450,557; and 7,741,235. Additional suitable processes and structures are described in US Patent Application Publication Nos. US2003/018741; US2009/0240222; US2012/0045620; US20120141742; US20120196091; US20120321839; US2013/0022784; US2013/0017370; US2013/013732; US2013/0165883; US2013/0158497; US2013/0280481; US2013/0184665; US2013/0178815; and US2013/0236700. Still additional suitable processes and structures are described with regard to PCT Patent Application Publication Nos. WO2008/156075; WO2010/055699; WO2011/125893; WO2012/137553; WO2013/018846; WO2013/047890; and WO2013/157365.

Referring to Figure 37, in some forms, the material webs of the present invention may comprise corrugations which extend in the MD and CD. As shown, a plurality of corrugations 3770 may comprise distal ends 3754 and sidewalls 3756. Adjacent discreet protrusions 3770 may be separated by grooves 3775 extending in both the MD and CD directions. Distance D1 represents a length of a distal end 3754 of a corrugation 3770 in the MD. Distance D2 is a length of a corrugation in the MD as measured between adjacent grooves 3775. In some forms, D1 may be equal to D2 depending on the formation of the tooling which creates the material web 10. In other forms D2 may be greater than D1.

Distance D3 is a length between adjacent corrugations 3770 in the MD as measured from a plane comprising the distal ends 3754. Distance D3 may be any suitable distance. Distance D6 is a width between adjacent corrugations 3770 in the CD as measured from a plane comprising the distal ends 3754.

Distance D4 is a width of the distal end 3754 of the corrugation 3770 in the CD. Distance D5 is a width of the corrugation in the CD as measured between adjacent grooves 3775. In some forms, D4 may be equal to D5 depending on the formation of the tooling which creates the material web 10. In other forms, D4 may be less than D5.

A suitable apparatus for forming the corrugations 3770 in the material web 10 of the present invention is described in U.S. Patent Application Publication No. 2009/0240222. In such forms, the corrugations may be provided as discrete elements in the MD and CD directions.

Additional configurations for corrugations are contemplated. Some suitable examples of corrugations are disclosed in U.S. Patent Application No. 2004/0137200. As shown in Figure 38, material webs 10 of the present invention may comprise a plurality discrete corrugations 3770.

An additional configuration for the material webs 10 of the present invention is also shown in Figure 39. As shown, in some forms, the corrugations 3770 may extend across the width of the material web 10 in the CD. However, in the grooves 3775 between adjacent corrugations 3770 may be wider than those shown in the prior Figures. Additionally, apertures 3725 may be provided in the grooves 3775. The process for forming such material webs is described in additional detail in U.S. Patent Application Publication No. 2012/0276331.

For each of the material webs 10 shown in Figures 37-39, the processes for forming each of these material web configurations involves the use of intermeshing rolls. In such forms, the resulting corrugations may have localized areas of high caliper and lower caliper and alternating regions of higher and lower basis weight. The higher caliper and higher basis weight regions may be provided at the distal ends 3754 of the corrugations 3770 and in the grooves 3775.

In contrast, the sidewalls 3756 may be provided with lower caliper and lower basis weight.

The utilization of corrugations may be utilized in conjunction with apertures, embossments, bond sites, tufts (all varieties) and/or any of the Z-direction characteristic differences disclosed herein, or may be used independently thereof.

## Zones

The MD and/or CD characteristic differences discussed herein, e.g. apertures, bond sites, embossing, tunnel tufts, filled tufts, nested tufts, corrugations, and/or grooves, may be provided in zones in order to create additional characteristic differences in the MD and/or CD of the material web. The zones in material webs of the present invention may be positioned in the machine direction, the cross direction, or may be concentric. If a product, such as an absorbent article, has two different zones in the machine direction, the zones may have the same or a similar cross-direction width (e.g., +/- 2mm) for ease in processing. One or more of the zones may have curved or straight boundaries or partial boundaries.

Any suitable number of zones, including more than two, of different or the same zones for a material web are envisioned within the scope of the present disclosure. The various zones may be in the topsheet as mentioned above, but may also be present on an outer cover or a cuff for example. In some instances, the same or a different pattern of zones of material webs may be used on the wearer-facing surface (e.g., topsheet) and the garment-facing surface (e.g., outer cover).

In one example, a topsheet or other portion of an absorbent article may have two or more zones in a material web. For example, a first zone of the material web may have a different discontinuity than a second zone. The first zone and the second zone may have different functionalities owing to the different discontinuities. A functionality of the first zone may be to provide liquid bodily exudate distribution (fluid moving on the material web), while the functionality of the second zone may be to provide liquid bodily exudate acquisition (fluid penetrating the material web). Benefits of such a zoned material webs can be better use of an absorbent core and more efficient liquid bodily exudate distribution within the absorbent core. This is especially important if an air-felt free core is used in that typical air-felt free cores somewhat struggle with liquid bodily exudate distribution once the liquid bodily exudate is received therein.

As stated previously, the material webs of the present invention may be utilized in a number of different components of absorbent articles. Referring to Figure 10, in one specific example, disposable absorbent articles utilizing the material webs of the present invention may comprise a plurality of zones. As shown, a topsheet 2014 of a disposable absorbent article 2010, may comprise a first zone 2007, a second zone 2011 and a third zone 2013. Absorbent articles may comprise more zones or less zones as described hereafter.

The first zone 2007 may comprise a first plurality of discontinuities, e.g. apertures. As shown the first zone 2007 may have a width parallel to a lateral axis 2090 which does not extend the full width of the topsheet 2014. Instead, the second zone 2011 and the third zone 2013 may be placed on either side of the first zone 2007. In some forms, the second zone 2011 and the third zone 2013 may comprise a second plurality of discontinuities. In some forms, the first plurality of discontinuities may be different than the second plurality of discontinuities. For example, the first plurality of discontinuities may comprise apertures while the second and third zones comprise tunnel tufts. Additional forms are contemplated where the first zone 2007, the second zone 2011, and/or the third zone 2013 may comprise additional pluralities of discontinuities. For example, the first zone 2007 may comprise a plurality of apertures and a plurality of bond sites. As another example, the second zone 2011and/or third zone 2013 may comprise a plurality of tunnel tufts and a plurality of bond sites. Additional pluralities of discontinuities are contemplated. For example, the first zone 2007, second zone 2011, and/or third zone 2013 may additionally comprise a plurality of embossments.

Suitable configurations of zones are described with regard to Figures 11-14. Figures 11-14 may represent a portion of a wearer-facing surface of an absorbent article, such as a diaper, an adult incontinence product, and/or a sanitary napkin.

Figure 11 illustrates an example of a substrate having three zones. The front portion, F, may be positioned in a front portion of an absorbent article or a back portion of an absorbent article. The back portion, B, may be positioned in a front portion of an absorbent article or a back portion of an absorbent article. A first zone 4004 and a second zone 4006 may be positioned intermediate two portions of the third zone 4008. The first zone 4004 may comprise a first plurality of discontinuities as described above. The second zone 4006 may comprise a second plurality of discontinuities. In some forms, the first plurality of discontinuities may be different than the second plurality of discontinuities. As shown, a substantially-laterally extending separation element, 4010, may extend between the intersection of the first zone 4004 and the second zone 4006.

In another instance, still referring to Figure 11, the first zone 4004 may comprise a pattern of discontinuities, e.g. apertures, wherein at least two apertures of the pattern of apertures have different sizes, shapes, and/or orientations. The pattern of apertures may be any of the various patterns described herein or other suitable patterns. The second zone 4006 may comprise a pattern of apertures, wherein at least two apertures of the pattern of apertures have different

sizes, shapes, and/or orientations. The pattern of apertures may be any of the various patterns described herein or other suitable patterns. The second zone 4006 may have a different or the same pattern of apertures as the first zone 4004. The third zone 4008 may comprise a plurality of discontinuities. The out-of-plane deformations may extend upwardly out of the page or downwardly into the page. A substantially-laterally extending separation element, 4010, may extend between the intersection of the first zone 4004 and the second zone 4006.

Figure 12 illustrates an example of a substrate having a first zone 4012 and a second zone 4014. The front portion, F, may be positioned in a front portion of an absorbent article or a back portion of an absorbent article. The back portion, B, may be positioned in a front portion of an absorbent article or a back portion of an absorbent article. The first zone 4012 may comprise a pattern of apertures, wherein at least two apertures of the pattern of apertures have different sizes, shapes, and/or orientations. The pattern of apertures may be any of the various patterns described herein or other suitable patterns. The second zone 4014 may comprise a plurality of discontinuities. Substantially-laterally extending separation element 4010, may extend between the intersection of the first zone 4012 and the second zone 4014.

In another instance, still referring to Figure 12, the second zone 4014 may comprise a pattern of apertures, wherein at least two apertures of the pattern of apertures have different sizes, shapes, and/or orientations. The pattern of apertures may be any of the various patterns described herein or other suitable patterns. The first zone 4012 may comprise a plurality of discontinuities. A substantially-laterally extending separation element, 4010, may extend between the intersection of the first zone 4012 and the second zone 4014.

Figure 13 illustrates an example of a material web having a first zone 4016 and a second zone 4018. The front portion, F, may be positioned in a front portion of an absorbent article or a back portion of an absorbent article. The back portion, B, may be positioned in a front portion of an absorbent article or a back portion of an absorbent article. The second zone 4018 may at least partially, or fully, surround the first zone 4016.

Still referring to Figure 13, the first zone 4016 may comprise a plurality of discontinuities. The second zone 4018 may comprise a plurality of discontinuities. The second zone 4018 may have a different or the same pattern, shape, size, and/or orientation of the discontinuities compared to the pattern, shape, size, and/or orientation of the discontinuities of the first zone 4016.

In another instance, still referring to Figure 13, the first zone 4016 may comprise a pattern of apertures, wherein at least two apertures of the pattern of apertures have different sizes, shapes, and/or orientations. The pattern of apertures may be any of the various patterns described herein or other suitable patterns. The second zone 4018 may comprise a plurality of discontinuities.

In yet another instance, still referring to Figure 13, the second zone 4018 may comprise a pattern of apertures, wherein at least two apertures of the pattern of apertures have different sizes, shapes, and/or orientations. The first zone 4016 may comprise a plurality of discontinuities.

In another instance, still referring to Figure 13, the first zone 4016 may comprise a pattern of apertures, wherein at least two apertures of the pattern of apertures have different sizes, shapes, and/or orientations. The pattern of apertures may be any of the various patterns described herein or other suitable patterns. The second zone 4018 may comprise a pattern of apertures, wherein at least two apertures of the pattern of apertures have different sizes, shapes, and/or orientations. The pattern of apertures may be any of the various patterns described herein or other suitable patterns. The patterns of apertures of the first zone 4016 and the second zone 4018 may be different or the same.

Figure 14 illustrates an example of a material web having a first zone 4020 and a second zone 4022. The front portion, F, may be positioned in a front portion of an absorbent article or a back portion of an absorbent article. The back portion, B, may be positioned in a front portion of an absorbent article or a back portion of an absorbent article. The second zone 4022 may at least partially, or fully, surround the first zone 4020.

Still referring to Figure 14, the first zone 4020 may comprise a pattern of apertures, wherein at least two apertures of the pattern of apertures have different sizes, shapes, and/or orientations. The second zone 4022 may comprise a pattern of apertures, wherein at least two apertures of the pattern of apertures have different sizes, shapes, and/or orientations. The pattern of apertures may be any suitable pattern. The patterns of apertures of the first zone 4020 and the second zone 4022 may be different or the same.

Still referring to Figure 14, the first zone 4020 may comprise a pattern of apertures, wherein at least two apertures of the pattern of apertures have different sizes, shapes, and/or orientations. The pattern of apertures may be any of the various patterns or other suitable patterns. The second zone 4022 may comprise a plurality of discontinuities.

Still referring to Figure 14, the second zone 4022 may comprise a pattern of apertures, wherein at least two apertures of the pattern of apertures have different sizes, shapes, and/or orientations. The pattern of apertures may be any suitable pattern. The first zone 4020 may comprise a plurality of discontinuities.

Still referring to Figure 14, the first zone 4020 may comprise a plurality of discontinuities. The second zone 4022 may comprise a plurality of discontinuities. The second zone 4022 may have a different or the same pattern, shape, size, and/or orientation of the discontinuities compared to the pattern, shape, size, and/or orientation of the discontinuities of the first zone 4020.

Patterned apertures and patterned discontinuities are disclosed in further detail in U.S. Patent Application Serial

Nos. 14/933,028; 14/933,017; and 14/933,001. The discontinuities described herein may be configured in any suitable manner to achieve the desired acquisition, rewet, and softness properties desired for the material web. And as noted previously, the discontinuities may be utilized in conjunction with the Z-direction gradient filament characteristics to similarly achieve the desired acquisition, rewet, and/or softness desired for the material webs of the present invention.

## Examples

### Example 1

A 25 gsm (gram/$m^2$)nonwoven web (about 12.5 gsm in the first spinbeam and about 12.5 gsm in the second spinbeam) of the present invention was produced on a 1 meter wide pilot line at Reifenhauser, GmbH in Troisdorf, Germany. In the first spinbeam comprising 12.5gsm, about 20.5 micron diameter, 60/40 side-by-side $PP_1/PP_2$ filaments were spun. Both components additionally comprised 16% Techmer PPM17000 High Load (40%) Hydrophobic masterbatch, and the second component comprised 1.5% of TiO2 masterbatch. In the second spin beam, about 18 micron diameter, 70/30 side/side $PP_1/PP_2$ filaments were spun. Both components from the second spin beam additionally comprised 2.0% Techmer™ PPM15560 hydrophilic masterbatch, and the first component additionally comprised 1.0% of TiO2 masterbatch. The first stratum and the second stratum were calendar bonded with a circular dot bond pattern having 12% bond area.

Figures 15A-15C are SEM photos depicting the first stratum 20 and the second stratum 30. Figure 15A is an SEM photo of a portion of the first plurality of filaments of the first stratum 20, and Figure 15B is an SEM photo of a portion of the second plurality of filaments of the second stratum 30. As shown, the hydrophobic melt additive of the first plurality of filaments appears as a combination of a plurality of fibrils.

### Example 2, 3, 4, and 5

For each of Examples 2, 3, 4, and 5, all materials were produced on 1 meter wide pilot line at Reicofil, in Troisdorf, Germany, and each comprises 2 denier per filament 70/30 side-by-side filaments of PP1/ PP2.

### Example 2

A material web in accordance with the present disclosure was created having a basis weight of 40 gsm with about 50 percent comprised by the first stratum and about 50 percent comprised by the second stratum. The nonwoven web was produced from two spinbeams. The first stratum comprised hydrophobic filaments where the first polypropylene component of the first plurality of filaments comprised 16 weight percent PPM1700 High Load Hydrophobic masterbatch from Techmer™ and 1 weight percent TiO2 masterbatch. The second stratum comprised hydrophilic filaments where both the first polypropylene component and the second polypropylene component additionally comprised 2 weight percent PPM 15560 hydrophilic masterbatch from Techmer™. The first polypropylene component additionally comprised 1 weight percent masterbatch.

### Example 3

A laminate comprising a pair of spunbond hydrophobic webs where each of the spunbond hydrophobic webs was produced at a total basis weight of 25 gsm. The two hydrophobic webs each comprised the same filament size and filament composition. In the filaments, both first polypropylene component and the second polypropylene component additionally comprised 16 weight percent PPM1700 high Load Hydrophobic masterbatch from Techmer. The first polypropylene component additionally comprised 1 weight % TiO2 masterbatch.

### Example 4

A laminate comprising a pair of spunbond hydrophilic webs where each of the spunbond hydrophilic webs was produced at a total basis weight of 25 gsm. The two hydrophilic webs each comprised the same filament size and filament composition. In the filaments, both the first polypropylene component and the second polypropylene component additionally comprised 2 weight % PPM 15560 hydrophilic masterbatch from Techmer. The first polypropylene component additionally comprised 1 weight % TiO2 masterbatch.

### Example 5

A nonwoven laminate was produced comprising one of the pair of nonwoven webs of Example 3 (25 gsm) and

one of the pair of nonwoven webs of Example 4 (25 gsm) thereby producing a 50 gsm laminate comprising a hydrophobic web over a hydrophilic web.

Data 1 - Unmodified Webs / Laminates

Table 1 shows basis weight, rewet, and acquisition data for Examples 2, 3, 4, and 5. None of the Examples comprised apertures or any other disruption as described herein.

Table 1

| **Material** | **Total Basis Weight** *(gsm)* | **Rewet** *(grams)* | **Gush Acquisition Time** *(sec)* |
|---|---|---|---|
| Example 2 | 40 | 0.45 | 803 |
| Example 5 | 50 | 0.36 | 790 |
| Example 3 | 50 | 0.21 | 803 |
| Example 4 | 50 | 0.67 | 801 |

As shown, the unmodified nonwoven web of the present invention (Example 2) performed better than the hydrophilic laminate (Example 4) from the standpoint of rewet. The acquisition time of Example 2 was on par with that of the hydrophobic laminate (Example 3).

Data 2 - Apertured Webs / Laminates

The above Examples were apertured as described herein. Photographs of Example 2 versus Example 5 are provided with regard to Figures 16A-16B, respectively. Both the material web 10 and the nonwoven laminate 1100 comprise apertures 125 as disclosed herein.

Table 2 shows basis weight, rewet, and acquisition data for Examples 2, 3, 4, and 5. Each of the Examples comprised apertures as described herein. Apertures sizes were about 2.5 mm in length and about 0.3 to about 0.35 mm in width.

Table 2

| **Material** | **Total Basis Weight** *(gsm)* | **Rewet** *(grams)* | **Gush Acquisition Time** *(sec)* |
|---|---|---|---|
| Example 2 - apertured | 40 | 0.53 | 235 |
| Example 5 - apertured | 50 | 0.15 | 226 |
| Example 3 - apertured | 50 | 0.32 | 501 |
| Example 4 - apertured | 50 | 1.04 | 134 |

As shown, the material web of the present invention (Example 2) performed better than the hydrophilic laminate (Example 4) from the standpoint of rewet. The acquisition time of Example 2 was much better with than that of the hydrophobic laminate (Example 3). The acquisition time of Example 2 was similar to the acquisition time of Example 5. And, as mentioned above, apertures can impact the acquisition speed of a material web. Without wishing to be bound by theory, it is believed that there may be a balance between acquisition and rewet. Although the apertures decreased the acquisition time, which can be desirable in absorbent articles, the addition of apertures may increase the rewet from the non-apertured version of Example 2.

Data 3 Tufted Webs / Laminates

The above Examples were tufted as described herein. Each of the examples comprised tufts. SEM photographs of Example 2 versus Example 5 are provided with regard to Figures 17A-17B, respectively. Both the material web 10 and the nonwoven laminate 1100 comprise tufts 270 as disclosed herein.

Table 3 shows basis weight, rewet, and acquisition data for Examples 2, 3, 4, and 5. Each of the Examples comprised apertures as described herein.

Table 3

| Material | **Total Basis Weight** *(gsm)* | **Rewet** *(grams)* | **Gush Acquisition Time** *(sec)* |
|---|---|---|---|
| Example 2 - tufted | 40 | 0.33 | 227 |
| Example 5 - tufted | 50 | 0.34 | 178 |
| Example 3 - tufted | 50 | 0.47 | 312 |
| Example 4 - tufted | 50 | 0.85 | 92 |

As shown, the material web wherein one stratum comprises a hydrophobic melt additive and another stratum comprises a hydrophilic melt additive (Example 2) performed better than the hydrophilic laminate (Example 4) and the hydrophobic laminate (Example 3) from the standpoint of rewet. The acquisition time of Example 2 was much better with than that of the hydrophobic laminate (Example 3). The acquisition time of Example 2 was similar to the acquisition time of Example 5. As such, contrary to conventional wisdom, a single layer topsheet may function adequately from an acquisition and rewet standpoint. Whether apertured or tufted, the modified examples provided faster liquid acquisition speed versus their unmodified counterparts. Additionally, as shown in Table 3, the material web of the present invention can provide reduced rewet.

The above data demonstrates that when the Z-direction characteristic differences, MD and/or CD characteristic differences are utilized, e.g. apertures, tufts, the material webs of the present invention may perform on par with the laminate produced from two separate webs (Example 5).

Additional benefits of the material webs of the present invention include the integral formation of the first stratum and the second stratum. This integral formation can facilitate production of absorbent articles which include the material webs of the present invention. In contrast, because laminates comprise separate nonwoven layers, additional equipment is required to form the laminate. For example, equipment is required to provide the two separate layers to a converting process. For high speed manufacturing, care must be taken to ensure that the two constituent layers of the laminate track within desired tolerances. The material webs of the present invention, however, as mentioned previously, are integrally formed. As such, the additional equipment required for formation of the nonwoven laminates is not required for the material webs of the present invention.

## Additional Contemplated Examples

Figures 40 and 41 illustrate a cross-sectional view of a spunbond-meltblown-spunbond (SMS) web at a calender bond site 4068 and a cut-away view of this web, respectively in accordance with the present disclosure. A three stratum material web 4012 is illustrated that was produced by processes described herein. The material web 4012 may comprise a first nonwoven component stratum 4020 which itself may be comprised of spunbond fibers, for example. The material web 4012 may comprise a second nonwoven component stratum 4025 which itself may be comprised of meltblown fibers. The meltblown stratum may comprise intermediate diameter fibers which may comprise fibers having an average diameter, alternatively number-average diameter, in the range of 0.7 microns to 8 microns, alternatively in the range of 1 micron to 8 microns, and, alternatively, in the range of 1 micron to 5 microns, with a relative standard deviation in the range of 20% to over 100%. The material web 4012 may comprise a third nonwoven stratum 4030 which itself is comprised of spunbond fibers. In some forms, the first stratum 4020 and the third stratum 4030 may be similar, or in other forms, the first stratum 4020 and the third stratum may be different as described herein.

Referring to Figures 42 and 43, a material web 4200 is depicted. As shown, in some forms of the present invention, a material web 4200 may comprise a first nonwoven component stratum 4020 comprising fibers having an average diameter in the range of 8 microns to 30 microns, a second nonwoven component stratum 4025 comprising fibers having a number average diameter of less than 1 micron, a mass-average diameter of less than 1.5 micron, and a polydispersity ratio less than 2, a third nonwoven component stratum 4027 comprising fibers having an average diameter in the range of 8 microns to 30 microns, and a fourth nonwoven component stratum 4030 comprising fibers having an average diameter in the range of 1 micron to 8 microns. Stated another way, the web of material 4200 may comprise the first nonwoven stratum 4020 comprising fibers having an average denier in the range of 0.4 to 6, the second nonwoven component stratum 4025 comprising fibers having an average denier in the range of 0.00006 to 0.006, a third nonwoven stratum 4027 comprising fibers having an average denier in the range of 0.4 to 6, and a fourth nonwoven stratum 4030 comprising fibers having an average denier in the range of 0.006 to 0.4. In such forms, the second nonwoven stratum 4025 and the fourth nonwoven component stratum 4030 may be disposed intermediate the first nonwoven component stratum 4020 and the third nonwoven component stratum 4027. Also, the first nonwoven component stratum 4020, the second nonwoven stratum 4025, the third nonwoven component stratum 4027, and the fourth nonwoven component stratum 4030 may be intermittently bonded to each other using any bonding process, such as a calendering

bonding process, for example.

In various forms, the material webs of the present invention may comprise a spunbond stratum, which may correspond to the first nonwoven component stratum 4020, a meltblown stratum, which may correspond to the second nonwoven component stratum 4025, an nano-fiber stratum, which may correspond to the third nonwoven component stratum 4027 and a second spunbond stratum, which may correspond to the fourth nonwoven component stratum 4030, together referred to herein as an "SMNS web." Additional configurations are contemplated. Some examples include a material web which comprises a spunbond stratum, a meltblown stratum, an N-fiber stratum, a second spunbond stratum, and a third spunbond stratum of different structure or composition, for example.

Without wishing to be bound by theory, it is believed that the inclusion of the N-fiber stratum within the webs allows the webs to maintain a desirable low surface tension fluid strikethrough time and air permeability without any hydrophobic materials. It is further believed that the N-fiber stratum reduces the pore size of the webs by filing in voids within the spunbond and meltblown strata. By creating webs with smaller pore sizes when compared to the pore sizes of related webs, the webs of the present disclosure may have higher capillary drag forces to fluid penetration and, thereby, a longer low surface tension fluid strikethrough time, even without comprising a hydrophobic material.

## Methods for Joining the Strata

The first stratum and the second stratum can be joined together by any suitable method. To start with, the filaments from the individual spin beams (or also meltblowing beams) are becoming somewhat entangled in the laydown of the filaments onto the already laid down stratum or strata. And as noted previously, the nonwoven strata may be bonded together via primary bond sites. Typically, the primary bond sites are thermal point bonds fusing or compressing all stata of the material web together in discrete areas forming film-like discrete primary bond sites. These primary bond sites are excluded from the MD and/or CD characteristic differences and/or Z-direction differences described herein.

Some suitable examples of more intimate and strong bonding include calendar bonding or thermal point bonding (with a selection from various possible or multiple patterns), through-air bonded, hydroentangled, and the like, each of which is well known in the art, or a combination of those. Another suitable example includes needlepunching which is well known in the art. Additionally, the attachment of the first nonwoven stratum to the second nonwoven stratum may be achieved by a variety of different processes.

For those material webs of the present invention for which filled tufts are desired, the percentage of bond area between the first stratum and the second stratum should be carefully considered. The inventors have found that with curled filaments, too low of a calendar bond area does not allow for good formation of filled tufts and outer tufts, which is opposite conventional wisdom in that lower bond area is usually considered favorable for texturing of a spunbond web. Also, too low of a calendar bond area yields a material web with low strength and poor abrasion resistance. However, too high of a calendar bond area reduces the length of filaments between adjacent bonds which inhibits the amount of uncoiling and/or displacement possible. Specifically, too high of a calendar bond area inhibits the movement of the filaments such that when subjected to the localized Z-direction urging, described herein for the formation of filled tufts and outer tufts, the curled filaments have very limited ability to uncoil. In such configurations, the curled filaments must undergo plastic deformation or break once the amount of uncoiling surpasses the amount of applied process strain. The inventors have found that calendar bond area above about 10 percent and less than about 18 percent allows for a good balance of filament mobility and free filament length available for uncoiling but still provides sufficient strength in the material web for manipulations of the curled filaments as well as abrasion and tearing resistance in use.

In some forms of the present invention, the material webs comprising curled filaments may comprise a calendar bond area of between about 10 percent to about 18 percent or between about 12 percent and 16 percent, specifically including all values within these ranges or any range created thereby. Material webs of the present invention which do not include curled filaments may comprise a calendar bond area of between about 5 percent to about 30 percent, between about 10 percent to about 20 percent, specifically including all values within these ranges and any ranges created thereby. The bonds can be shaped like dots, diamonds, ovals or any other suitable shape and may be arranged in any suitable pattern to provide the desired mechanical properties.

## Disposable Absorbent Articles

As noted heretofore, the material webs of the present invention may comprise any suitable portion of a disposable absorbent article. Some examples, include topsheet, backsheet, barrier cuff, intermediate layers between the topsheet and an absorbent core and/or intermediate layers between the backsheet and the absorbent core.

Referring to Figure 33, an absorbent article 1710 which may utilize the material webs described herein may be a sanitary napkin / feminine hygiene pad. As shown, the sanitary napkin 1710 may comprise a chassis comprising a liquid permeable topsheet 1714, a liquid impermeable, or substantially liquid impermeable, backsheet 1716, and an absorbent core 1718 positioned intermediate the topsheet 1714 and the backsheet 1716. The sanitary napkin 1710 may

comprise wings 1720 extending outwardly with respect to a longitudinal axis 1780 of the sanitary napkin 1710. The sanitary napkin 1710 may also comprise a lateral axis 1790. The wings 1720 may be joined to the topsheet 1714, the backsheet 1716, and/or the absorbent core 1718. The sanitary napkin 1710 may also comprise a front edge 1722, a rear edge 1724 longitudinally opposing the front edge 1722, a first side edge 1726, and a second side edge 1728 laterally opposing the first side edge 1726. The longitudinal axis 1780 may extend from a midpoint of the front edge 1722 to a midpoint of the rear edge 1724. The lateral axis 1790 may extend from a midpoint of the first side edge 1728 to a midpoint of the second side edge 1728. The sanitary napkin1710 may also be provided with additional features commonly found in sanitary napkins as is known in the art. In some forms of the present invention, the wings may be provided with zones of extensibility as described in U.S. Patent No. 5,972,806.

Any suitable absorbent core known in the art may be utilized. The absorbent core1718 may be any absorbent member which is generally compressible, conformable, non-irritating to the wearer’s skin, and capable of absorbing and retaining liquids such as urine, menses, and/or other body exudates. The absorbent core1718 may be manufactured from a wide variety of liquid-absorbent materials commonly used in disposable absorbent articles such as comminuted wood pulp which is generally referred to as airfelt. The absorbent core 1718 may comprise superabsorbent polymers (SAP) and less than 15%, less than 10%, less than 5%, less than 3%, or less than 1% of airfelt, or be completely free of airfelt. Examples of other suitable absorbent materials comprise creped cellulose wadding, meltblown polymers including coform, chemically stiffened, modified or cross-linked cellulosic fibers, tissue including tissue wraps and tissue laminates, absorbent foams, absorbent sponges, superabsorbent polymers, absorbent gelling materials, or any equivalent material or combinations of materials.

The configuration and construction of the absorbent core 1718 may vary (e.g., the absorbent core may have varying caliper zones, a hydrophilic gradient, a superabsorbent gradient, or lower average density and lower average basis weight acquisition zones; or may comprise one or more layers or structures). In some forms, the absorbent core 1718 may comprise one or more channels, such as two, three, four, five, or six channels.

The absorbent core1718 of the present disclosure may comprise one or more adhesives, for example, to help immobilize the SAP or other absorbent materials within a core wrap and/or to ensure integrity of the core wrap, in particular when the core wrap is made of two or more substrates. The core wrap may extend to a larger area than required for containing the absorbent material(s) within.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen.

Other forms and more details regarding channels and pockets that are free of, or substantially free of absorbent materials, such as SAP, within absorbent cores are discussed in greater detail in U.S. Patent Application Publication Nos. 2014/0163500, 2014/0163506, and 2014/0163511, all published on June 12, 2014.

The absorbent article 1710 may comprise additional layers between the topsheet 1714 and the absorbent core 1718. For example, the absorbent article 1710 may comprise a secondary topsheet and/or an acquisition layer positioned between the topsheet 1714 and the absorbent core 1718.

The backsheet can comprise a liquid impervious film. The backsheet can be impervious to liquids (e.g., body fluids) and can be typically manufactured from a thin plastic film. However, typically the backsheet can permit vapours to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 $g/m^2$ to about 35 $g/m^2$. However, it should be noted that other flexible liquid impervious materials may be used as the backsheet. Herein, "flexible" refers to materials which are compliant and which will readily conform to the general shape and contours of the wearer’s body.

The backsheet can be typically positioned adjacent an outer-facing surface of the absorbent core and can be joined thereto by any suitable attachment device known in the art. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment device may include heat bonds, thermal fusion bonds, pressure bonds, ultrasonic bonds, dynamic mechanical bonds, or any other suitable attachment device or combinations of these attachment devices. The backsheet may be additionally secured

to the topsheet by any of the above-cited attachment devices / methods.

Still another example of a disposable absorbent article which may utilize the material webs of the present invention are diapers which include non-refastenable pants and/or refastenable diapers. Diapers have can have a similar construction to that of sanitary napkins. An exemplary diaper is described below.

Referring to Figure 34, a plan view of an example absorbent article that is a diaper 1900 in its flat-out, uncontracted state (i.e., with elastic induced contraction pulled out) with portions of the structure being cut-away to more clearly show the construction of the diaper 1900 and with its wearer-facing surface toward the viewer. This diaper is shown for illustration purpose only as the present disclosure may be used for making a wide variety of diapers and other absorbent articles.

The absorbent article may comprise a liquid permeable topsheet 1924, a liquid impermeable backsheet 1925, an absorbent core 1928 positioned at least partially intermediate the topsheet 1924 and the backsheet 1925, and barrier leg cuffs 1934. The absorbent article may also comprise a liquid management system ("LMS") 1950 (shown in Figure 35), which, in the example represented, comprises a distribution layer 1954 and an acquisition layer 1952 that will both be further discussed below. In various forms, the acquisition layer 1952 may instead distribute bodily exudates and the distribution layer 1954 may instead acquire bodily exudates or both layers may distribute and/or acquire bodily exudates. The LMS 1950 may also be provided as a single layer or two or more layers. The absorbent article may also comprise elasticized gasketing cuffs 1932 joined to the chassis of the absorbent article, typically via the topsheet and/or backsheet, and substantially planar with the chassis of the diaper.

The Figures also show typical taped diaper components such as a fastening system comprising adhesive tabs 1942 or other mechanical fasteners attached towards the rear edge of the absorbent article 1920 and cooperating with a landing zone 1944 on the front of the absorbent article 1920. The absorbent article may also comprise other typical elements, which are not represented, such as a rear elastic waist feature and a front elastic waist feature, for example.

The absorbent article 1920 may comprise a front waist edge 1910, a rear waist edge 1912 longitudinally opposing the front waist edge 1910, a first side edge 1903, and a second side edge 1904 laterally opposing the first side edge 1903. The front waist edge 1910 is the edge of the absorbent article 1920 which is intended to be placed towards the front of the user when worn, and the rear waist edge 1912 is the opposite edge. Together the front waist edge 1910 and the rear waist edge form waist opening when the absorbent article 1920 is donned on a wearer. The absorbent article 1920 may have a longitudinal axis 1980 extending from the lateral midpoint of the front waist edge 1910 to a lateral midpoint of the rear waist edge 1912 of the absorbent article 1920 and dividing the absorbent article 1920 in two substantially symmetrical halves relative to the longitudinal axis 1980, with article placed flat and viewed from the wearer-facing surface as illustrated Figure 34. The absorbent article may also have a lateral axis 1990 extending from the longitudinal midpoint of the first side edge 1903 to the longitudinal midpoint of the second side edge 1904. The length L of the absorbent article 1920 may be measured along the longitudinal axis 1980 from the front waist edge 1910 to the rear waist edge 1912. The crotch width of the absorbent article 1920 may be measured along the lateral axis 1990 from the first side edge 1903 to the second side edge 1904. The absorbent article 1920 may comprise a front waist region 1905, a rear waist region 1906, and a crotch region 1907. The front waist region, the rear waist region, and the crotch region each define 1/3 of the longitudinal length of the absorbent article. Front and back portions may also be defined on opposite sides of the lateral axis 1990.

The topsheet 1924, the backsheet 1925, the absorbent core 1928, and the other article components may be assembled in a variety of configurations, in particular by gluing or heat embossing, for example. Example diaper configurations are described generally in U.S. Pat. No. 3,860,003, U.S. Pat. No. 5,221,274, U.S. Pat. No. 5,554,145, U.S. Pat. No. 5,569,234, U.S. Pat. No. 5,580,411, and U.S. Pat. No. 6,004,306.

The absorbent core 1928 may comprise an absorbent material comprising 75% to 100%, at least 80%, at least 85%, at least 90%, at least 95%, or at least 99%, all by weight, of the absorbent material, specifically reciting all 0.1% increments within the above-specified ranges and all ranges formed therein or thereby, and a core wrap enclosing the absorbent material. The core wrap may typically comprise two materials, substrates, or nonwoven materials 16 and 16' for the top side and bottom side of the core.

The absorbent core 1928 may comprises one or more channels, represented in Figure 34 as the four channels 1926, 1926' and 1927, 1927'. Additionally or alternative, the LMS 1950 may comprises one or more channels, represented in Figures 34-36 as channels 1949, 1949'. In some forms, the channels of the LMS 1950 may be positioned within the absorbent article 1920 such they aligned with, substantially aligned with, overlap, or at least partially overlap, the channels of the absorbent core 1928. These and other components of the absorbent articles will now be discussed in more details.

The topsheet 1924 is the part of the absorbent article that is directly in contact with the wearer's skin. The topsheet 1924 may be joined to the backsheet 1925, the core 1928 and/or any other layers as is known to those of skill in the art. Usually, the topsheet 1924 and the backsheet 1925 are joined directly to each other in some locations (e.g., on or close to the periphery of the article) and are indirectly joined together in other locations by directly joining them to one or more other elements of the absorbent article 1920.

The backsheet 1925 is generally that portion of the absorbent article 1920 positioned adjacent the garment-facing surface of the absorbent core 1928 and which prevents, or at least inhibits, the bodily exudates absorbed and contained therein from soiling articles such as bedsheets and undergarments. The backsheet 1925 is typically impermeable, or at least substantially impermeable, to liquids (e.g., urine, running BM), but permeable to vapors to allow the diaper to "breath". The backsheet may, for example, be or comprise a thin plastic film such as a thermoplastic film having a thickness of about 0.012 mm to about 0.051 mm. Example backsheet films include those manufactured by Tredegar Corporation, based in Richmond, VA, and sold under the trade name CPC2 film. Other suitable backsheet materials may include breathable materials which permit vapors to escape from the absorbent article 1920 while still preventing, or at least inhibiting, bodily exudates from passing through the backsheet 1925. Example breathable materials may include materials such as woven webs, nonwoven webs, and composite materials such as film-coated nonwoven webs, microporous films, and monolithic films.

The backsheet 1925 may be joined to the topsheet 1924, the absorbent core 1928, and/or any other element of the absorbent article 1920 by any attachment methods known to those of skill in the art. Suitable attachment methods are described above with respect to methods for joining the topsheet 1924 to other elements of the absorbent article 1920.

As used herein, the term "absorbent core" refers to the individual component of the absorbent article having the most absorbent capacity and that comprises an absorbent material. The absorbent core may comprise a core wrap or core bag (hereafter "core wrap") enclosing the absorbent material. The term "absorbent core" does not include the LMS or any other component of the absorbent article which is not either integral part of the core wrap or placed within the core wrap. The absorbent core may comprise, consist essentially of, or consist of, a core wrap, absorbent material as defined below, and glue enclosed within the core wrap. Pulp or air-felt may also be present within the core wrap and may form a portion of the absorbent material. The absorbent core periphery, which may be the periphery of the core wrap, may define any suitable shape, such as a "T," "Y," "hour-glass," or "dog-bone" shape, for example. An absorbent core periphery having a generally "dog bone" or "hour-glass" shape may taper along its width towards the middle or "crotch" region of the core. In this way, the absorbent core may have a relatively narrow width in an area of the absorbent core intended to be placed in the crotch region of an absorbent article.

The absorbent core 1928 of the present disclosure may comprise an absorbent material with a high amount of superabsorbent polymers (herein abbreviated as "SAP") enclosed within a core wrap. The SAP content may represent 70% to 100% or at least 70%, 75%, 80%, 85%, 90%, 95%, 99%, or 100% by weight of the absorbent material contained in the core wrap. The SAP useful with the present disclosure may include a variety of water-insoluble, but water-swellable polymers capable of absorbing large quantities of fluids. The core wrap is not considered as absorbent material for the purpose of assessing the percentage of SAP in the absorbent core. The remainder of the absorbent material in the core 1928 may be air-felt.

"Absorbent material" means a material which has some absorbency property or liquid retaining properties, such as SAP, cellulosic fibers as well as synthetic fibers. Typically, glues used in making absorbent cores have no absorbency properties and are not considered as absorbent material. The SAP content may be higher than 80%, for example at least 85%, at least 90%, at least 95%, at least 99%, and even up to and including 100% of the weight of the absorbent material contained within the core wrap, as stated above. This provides a relatively thin core compared to conventional cores typically comprising between 40-60% SAP, for example, and high content of cellulose fibers or airfelt. The absorbent material may comprise less than 15% or less than 10% weight percent of natural or synthetic fibers, less than 5% weight percent, less than 3% weight percent, less than 2% weight percent, less than 1% weight percent, or may even be substantially free of, or free of, natural and/or synthetic fibers, specifically reciting all 0.1% increments within the specified ranges and all ranges formed therein or thereby. The absorbent material may comprise little or no airfelt (cellulose) fibers, in particular the absorbent core may comprise less than 15%, 10%, 5%, 3%, 2%, 1% airfelt (cellulose) fibers by weight, or may even be substantially free of, or free of, cellulose fibers, specifically reciting all 0.1% increments within the specified ranges and all ranges formed therein or thereby.

The absorbent core 1928 may also comprise a generally planar top side and a generally planar bottom side. The core 1928 may have a longitudinal axis 80' corresponding substantially to the longitudinal axis 80 of the absorbent article, as seen from the top in a planar view as in Figure 34. The absorbent material may be distributed in higher amount towards the front side than towards the rear side as more absorbency may be required at the front in particular articles. The absorbent material may have a non-uniform basis weight or a uniform basis weight across any portion of the core. The core wrap may be formed by two nonwoven materials, substrates, laminates, or other materials, 1916, 1916' which may be at least partially sealed along the sides of the absorbent core. The core wrap may be at least partially sealed along its front side, rear side, and two longitudinal sides so that substantially no absorbent material leaks out of the absorbent core wrap. The first material, substrate, or nonwoven 1916 may at least partially surround the second material, substrate, or nonwoven 1916' to form the core wrap. The first material 1916 may surround a portion of the second material 1916' proximate to the first and second side edges 1903 and 1904.

Cores comprising relatively high amount of SAP with various core designs are disclosed in U.S. Pat. No. 5,599,335 (Goldman), EP 1,447,066 (Busam), WO 95/11652 (Tanzer), U.S. Pat. Publ. No. 2008/0312622A1 (Hundorf), and WO 2012/052172 (Van Malderen).

The absorbent material may be one or more continuous layers present within the core wrap. Alternatively, the absorbent material may be comprised of individual pockets or stripes of absorbent material enclosed within the core wrap. In the first case, the absorbent material may be, for example, obtained by the application of a single continuous layer of absorbent material. The continuous layer of absorbent material, in particular of SAP, may also be obtained by combining two or more absorbent layers having discontinuous absorbent material application pattern, wherein the resulting layer is substantially continuously distributed across the absorbent particulate polymer material area, as disclosed in U.S. Pat. Appl. Publ. No. 2008/0312622A1 (Hundorf), for example. The absorbent core 1928 may comprise a first absorbent layer and a second absorbent layer. The first absorbent layer may comprise the first material 1916 and a first layer 1961 of absorbent material, which may be 100% or less of SAP. The second absorbent layer may comprise the second material 1916' and a second layer 1962 of absorbent material, which may also be 100% or less of SAP.

The fibrous thermoplastic adhesive material 1951 may be at least partially in contact with the absorbent material 1961, 1962 in the land areas and at least partially in contact with the materials 1916 and 1916' in the junction areas. This imparts an essentially three-dimensional structure to the fibrous layer of thermoplastic adhesive material 591, which in itself is essentially a two-dimensional structure of relatively small thickness, as compared to the dimension in length and width directions. Thereby, the fibrous thermoplastic adhesive material may provide cavities to cover the absorbent material in the land area, and thereby immobilizes this absorbent material, which may be 100% or less of SAP.

The core wrap may be made of a single substrate, material, or nonwoven folded around the absorbent material, or may comprise two (or more) substrates, materials, or nonwovens which are attached to another. Typical attachments are the so-called C-wrap and/or sandwich wrap. In a C-wrap, the longitudinal and/or transversal edges of one of the substrates are folded over the other substrate to form flaps. These flaps are then bonded to the external surface of the other substrate, typically by gluing. Other techniques may be used to form a core wrap. For example, the longitudinal and/or transversal edges of the substrates may be bonded together and then folded underneath the absorbent core 28 and bonded in that position.

The core wrap may be at least partially sealed along all the sides of the absorbent core so that substantially no absorbent material leaks out of the core. By "substantially no absorbent material" it is meant that less than 5%, less than 2%, less than 1%, or about 0% by weight of absorbent material escape the core wrap. The term "seal" is to be understood in a broad sense. The seal does not need to be continuous along the whole periphery of the core wrap but may be discontinuous along part or the whole of it, such as formed by a series of seal points spaced on a line. A seal may be formed by gluing and/or thermal bonding.

The core wrap may also be formed by a single substrate which may enclose as in a parcel wrap the absorbent material and be sealed along the front side and rear side of the core and one longitudinal seal.

The absorbent article may comprise a pair of barrier leg cuffs 1934. Each barrier leg cuff may be formed by a piece of material which is bonded to the absorbent article so it can extend upwards from the inner surface of the absorbent article and provide improved containment of liquids and other bodily exudates approximately at the junction of the torso and legs of the wearer. The barrier leg cuffs 1934 are delimited by a proximal edge 1964 joined directly or indirectly to the topsheet 1924 and/or the backsheet 1925 and a free terminal edge 1966, which is intended to contact and form a seal with the wearer's skin. The barrier leg cuffs 1934 extend at least partially between the front waist edge 1910 and the rear waist edge 1912 of the absorbent article on opposite sides of the longitudinal axis 1980 and are at least present in the crotch region 1907. The barrier leg cuffs 1934 may be joined at the proximal edge 1964 with the chassis of the absorbent article by a bond 1965 which may be made by gluing, fusion bonding, or combination of other suitable bonding processes. The bond 1965 at the proximal edge 64 may be continuous or intermittent. The bond 1965 closest to the raised section of the leg cuffs 1934 delimits the proximal edge 1964 of the standing up section of the leg cuffs 1934.

The barrier leg cuffs 1934 may be integral with the topsheet 1924 or the backsheet 1925 or may be a separate material joined to the absorbent article's chassis. The material of the barrier leg cuffs 1934 may extend through the whole length of the diapers but may be "tack bonded" to the topsheet 1924 towards the front waist edge 1910 and rear waist edge 1912 of the absorbent article so that in these sections the barrier leg cuff material remains flush with the topsheet 1924.

Each barrier leg cuff 1934 may comprise one, two or more elastic strands or strips of film 1935 close to this free terminal edge 1966 to provide a better seal.

In addition to the barrier leg cuffs 1934, the absorbent article may comprise gasketing cuffs 1932, which are joined to the chassis of the absorbent article, in particular to the topsheet 1924 and/or the backsheet 1925 and are placed externally relative to the barrier leg cuffs 1934. The gasketing cuffs 1932 may provide a better seal around the thighs of the wearer. Each gasketing leg cuff may comprise one or more elastic strings or elastic elements in the chassis of the absorbent article between the topsheet 1924 and backsheet 1925 in the area of the leg openings. All or a portion of the barrier leg and/or gasketing cuffs may be treated with a lotion or skin care composition. The barrier leg cuffs may be constructed in a number of different configurations, including those described in U.S. Pat. App. Publ. No. 2012/0277713.

In a form, the absorbent article may comprise front ears 1946 and rear ears 1940. The ears may be an integral part of the chassis, such as formed from the topsheet 1924 and/or backsheet 1925 as side panel. Alternatively, as

represented on Figure 34, the ears (1946, 1940) may be separate elements attached by gluing, heat embossing, and/or pressure bonding. The rear ears 1940 may be stretchable to facilitate the attachment of the tabs 1942 to the landing zone 1944 and maintain the taped diapers in place around the wearer's waist. The rear ears 1940 may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

One function of the LMS 1950 is to quickly acquire the fluid and distribute it to the absorbent core 1928 in an efficient manner. The LMS 1950 may comprise one or more layers, which may form a unitary layer or may remain as discrete layers which may be attached to each other. The LMS 1950 may comprise two layers: a distribution layer 1954 and an acquisition layer 1952 disposed between the absorbent core and the topsheet, but the present disclosure is not limited to such a configuration.

The LMS 1950 may comprise SAP as this may slow the acquisition and distribution of the fluid. In other forms, the LMS may be substantially free (e.g., 80%, 85%, 90%, 95%, or 99% free of) or completely free of SAP. The LMS may also comprise one or more of a variety of other suitable types of materials, such as opened-cell foam, air-laid fibers, or carded, resin bonded nonwoven materials, for example. Suitable example LMSs are described in WO 2000/59430 (Daley), WO 95/10996 (Richards), U.S. Pat. No. 5,700,254 (McDowall), and WO 02/067809 (Graef), for example.

The LMS 1950 may comprise a distribution layer 1954. The distribution layer 1954 may comprise at least 50% or more by weight of cross-linked cellulose fibers, for example. The cross-linked cellulosic fibers may be crimped, twisted, or curled, or a combination thereof including crimped, twisted, and curled. This type of material is disclosed in U.S. Pat. Publ. No. 2008/0312622 A1 (Hundorf).

The LMS 1950 may alternatively or additionally comprise an acquisition layer 1952. The acquisition layer 1952 may be disposed, for example, between the distribution layer 1954 and the topsheet 1924. The acquisition layer 1952 may be or may comprise a non-woven material, such as an SMS or SMMS material, comprising a spunbonded, a melt-blown and a further spunbonded layer or alternatively a carded chemical-bonded nonwoven. The acquisition layer 1952 may comprise air or wet-laid cellulosic, cross-linked cellulosic, or synthetic fibers, or blends thereof. The acquisition layer 1952 may comprise a roll-stock web of synthetic fibers (which may be processed to increase void space, such as by solid state formation), or a combination of synthetic and cellulosic fibers, bonded together to form a highloft material. Alternatively, the acquisition layer 1952 may comprise absorbent open cell foam. The nonwoven material may be latex bonded.

The LMS 1950 of the absorbent article 1920 may comprise channels that may generally enable better conformation of the absorbent article to the wearer's anatomy, leading to increased freedom-of-movement and reduced gapping. One or more of the channels of the LMS 1950 may be configured to work in concert with various channels in the absorbent core 1928, as discussed above. Furthermore, channels in the LMS 1950 may also provide increased void space to hold and distribute urine, BM or other bodily exudates within the absorbent article, leading to reduced leakage and skin contact. Channels in the LMS 1950 may also provide internal serviceable indicia, especially when highlighted via physical differences in texture, color, and/or pattern, to facilitate achieving the correct alignment of the absorbent article on a wearer. Thus, such physical differences may be, for example, visually and/or tactilely noticeable.

As stated previously, the material webs of the present invention may be utilized as a topsheet for a disposable absorbent article, examples of which include the sanitary napkin 1810 and diaper 1920 discussed heretofore.

The material webs of the present disclosure may be used as components of absorbent articles. More than one material web may be used in a single absorbent article. In such a context, the material webs may form at least a portion of: a topsheet; a topsheet and an acquisition layer; a topsheet and a distribution layer; an acquisition layer and a distribution layer; a topsheet, an acquisition layer, and a distribution layer; an outer cover; a backsheet; an outer cover and a backsheet, wherein a film (non-apertured layer) forms the backsheet and a nonwoven web forms the outer cover; a leg cuff; an ear or side panel; a fastener; a waist band; belt or any other suitable portion of an absorbent article. The number of strata in a material web may also be determined by the material webs' particular use.

In some forms, additional layers may be positioned between the topsheet and the absorbent core. For example, a secondary topsheet, acquisition layer, and/or distribution layer, each of which are known in the art, may be positioned between the topsheet and the absorbent core of the absorbent article.

## Arrays of Absorbent Articles

As mentioned heretofore, material webs of the present invention may be utilized in a plurality of absorbent articles. And, as noted previously, the material webs of the present invention can facilitate the construction of absorbent articles. Forms of the present invention are contemplated where an array of absorbent articles, each comprising a topsheet, backsheet, and an absorbent core disposed therebetween comprise material webs of the present invention. The array comprises a first plurality of absorbent articles comprising a first nonwoven web. The first material web comprises a first stratum and a second stratum integrally formed. The first material web may form at least a portion of

each of the first plurality of absorbent articles, e.g. topsheet, backsheet, absorbent core. The first stratum and the second stratum may be different from one another.

The array further comprises a second plurality of absorbent articles. Each of the second plurality of absorbent articles comprises a second material web which forms a portion of at least one of the topsheet, backsheet and/or absorbent core. The second material web may comprise a first stratum and a second stratum integrally formed. The first stratum and the second stratum may be different. The first material web may be different than the second material web. And, in some forms, the first plurality of absorbent articles may comprise a first type of absorbent article while the second plurality of absorbent articles comprise a second type of absorbent article. For example, the first plurality of absorbent articles may comprise adult incontinence articles and the second plurality of absorbent articles comprise sanitary pads.

Forms of the present invention are contemplated where the first material web comprises an MD/CD gradient which is different than an MD/CD gradient comprised by the second material web. For example, adult incontinence articles may be expected to absorb more liquid at a quicker rate. As such, the first nonwoven may comprise a first plurality of apertures having a first Effective Aperture Area. The second nonwoven may comprise a second plurality of apertures having a second Effective Aperture Area. The first Effective Aperture Area may be greater than the second Effective Aperture Area.

Forms of the present invention are contemplated where the array comprises additional pluralities of absorbent articles. Such additional pluralities may comprise material webs of the present invention. These material webs may be different than the first material web and/or second material web. Additionally, these material webs may comprise MD/CD gradients which are different than those of the first material web and/or second material web.

Depending on the use of the material webs of the present invention, the basis weight of material web may vary. The basis weight of material webs is usually expressed in grams per square meter (gsm). The basis weight of a material webs can range from about 8 gsm to about 100 gsm, depending on the ultimate use of the material 30. For example, the material webs of the present invention may have a basis weight from about 8 to about 40 gsm or from about 8 to about 30 gsm, or from about 8 to about 20 gsm. The basis weight of a multi-layer material is the combined basis weight of the constituent layers and any other added components, e.g. material web plus other constituent layers. The basis weight of multi-layer materials of interest herein can range from about 20 gsm to about 150 gsm, depending on the ultimate use of the material 30. The material webs may have a density that is between about 0.01 and about 0.4 g/cm3 measured at 0.3 psi (2 KPa).

## TESTS

### Basis Weight Test

A 9.00cm$^2$ large piece of nonwoven substrate, i.e., 1.0cm wide by 9.0cm long, is used. The sample may be cut out of a consumer product, such as a wipe or an absorbent article or a packaging material therefor. The sample needs to be dry and free from other materials like glue or dust. Samples are conditioned at 23° Celsius (± 2°C) and at a relative humidity of about 50% (± 5%) for 2 hours to reach equilibrium. The weight of the cut nonwoven substrate is measured on a scale with accuracy to 0.0001g. The resulting mass is divided by the specimen area to give a result in g/m$^2$ (gsm). Repeat the same procedure for at least 20 specimens from 20 identical consumer products or packaging materials therefor. If the consumer product or packaging materials therefore are large enough, more than one specimen can be obtained from each. An example of a sample is a portion of a topsheet of an absorbent article. If the local basis weight variation test is done, those same samples and data are used for calculating and reporting the average basis weight.

### Filament Diameter and Denier Test

The diameter of filaments in a sample of a nonwoven substrate is determined by using a Scanning Electron Microscope (SEM) and image analysis software. A magnification of 500 to 10,000 times is chosen such that the filaments are suitably enlarged for measurement (such that at least 3-5 pixels cross the diameter ("width") of a filament. The samples are sputtered with gold or a palladium-gold compound to avoid electric charging and vibrations of the filaments in the electron beam. A manual procedure for determining the filament diameters is used. Using a mouse and a cursor tool, the edge of a randomly selected filament is sought and then measured across its width (i.e., perpendicular to filament direction at that point) to the other edge of the filament. For non-circular filaments, the area of the cross-section is measured using the image analysis software by analyzing the Z-plane cross-sections of the filaments. The effective diameter is then calculated by calculating the diameter as if the found area was that of a circle. A scaled and calibrated image analysis tool provides the scaling to get actual reading in micrometers (μm). Several filaments are thus randomly selected across the sample of the nonwoven substrate using the SEM. At least two specimens from the nonwoven substrate are cut and tested in this manner. Altogether, at least 100 such measurements are made and then all data are recorded for statistical analysis. The recorded data are used to calculate average (mean) of the filament diameters,

standard deviation of the filament diameters, and median of the filament diameters. Another useful statistic is the calculation of the amount of the population of filaments that is below a certain upper limit. To determine this statistic, the software is programmed to count how many results of the filament diameters are below an upper limit and that count (divided by total number of data and multiplied by 100%) is reported in percent as percent below the upper limit, such as percent below 1 micrometer diameter or %-submicron, for example.

If the results are to be reported in denier, then the following calculations are made.

Filament Diameter in denier = Cross-sectional area (in m2) * density (in kg/m3)* 9000 m * 1000 g/kg.

For round filaments, the cross-sectional area is defined by the equation:

$$A=\pi*(D/2)^2.$$

The density for polypropylene, for example, may be taken as 910 kg/m3.

Given the filament diameter in denier, the physical circular filament diameter in meters (or micrometers) is calculated from these relationships and vice versa. We denote the measured diameter (in microns) of an individual circular filament as D.

Fiber Diameter Calculations:

The number-average diameter, which is typically just called the y average diameter, can be determined from the following equation.

$$d_{num} = \frac{\sum_{i=1}^{n} d_i}{n}$$

The filament cross sectional shape may be determined from the above images of the cross-sections in the Z-plane as well. The nonwoven filaments near the first surface of the material web should be evaluated for cross-sectional shape. The cross-sectional shape of the filaments near the first surface of the material web should be recorded. Nonwoven filaments near the second surface of the material web should be evaluated for cross-sectional shape. The cross-sectional shape of the filaments near the second surface of the material web should be recorded.

Specific Surface Area

The specific surface area of the nonwoven substrates of the present disclosure is determined by Krypton gas adsorption using a Micromeritic ASAP 2420 or equivalent instrument, using the continuous saturation vapor pressure (Po) method (according to ASTM D-6556-10), and following the principles and calculations of Brunauer, Emmett, and Teller, with a Kr-BET gas adsorption technique including automatic degas and thermal correction. Note that the specimens should not be degassed at 300 degrees Celsius as the method recommends, but instead should be degassed at room temperature. The specific surface area should be reported in $m^2/g$.

*Obtaining Samples of Nonwoven Substrates*

Each surface area measurement is taken from a specimen totaling 1 g of the nonwoven substrate of the present disclosure. In order to achieve 1 g of material, multiple specimens may be taken from one or more absorbent articles, one or more packages, or one or more wipes, depending on whether absorbent articles, packages, or wipes are being tested. Wet wipe specimens will be dried at 40 degrees C for two hours or until liquid does not leak out of the specimen under light pressure. The specimens are cut from the absorbent articles, packages, or wipes (depending on whether absorbent articles, packages, or wipes are being tested) in areas free of, or substantially free of, adhesives using scissors. An ultraviolet fluorescence analysis cabinet is then used on the specimens to detect the presence of adhesives, as the adhesives will fluoresce under this light. Other methods of detecting the presence of adhesives may also be used. Areas of the specimens showing the presence of adhesives are cut away from the specimens, such that the specimens are free of the adhesives. The specimens may now be tested using the specific surface area method above.

Mass-Average Diameter

The mass-average diameter of filaments is calculated as follows:

$$\text{mass average diameter, } d_{mass} = \frac{\sum_{i=1}^{n}(m_i \cdot d_i)}{\sum_{i=1}^{n} m_i} = \frac{\sum_{i=1}^{n}(\rho \cdot V_i \cdot d_i)}{\sum_{i=1}^{n}(\rho \cdot V_i)} = \frac{\sum_{i=1}^{n}\left(\rho \cdot \frac{\pi d_i^2 \cdot \partial x}{4} \cdot d_i\right)}{\sum_{i=1}^{n}\left(\rho \cdot \frac{\pi d_i^2 \cdot \partial x}{4}\right)} = \frac{\sum_{i=1}^{n} d_i^3}{\sum_{i=1}^{n} d_i^2}$$

where

filaments in the sample are assumed to be circular/cylindrical,
$d_i$ = measured diameter of the $i^{th}$ filament in the sample,
$\partial x$ = infinitesimal longitudinal section of filament where its diameter is measured, same for all the filaments in the sample,
$m_i$ = mass of the $i^{th}$ filament in the sample,
n = number of filaments whose diameter is measured in the sample
p = density of filaments in the sample, same for all the filaments in the sample
$V_i$ = volume of the $i^{th}$ filament in the sample.
The mass-average filament diameter should be reported in μm.

Gravimetric Weight Loss Test

The Gravimetric Weight Loss Test is used to determine the amount of a melt-additive such as lipid ester (e.g., Glycerol Tri-Stearate GTS) in a nonwoven substrate of the present disclosure. One or more samples of the nonwoven substrate are placed, with the narrowest sample dimension no greater than 1mm, into acetone at a ratio of lg nonwoven substrate sample per 100g of acetone using a refluxing flask system. First, the sample is weighed before being placed into the reflux flask, and then the mixture of the sample and the acetone is heated to 60°C for 20hours. The sample is then removed and air dried for 60 minutes and a final weight of the sample is determined. The equation for calculating the weight percent lipid ester in the sample is:

weight % lipid ester =( [initial mass of the sample - final mass of the sample] / [initial mass of the sample]) x 100%.

Aperture / Feret Angle Test

Aperture dimensions, Effective Open Area and Inter-Aperture Distance measurements are obtained from specimen images acquired using a flatbed scanner. The scanner is capable of scanning in reflectance mode at a resolution of 6400 dpi and 8 bit grayscale (a suitable scanner is an Epson Perfection V750 Pro from Epson America Inc., Long Beach CA or equivalent). The scanner is interfaced with a computer running an image analysis program (a suitable program is ImageJ v. 1.47 or equivalent, National Institute of Health, USA). The specimen images are distance calibrated against an acquired image of a ruler certified by NIST. A steel frame is used to mount the specimen, which is then backed with a black glass tile (P/N 11-0050-30, available from HunterLab, Reston, VA) prior to acquiring the specimen image. The resulting image is then threshold, separating open aperture regions from specimen material regions, and analyzed using the image analysis program. All testing is performed in a conditioned room maintained at about 23 ± 2 °C and about 50 ± 2 % relative humidity.

*Sample Preparation:*

To obtain a specimen, tape the absorbent article to a rigid flat surface in a planar configuration. Any elastics strands may be cut to facilitate laying the article flat. A rectilinear steel frame (100 mm square, 1.5 mm thick with an opening 60 mm square) is used to mount the specimen. Take the steel frame and place double-sided adhesive tape on the bottom surface surrounding the interior opening. Remove the release paper of the tape, and adhere the steel frame to the nonwoven substrate of the article that will be evaluated. Align the frame so that it is parallel and perpendicular to the machine direction (MD) and cross direction (CD) of the nonwoven substrate. Using a razor blade excise the nonwoven

substrate from the underlying layers of the article around the outer perimeter of the frame. Carefully remove the specimen such that its longitudinal and lateral extension is maintained to avoid distortion of the apertures or any other discontinuities. A cryogenic spray (such as Cyto-Freeze, Control Company, Houston TX) can be used to remove the specimen from the underlying layers if necessary. Five replicates obtained from five substantially similar articles are prepared for analysis. If the nonwoven substrate of interest is too small to accommodate the steel frame, reduce the frame dimensions accordingly to accomplish the goals of removal of the specimen without distortion of the apertures and/or any other discontinuities while leaving an opening of sufficient size to allow for scanning a significant portion of the nonwoven substrate. A nonwoven substrate raw material is prepared for testing by extending or activating it under the same process conditions, and to the same extent, as it would be for use on the absorbent article, and then in its extended state adhering it to the steel frame as described above for testing. Condition the samples at about 23 °C ± 2 C° and about 50% ± 2% relative humidity for 2 hours prior to testing.

*Image acquisition:*

Place the ruler on the scanner bed, oriented parallel to the sides of the scanner glass, and close the lid. Acquire a calibration image of the ruler in reflectance mode at a resolution of 6400 dpi (approximately 252 pixels per mm) and 8 bit grayscale, with the field of view corresponding to the dimensions of the interior of the steel frame. Save the calibration image as an uncompressed TIFF format file. Lift the lid and remove the ruler. After obtaining the calibration image, all specimens are scanned under the same conditions and measured based on the same calibration file. Next, place the framed specimen onto the center of the scanner bed, lying flat, with the outward facing surface of the specimen facing the scanner's glass surface. Orient the specimen so that sides of the frame are aligned parallel with and perpendicular to the sides of the scanner's glass surface, so that the resulting specimen image will have the MD vertically running from top to bottom. Place the black glass tile on top of the frame covering the specimen, close the lid and acquire a scanned image. Scan the remaining four replicates in like fashion. If necessary, crop all images to a rectangular field of view circumscribing the apertured region, and resave the files.

*Effective Open Area Calculation:*

Open the calibration image file in the image analysis program and perform a linear distance calibration using the imaged ruler. This distance calibration scale will be applied to all subsequent specimen images prior to analysis. Open a specimen image in the image analysis program and set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image the apertures appear as black, with a GL value of 255, and specimen as white, with a GL value of 0.

Using the image analysis program, analyze each of the discrete aperture regions. Measure and record all of the individual aperture areas to the nearest 0.01 $mm^2$, including partial apertures along the edges of the image. Discard any apertures with an area less than 0.3 $mm^2$. Apertures having a lower area than 0.3 $mm^2$ may prove difficult to measure particularly when stray fibers cross the boundary of the aperture. And such apertures with that small of an area are considered to contribute insignificantly to the Effective Open Area. Sum the remaining aperture areas (including whole and partial apertures), divide by the total area included in the image and multiply by 100. Record this value as the % Effective Open Area to the nearest 0.01%.

In like fashion, analyze the remaining four specimen images. Calculate and report the average % effective area values to the nearest 0.01% for the five replicates.

*Effective Aperture Area and Absolute Feret Angle:*

Open the calibration image (containing the ruler) file in the image analysis program. Resize the resolution of the original image from 6400 dpi to 640 dpi (approximately 25.2 pixels per mm) using a bicubic interpolation. Perform a linear distance calibration using the imaged ruler. This distance calibration scale will be applied to all subsequent specimen images prior to analysis. Open a specimen image in the image analysis program. Resize the resolution of the original image from 6400 dpi to 640 dpi (approximately 25.2 pixels per mm) using a bicubic interpolation. Set the distance scale. View the 8 bit histogram (0 to 255, with one bin per GL) and identify the gray level (GL) value for the minimum population located between the dark pixel peak of the aperture holes and the lighter pixel peak of the specimen material. Threshold the image at the minimum gray level value to generate a binary image. In the binary image the apertures appear as black, with a GL value of 255, and specimen as white, with a GL value of 0. Next, two morphological operations are performed on the binary image. First, a closing (a dilation operation followed by an erosion operation, iterations=1, pixel count=1), which removes stray filaments within an aperture hole. Second, an opening (an erosion operation followed

by a dilation operation, iterations=1, pixel count=1), which removes isolated black pixels. Pad the edges of the image during the erosion step to ensure that black boundary pixels are maintained during the operation. Lastly, fill any remaining voids enclosed within the black aperture regions.

Using the image analysis program, analyze each of the discrete aperture regions. During the analysis exclude measurements of partial apertures along the edges of the image, so that only whole apertures are measured. Measure and record all of the individual aperture areas, perimeters, feret diameters (length of the apertures) along with its corresponding angle of orientation in degrees from 0 to 180, and minimum feret diameters (width of the apertures). Record the measurements for each of the individual aperture areas to the nearest 0.01 $mm^2$, the perimeters and feret diameters (length and width), to the nearest 0.01 mm, and angles to the nearest 0.01 degree. Discard any apertures with an area less than 0.3 $mm^2$. Record the number of remaining apertures, divide by the area of the image and record as the Aperture Density value. The angle of orientation for an aperture aligned with the MD (vertical in the image) will have an angle of 90 degrees. Apertures with a positive slope, increasing from left to right, will have an angle between zero and 90 degrees. Apertures with a negative slope, decreasing from left to right, will have an angle between 90 and 180 degrees. Using the individual aperture angles calculate an Absolute Aperture Angle by subtracting 90 degrees from the original angle of orientation and taking its absolute value. In addition to these measurements, calculate an Aspect Ratio value for each individual aperture by dividing the aperture length by its width. Repeat this analysis for each of the remaining four replicate images. Calculate and report the statistical mean and standard deviation for each of the effective aperture dimension measurements using all of the aperture values recorded from the replicates. Calculate and report the % relative standard deviation (RSD) for each of the aperture dimension measurements by dividing the standard deviation by the mean and multiplying by 100.

*Inter-Aperture Distance Measurements:*

The average, standard deviation, median, and maximum distance between the apertures can be measured by further analyzing the binary image that was analyzed for the aperture dimension measurements. First, obtain a duplicate copy of the resized binary image following the morphological operations, and using the image analysis program, perform a Voronoi operation. This generates an image of cells bounded by lines of pixels having equal distance to the borders of the two nearest pattern apertures, where the pixel values are outputs from a Euclidian distance map (EDM) of the binary image. An EDM is generated when each inter-aperture pixel in the binary image is replaced with a value equal to that pixel's distance from the nearest pattern aperture. Next, remove the background zeros to enable statistical analysis of the distance values. This is accomplished by using the image calculator to divide the Voronoi cell image by itself to generate a 32-bit floating point image where all of the cell lines have a value of one, and the remaining parts of the image are identified as Not a Number (NaN). Lastly, using the image calculator, multiply this image by the original Voronoi cell image to generate a 32-bit floating point image where the distance values along the cell lines remain, and all of the zero values have been replaced with NaN. Next, convert the pixel distance values into actual inter-aperture distances by multiplying the values in the image by the pixel resolution of the image (approximately 0.04 mm per pixel), and then multiply the image again by 2 since the values represent the midpoint distance between apertures. Measure and record the mean, standard deviation, median and maximum inter-aperture distances for the image to the nearest 0.01 mm. Repeat this procedure for all replicate images. Calculate the % relative standard deviation (RSD) for the inter-aperture distance by dividing the standard deviation by the mean and multiplying by 100.

*Aperture Aspect Ratio and Area*

The apertures of the material webs of the present invention may have an aspect ratio of greater than one (ratio of the longest visible axis of an elliptical aperture to the shortest visible axis), for example, greater than two, greater than 3, greater than 5, or greater than 10, but typically less than 15. The aperture patterns in the material webs may comprise apertures having more than one aspect ratio, such as two or more distinct populations or having a substantially continuous distribution of aspect ratios having a slope greater than zero. Additionally, the aperture patterns may comprise apertures with more than two effective aperture area, either as two or more distinct populations or as a distribution of aperture areas having a slope greater than zero. The Relative Standard Deviation (RSD) of the aperture aspect ratios may be at least about 15%, at least about 25%, at least about 30%, or at least about 40%, or at least about 45%.

Filament Curl

The curl of filaments within a nonwoven is measured from a 3D x-ray sample image obtained on a micro-CT instrument (a suitable instrument is the Scanco μCT 50 available from Scanco Medical AG, Switzerland, or equivalent). The micro-CT instrument is a cone beam microtomograph with a shielded cabinet. A maintenance free x-ray tube is used as the source with an adjustable diameter focal spot. The x-ray beam passes through the sample, where some of

the x-rays are attenuated by the sample. The extent of attenuation correlates to the mass of material the x-rays have to pass through. The transmitted x-rays continue on to the digital detector array and generate a 2D projection image of the sample. A 3D image of the sample is generated by collecting several individual projection images of the sample as it is rotated, which are then reconstructed into a single 3D image. The instrument is interfaced with a computer running software to control the image acquisition and save the raw data. The 3D image is then analyzed using image analysis software (a suitable package is Avizo 3D Software, available from FEI, Hillsboro, OR, or equivalent).

*Sample Preparation*

To obtain a sample for measurement, lay a single layer of the dry substrate material out flat and die cut a circular piece with a diameter of 16 mm. Care should be taken to avoid folds, wrinkles or tears when selecting a location for sampling.

If the substrate material is a layer of an absorbent article, for example a topsheet, backsheet nonwoven, acquisition layer, distribution layer, or other component layer; tape the absorbent article to a rigid flat surface in a planar configuration. Carefully separate the individual substrate layer from the absorbent article. A scalpel and/or cryogenic spray (such as Cyto-Freeze, Control Company, Houston TX) can be used to remove a substrate layer from additional underlying layers, if necessary, to avoid any longitudinal and lateral extension of the material. Once the substrate layer has been removed from the article proceed with die cutting the sample as described above.

*Image Acquisition*

Set up and calibrate the micro-CT instrument according to the manufacturer's specifications. Place the sample into the appropriate holder, between two rings of low density material, which have an inner diameter of 8mm. This will allow the central portion of the sample to lay horizontal and be scanned without having any other materials directly adjacent to its upper and lower surfaces. Measurements should be taken in this region. The 3D image field of view is approximately 20 mm on each side in the xy-plane with a resolution of approximately 5000 by 5000 pixels, and with a sufficient number of 4 micron thick slices collected to fully include the z-direction of the sample. The reconstructed 3D image resolution contains isotropic voxels of 4 microns. Images are acquired with the source at 45 kVp and 88 μA with no additional low energy filter. These current and voltage settings may be optimized to produce the maximum contrast in the projection data with sufficient x-ray penetration through the sample, but once optimized held constant for all substantially similar samples. A total of 1200 projections images are obtained with an integration time of 1000 ms and 4 averages. The projection images are reconstructed into the 3D image, and saved in 16-bit RAW format to preserve the full detector output signal for analysis.

*Image Processing*

Load the 3D image into the image analysis software. Threshold the 3D image at a value which separates, and removes, the background signal due to air but maintains the signal from the sample fibers within the substrate. Select four regions 0.8 mm by 0.8 mm in the xy plane and by the thickness of the specimen in the z direction. These regions are chosen such that they avoid the thermal bonds of the nonwoven. To assess the fiber curvature within the dual layers of the nonwoven, divide the z direction into three equal parts. To avoid the layer's boundary, only the top and bottom third are cropped and analyzed.

The cropped 3D image is processed to trace the medial axes of the fibers to create a "skeleton" network of the fiber paths. Next the fiber paths are segmented at any intersection of the fibers. For example, two fibers intersecting in a cross would be divided into four segments. After all segments have been identified, each is further divided into sections as follows. From the originating point of the segment, the fiber path is transversed to a point along the path at which the starting path point and the current path point can be connect by a linear cord exactly 200 μm in length. The length of segment path between the start and the end of the cord is the edge length for that cord section. This process is repeated using the current path point as the new starting path point and continuing to transverse the fiber path to the next path point that can be connect by a linear cord exactly 200 μm in length. In this fashion the segment is sectioned until a cord can no longer be fitted. Any remaining segment length is discarded. Also if a segment is not long enough to fit a cord this segment is also discarded. Each fiber segment of the 3D skeleton is sectioned in this fashion and the average edge length for all sections is calculated and recorded to the nearest micrometer.

Each of the four cropped images from the top side of the nonwoven are processed and a grand average edge length is calculated and reported to the nearest micrometer as Curvature for the top side. Likewise the four cropped images from the bottom side are processed and a grand average edge length is calculated and reported to the nearest micron as Curvature for the bottom side.

Surface Energy / Contact Angle Method

Contact angles on substrates are determined using ASTM D7490-13 modified with the specifics as describe herein, using a goniometer and appropriate image analysis software (a suitable instrument is the FTA200, First Ten Angstroms, Portsmouth, VA, or equivalent) fitted with a 1 mL capacity, gas tight syringe with a No. 27 blunt tipped stainless steel needle. Two test fluids are used: Type II reagent water (distilled) in accordance with ASTM Specification D1193-99 and 99+% purity diiodomethane (both available from Sigma Aldrich, St. Louis, MO). Contact angles from these two test fluids can further be used to calculate surface energy based on the Owens-Wendt-Kaelble equation. All testing is to be performed at about 23 °C ± 2 C° and a relative humidity of about 50% ± 2%.

A 50 mm by 50 mm nonwoven substrate to be tested is removed from the article taking care to not touch the region of interest or otherwise contaminate the surface during harvesting or subsequent analysis. Condition the samples at about 23 °C ± 2 C° and a relative humidity of about 50% ± 2% for 2 hours prior to testing.

Set up the goniometer on a vibration-isolation table and level the stage according to the manufacturer's instructions. The video capture device must have an acquisition speed capable of capturing at least 10-20 images from the time the drop hits the surface of the specimen to the time it cannot be resolved from the specimen's surface. A capture rate of 900 images/sec is typical. Depending on the hydrophobicity / hydrophilicity of the specimen, the drop may or may not rapidly wet the surface of the nonwoven sample. In the case of slow acquisition, the images should be acquired until 2% of the volume of the drop is absorbed into the specimen. If the acquisition is extremely fast, the first resolved image should be used if the second image shows more than 2% volume loss.

Place the specimen on the goniometer's stage and adjust the hypodermic needle to the distance from the surface recommended by the instrument's manufacturer (typically 3 mm). If necessary adjust the position of the specimen to place the target site under the needle tip. Focus the video device such that a sharp image of the drop on the surface of the specimen can be captured. Start the image acquisition. Deposit a 5 μL ± 0.1 μL drop onto the specimen. If there is visible distortion of the drop shape due to movement, repeat at a different, but equivalent, target location. Make two angle measurements on the drop (one on each drop edge) from the image at which there is a 2% drop volume loss. If the contact angles on two edges are different by more than 4°, the values should be excluded and the test repeated at an equivalent location on the specimen. Identify five additional equivalent sites on the specimen and repeat for a total of 6 measurements (12 angles). Calculate the arithmetic mean for this side of the specimen and report to the nearest 0.01°. In like fashion, measure the contact angle on the opposite side of the specimen for 6 drops (12 angles) and report separately to the nearest 0.01°.

To calculate surface energy, the contact angle for both water and diiodomethane must be tested as described above. The value for each test fluid is then substituted into two separate expressions of the Owens-Wendt-Kaelble equation (one for each fluid). This results in two equations and two unknowns, which are then solved for the dispersion and polar components of surface tension.

The Owens-Wendt-Kaelble equation:

$$\frac{\gamma_l(1+cos\theta)}{2} = \left(\gamma_l^d + \gamma_s^d\right)^{0.5} + \left(\gamma_l^p + \gamma_s^p\right)^{0.5}$$

where:

θ = the average contact angle for the test liquid on the test specimen
$\gamma_l$ and $\gamma_s$ = the surface tension of the test liquid and test specimen, respectively, in dyn/cm
$\gamma^d$ and $\gamma^p$ = the dispersion and polar components of the surface tension, respectively, in dyn/cm

| | Surface Tension ($\gamma_l$) (dyn/cm) | | |
|---|---|---|---|
| Solvent | Dispersion | Polar | Total |
| Diiodomethane | 50.8 | 0.0 | 50.8 |
| Water | 21.8 | 51.0 | 72.8 |

The Owens-Wendt-Kaelble equation is simplified to the following when a dispersive solvent such as diiodomethane is used since the polar component is zero:

$$\frac{\gamma_l(1 + cos\theta)}{2} = \left(\gamma_l^d + \gamma_s^d\right)^{0.5}$$

Using the values from the table and θ (measured) for diiodomethane, the equation can be solved for the dispersive component of surface energy ($\gamma^d{}_s$). Now using the values from the table and θ (measured) for water, and the calculated value ($\gamma^d{}_s$), the Owens-Wendt-Kaelble equation can be solved for the polar component of surface energy ($\gamma^p{}_s$). The sum of $\gamma^d{}_s$ + $\gamma^d{}_s$ is the total solid surface tension and is reported to the nearest 0.1 dyn/cm.

Filament Composition

Fiber composition is identified using FTIR microscopy. A suitable system allows for the spatial separation and visualization of the fiber of interest and then the collection of localized FTIR spectra, using either an All-Reflecting or Attenuated Total Reflection (ATR) objective (an example system is an Olympus BX-51 Microscope with IlluminatIR II Infrared Microprobe and PixeLink camera available from Smith Detection, Edgewood, MD). The instrument is calibrated and operated as per the instructions from the vendor of the specific instrument.

Remove the nonwoven of interest from the product using cryogen freeze spray (such as Cyto-Freeze, Control Company, Houston TX) as necessary. Under magnification, use tweezers to remove a fiber from within the outermost layer of side one of the spunmelt sample. If the fiber is a bico-fiber, cut diagonally across the fiber to expose the core. Mount the fiber on a microscope slide and place the slide on the stage of the FTIR microscope. Move the sample fiber underneath the objective and focus the scope on the fiber. Move to a region where there is no sample and collect a blank FTIR-spectrum. Return the fiber underneath the objective and collect a FTIR spectrum of the fiber. If the fiber is a bico-fiber collect spectra of both the outer sheath and core. Compare background subtracted spectra against library spectra for identification.

In like fashion, remove a fiber from within the outermost layer of side two of the spunmelt sample and collect FTIR-spectra for identification. A total of three fibers from each surface of the nonwoven are collect and analyzed to confirm identification.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A disposable absorbent (1710, 1900) article having a wearer-facing surface and a garment-facing surface, a longitudinal axis (1780, 1980) and a lateral axis (1790, 1990) perpendicular to the longitudinal axis, the disposable absorbent article further comprising:

    a topsheet (1714, 2014) forming at least a portion of the wearer- facing surface;
    a backsheet (1716, 1925) forming at least a portion of the garment-facing surface;
    an absorbent core (1718, 1928) disposed between the topsheet and the backsheet;
    a material web (10) having a first surface (50) and a second surface (52) opposite the first surface, a machine direction (MD) generally parallel to the longitudinal axis and a cross machine direction (CD) generally parallel to the lateral axis and perpendicular to the MD, and a Z-direction perpendicular to a plane comprising the MD and CD, the material web further comprising:

        a first stratum (20) comprising a first plurality of filaments, the first stratum forming a portion of the first surface; and
        a second stratum (30) comprising a second plurality of filaments, the second stratum forming a portion of the second surface;
        wherein, the first stratum and the second stratum are integrally formed, wherein
        the material web comprises a Z-direction characteristic difference from the first stratum to the second stratum and an MD and/or CD characteristic difference, and wherein the material web forms a portion of the disposable absorbent article; and
        wherein the material web comprises a third stratum integrally formed with the material web and disposed on the second surface of the material web, wherein the first stratum comprises a hydrophobic melt additive and the third stratum comprises a hydrophilic melt additive such that the first stratum has a lower surface

energy than the second stratum, and wherein the second plurality of filaments have a diameter of less than about 8 microns.

2. The absorbent article of claim 1, wherein the Z-direction characteristic difference comprises surface energy.

3. The absorbent article of any of the preceding claims, further comprising a plurality of apertures extending from the first surface of the material web to the second surface of the material web.

4. The absorbent article of any of the preceding claims, wherein the material web forms a portion of the topsheet such that the first surface forms a portion of the wearer-facing surface.

5. The absorbent article of any of the preceding claims, further comprising a first zone in the MD/CD plane and a second zone in the MD/CD plane, wherein the second zone has an MD or CD characteristic difference that is different than the first zone.

6. The absorbent article of claim 5, wherein the first zone comprises a different texture than the second zone.

7. The absorbent article of claims 5-6, wherein the first zone comprises a plurality of discontinuities extending from the first surface in the positive Z-direction.

8. The absorbent article of claims 5-7, wherein the second zone comprises a plurality of apertures.

9. The absorbent article of claim 5-8, wherein the first zone comprises a plurality of discontinuities extending from the second surface in the negative Z-direction.

10. The absorbent article of claims 1-8, wherein a first plurality of discontinuities extend from the first surface in a positive Z-direction.

11. The absorbent article of claims 1-9, wherein a first plurality of discontinuities extend from the second surface in the negative Z-direction.

12. The absorbent article of claims 1-3, wherein the material web forms a portion of the garment-facing surface.

13. The absorbent article of any of the preceding claims, wherein the absorbent article further comprises a pair of longitudinal side edges and a pair of wings extending laterally outboard of the longitudinal side edges or a pair of barrier cuffs extending along the longitudinal side edges and wherein the material web forms a portion of the wings or a portion of the pair of barrier cuffs.

## Patentansprüche

1. Einweg-Absorptionsartikel (1710, 1900) mit einer trägerseitigen Oberfläche und einer bekleidungsseitigen Oberfläche, einer Längsachse (1780, 1980) und einer Querachse (1790, 1990) senkrecht zur Längsachse, wobei der Einweg-Absorptionsartikel ferner Folgendes umfasst:

   eine Oberschicht (1714, 2014), die wenigstens einen Teil der trägerseitigen Oberfläche bildet;
   eine Unterschicht (1716, 1925), die wenigstens einen Teil der bekleidungsseitigen Oberfläche bildet;
   einen Absorptionskern (1718, 1928), der zwischen der Oberschicht und der Unterschicht angeordnet ist;
   eine Materialbahn (10) mit einer ersten Oberfläche (50) und einer zweiten Oberfläche (52) gegenüber der ersten Oberfläche, einer Maschinenlaufrichtung (MD) im Allgemeinen parallel zur Längsachse und einer Maschinenquerrichtung (CD) im Allgemeinen parallel zur Querachse und senkrecht zur MD und einer Z-Richtung senkrecht zu einer Ebene, die MD und CD umfasst, wobei die Materialbahn ferner Folgendes umfasst:

      eine erste Schicht (20), die eine erste Vielzahl von Filamenten umfasst, wobei die erste Schicht einen Teil der ersten Oberfläche bildet; und
      eine zweite Schicht (30), die eine zweite Vielzahl von Filamenten umfasst, wobei die zweite Schicht einen Teil der zweiten Oberfläche bildet;
      wobei die erste Schicht und die zweite Schicht integral ausgebildet sind, wobei die Materialbahn eine

charakteristische Differenz in Z-Richtung von der ersten Schicht zu der zweiten Schicht und eine charakteristische Differenz in MD und/oder CD umfasst, und wobei die Materialbahn einen Teil des Einweg-Absorptionsartikels bildet; und
wobei die Materialbahn eine dritte Schicht umfasst, die integral mit der Materialbahn ausgebildet und auf der zweiten Oberfläche der Materialbahn angeordnet ist, wobei die erste Schicht ein hydrophobes Schmelzadditiv umfasst und die dritte Schicht ein hydrophiles Schmelzadditiv umfasst, so dass die erste Schicht eine niedrigere Oberflächenenergie als die zweite Schicht aufweist, und wobei die zweite Vielzahl von Filamenten einen Durchmesser von weniger als etwa 8 Mikrometern aufweist.

**2.** Absorptionsartikel nach Anspruch 1, wobei die Z-Richtungscharakteristikdifferenz Oberflächenenergie umfasst.

**3.** Absorptionsartikel nach einem der vorstehenden Ansprüche, ferner umfassend eine Vielzahl von Öffnungen, die sich von der ersten Oberfläche der Materialbahn zur zweiten Oberfläche der Materialbahn erstrecken.

**4.** Absorptionsartikel nach einem der vorstehenden Ansprüche, wobei die Materialbahn einen Teil der Oberschicht bildet, sodass die erste Oberfläche einen Teil der trägerseitigen Oberfläche bildet.

**5.** Absorptionsartikel nach einem der vorstehenden Ansprüche, ferner umfassend eine erste Zone in der MD/CD-Ebene und eine zweite Zone in der MD/CD-Ebene, wobei die zweite Zone eine MD- oder CD-Charakteristikdifferenz aufweist, die sich von der ersten Zone unterscheidet.

**6.** Absorptionsartikel nach Anspruch 5, wobei die erste Zone eine andere Textur als die zweite Zone umfasst.

**7.** Absorptionsartikel nach den Ansprüchen 5-6, wobei die erste Zone eine Vielzahl von Diskontinuitäten umfasst, die sich von der ersten Oberfläche in der positiven Z-Richtung erstrecken.

**8.** Absorptionsartikel nach den Ansprüchen 5-7, wobei die zweite Zone eine Vielzahl von Öffnungen umfasst.

**9.** Absorptionsartikel nach Anspruch 5-8, wobei die erste Zone eine Vielzahl von Diskontinuitäten umfasst, die sich von der zweiten Oberfläche in der negativen Z-Richtung erstrecken.

**10.** Absorptionsartikel nach den Ansprüchen 1-8, wobei sich eine erste Vielzahl von Diskontinuitäten von der ersten Oberfläche in einer positiven Z-Richtung erstreckt.

**11.** Absorptionsartikel nach den Ansprüchen 1-9, wobei sich eine erste Vielzahl von Diskontinuitäten von der zweiten Oberfläche in der negativen Z-Richtung erstreckt.

**12.** Absorptionsartikel nach den Ansprüchen 1-3, wobei die Materialbahn einen Teil der bekleidungsseitigen Oberfläche bildet.

**13.** Absorptionsartikel nach einem der vorstehenden Ansprüche, wobei der Absorptionsartikel ferner ein Paar Längsseitenränder und ein Paar Flügel, die sich seitlich außerhalb der Längsseitenränder erstrecken, oder ein Paar Sperrbündchen, die sich entlang der Längsseitenränder erstrecken, umfasst und wobei die Materialbahn einen Teil der Flügel oder einen Teil des Paars Sperrbündchen bildet.

## Revendications

**1.** Article absorbant jetable (1710, 1900) ayant une surface faisant face au porteur et une surface faisant face au vêtement, un axe longitudinal (1780, 1980) et un axe latéral (1790, 1990) perpendiculaire à l'axe longitudinal, l'article absorbant jetable comprenant en outre :

une feuille de dessus (1714, 2014) formant au moins une partie de la surface faisant face au porteur ;
une feuille de fond (1716, 1925) formant au moins une partie de la surface faisant face au vêtement ;
une âme absorbante (1718, 1928) disposée entre la feuille de dessus et la feuille de fond ;
une nappe de matériau (10) ayant une première surface (50) et une deuxième surface (52) opposée à la première surface, une direction machine (MD) généralement parallèle à l'axe longitudinal et une direction transversale de la machine (CD) généralement parallèle à l'axe latéral et perpendiculaire à la MD, et une direction Z perpendiculaire à un plan comprenant la MD et la CD, la nappe de matériau comprenant en outre :

une première couche (20) comprenant une première pluralité de filaments, la première couche formant une partie de la première surface ; et
une deuxième couche (30) comprenant une deuxième pluralité de filaments, la deuxième couche formant une partie de la deuxième surface ;
dans lequel, la première couche et la deuxième couche sont formées d'un seul tenant, dans lequel la nappe de matériau comprend une différence de caractéristique de direction Z de la première couche à la deuxième couche et une différence de caractéristique de MD et/ou CD, et dans lequel la nappe de matériau forme une partie de l'article absorbant jetable ; et
dans lequel la nappe de matériau comprend une troisième couche formée d'un seul tenant avec la nappe de matériau et disposée sur la deuxième surface de la nappe de matériau, dans lequel la première couche comprend un additif de fusion hydrophobe et la troisième couche comprend un additif de fusion hydrophile de telle sorte que la première couche a une plus faible énergie de surface que la deuxième couche, et dans lequel la deuxième pluralité de filaments ont un diamètre inférieur à environ 8 micromètres.

**2.** Article absorbant selon la revendication 1, dans lequel la différence de caractéristique de direction Z comprend l'énergie de surface.

**3.** Article absorbant selon l'une quelconque des revendications précédentes, comprenant en outre une pluralité d'ouvertures s'étendant de la première surface de la nappe de matériau à la deuxième surface de la nappe de matériau.

**4.** Article absorbant selon l'une quelconque des revendications précédentes, dans lequel la nappe de matériau forme une partie de la feuille de dessus de telle sorte que la première surface forme une partie de la surface faisant face au porteur.

**5.** Article absorbant selon l'une quelconque des revendications précédentes, comprenant en outre une première zone dans le plan MD/CD et une deuxième zone dans le plan MD/CD, dans lequel la deuxième zone a une différence de caractéristique de MD ou CD qui est différente par rapport à la première zone.

**6.** Article absorbant selon la revendication 5, dans lequel la première zone comprend une texture différente par rapport à la deuxième zone.

**7.** Article absorbant selon les revendications 5 ou 6, dans lequel la première zone comprend une pluralité de discontinuités s'étendant à partir de la première surface dans la direction Z positive.

**8.** Article absorbant selon les revendications 5 à 7, dans lequel la deuxième zone comprend une pluralité d'ouvertures.

**9.** Article absorbant selon la revendication 5 à 8, dans lequel la première zone comprend une pluralité de discontinuités s'étendant à partir de la deuxième surface dans la direction Z négative.

**10.** Article absorbant selon les revendications 1 à 8, dans lequel une première pluralité de discontinuités s'étendent à partir de la première surface dans une direction Z positive.

**11.** Article absorbant selon les revendications 1 à 9, dans lequel une première pluralité de discontinuités s'étendent à partir de la deuxième surface dans la direction Z négative.

**12.** Article absorbant selon les revendications 1 à 3, dans lequel la nappe de matériau forme une partie de la surface faisant face au vêtement.

**13.** Article absorbant selon l'une quelconque des revendications précédentes, dans lequel l'article absorbant comprend en outre une paire de bords latéraux longitudinaux et une paire d'ailettes s'étendant latéralement à l'extérieur des bords latéraux longitudinaux ou une paire de revers formant barrière s'étendant le long des bords latéraux longitudinaux et dans lequel la nappe de matériau forme une partie des ailettes ou une partie de la paire de revers formant barrière.

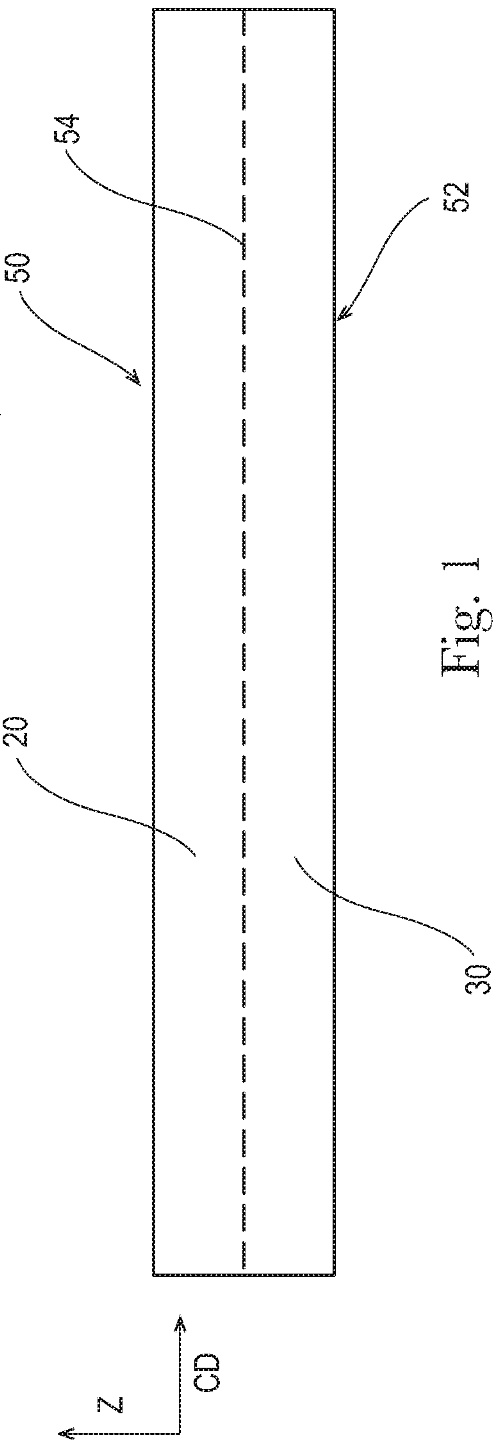


Fig. 1

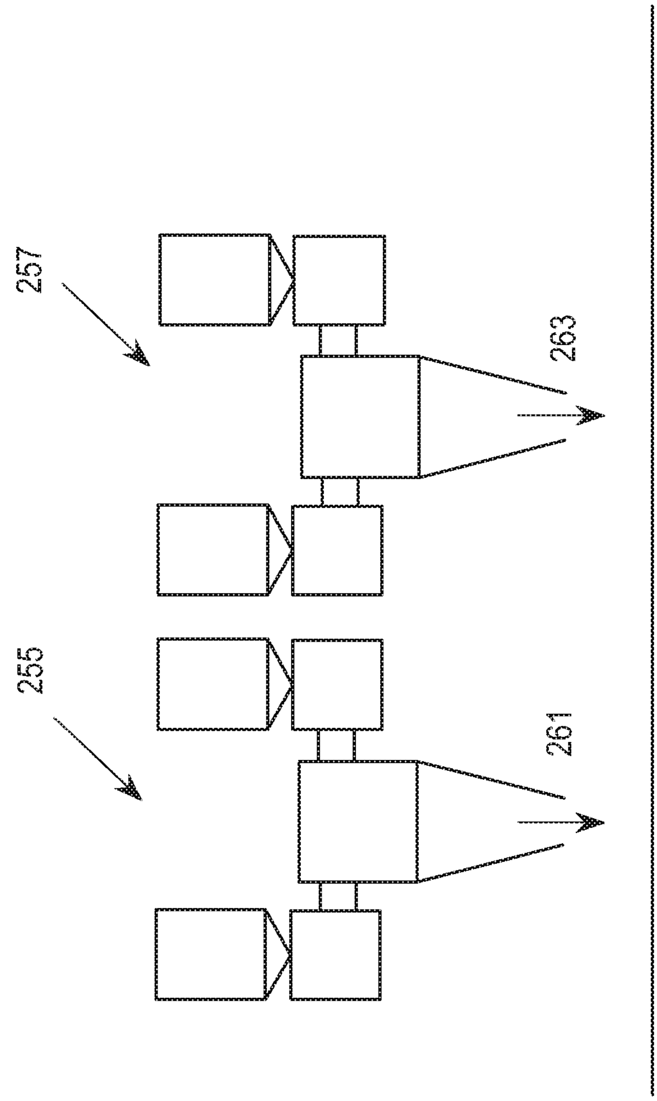


Fig. 2

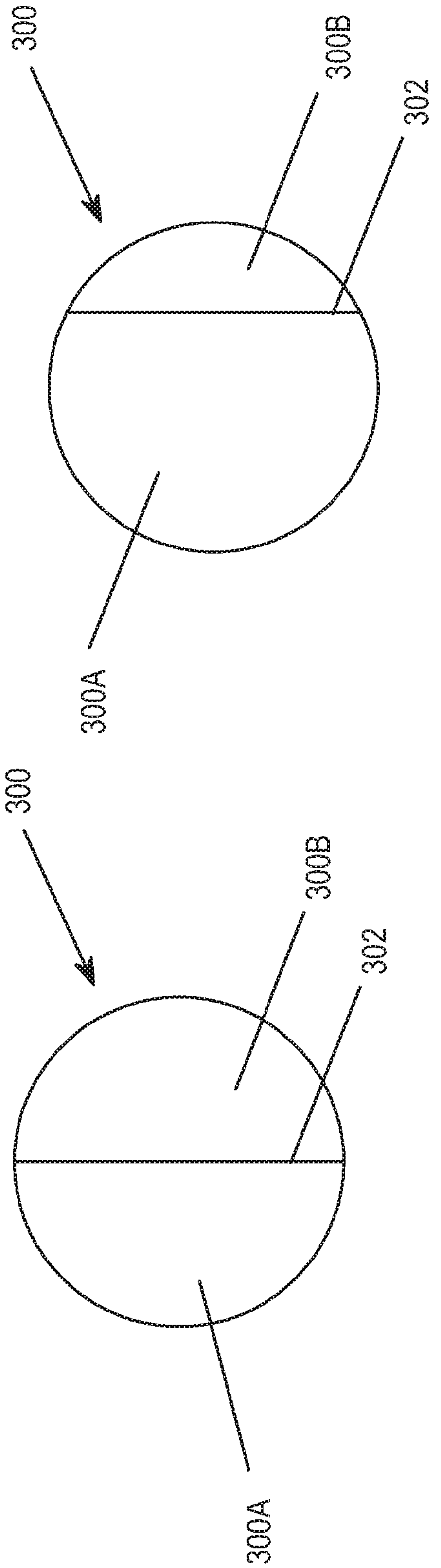
300
300B
302
300A
300
300B
302
300A

Fig. 3A

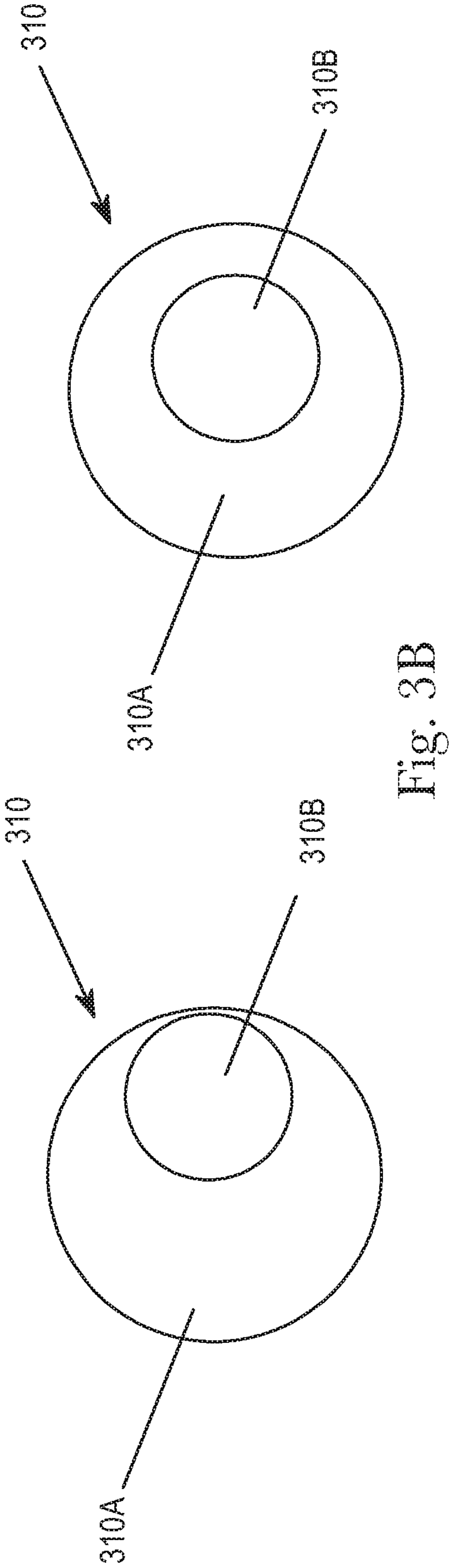
310
310B
310A
310
310B
310A

Fig. 3B

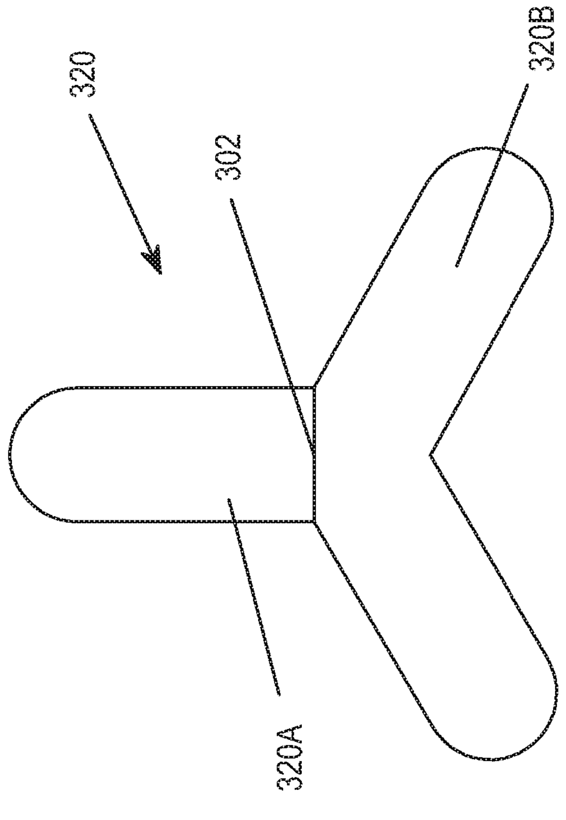


Fig. 3C

Fig. 4A

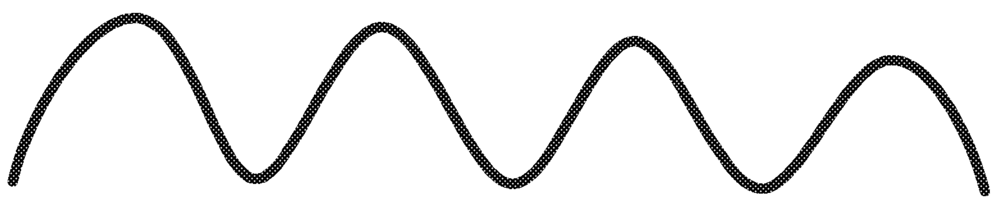

Fig. 4B

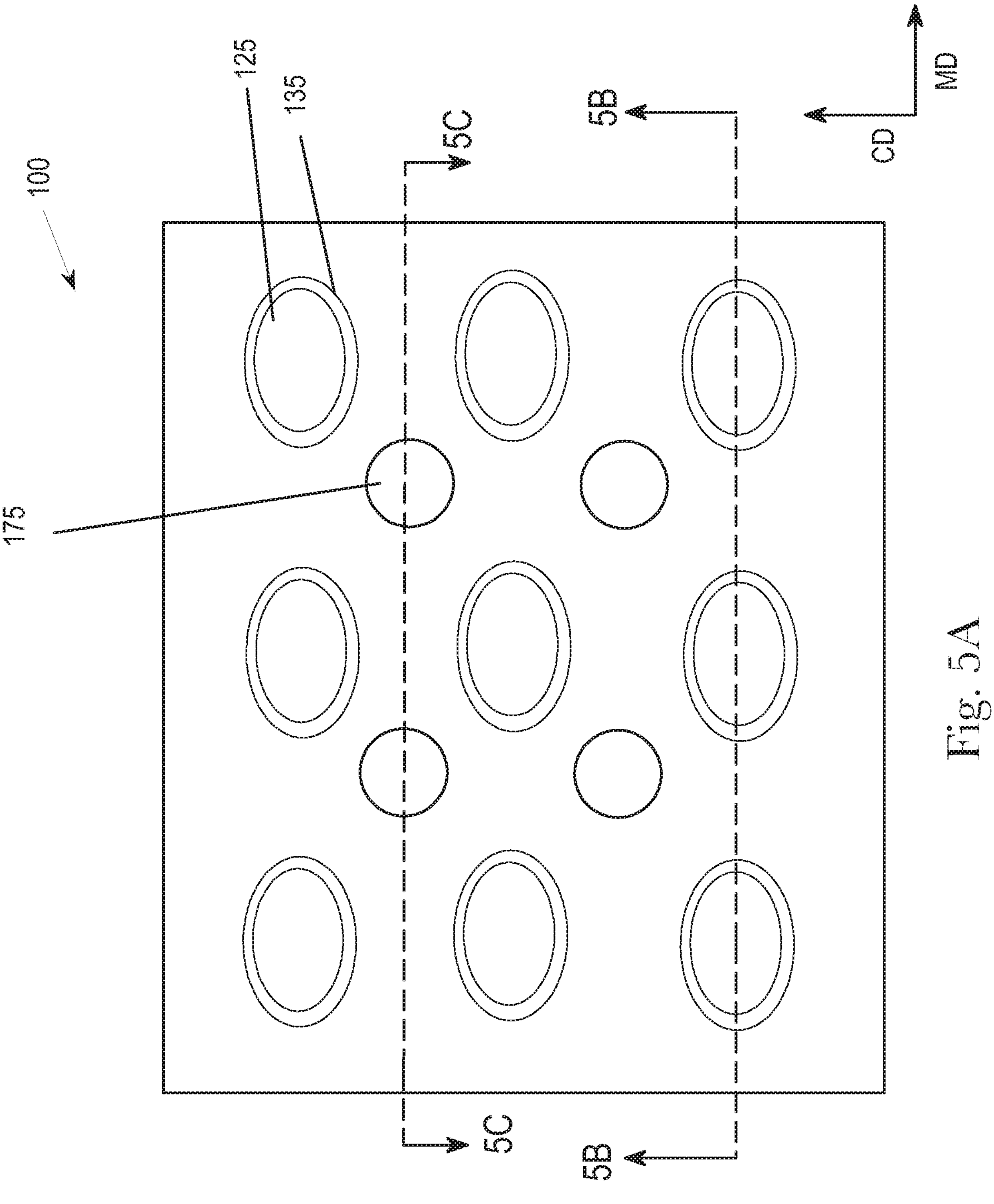


Fig. 5A

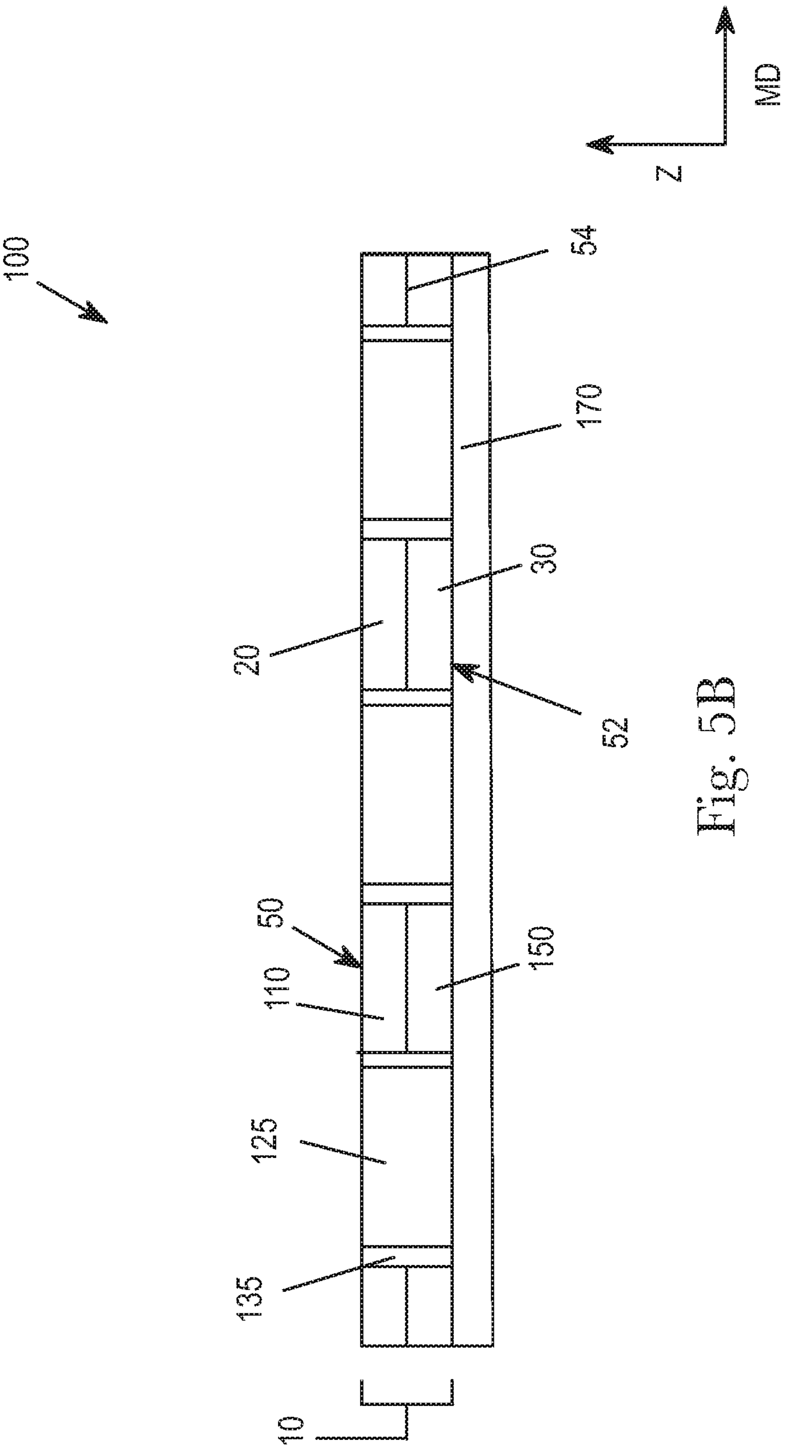


Fig. 5B

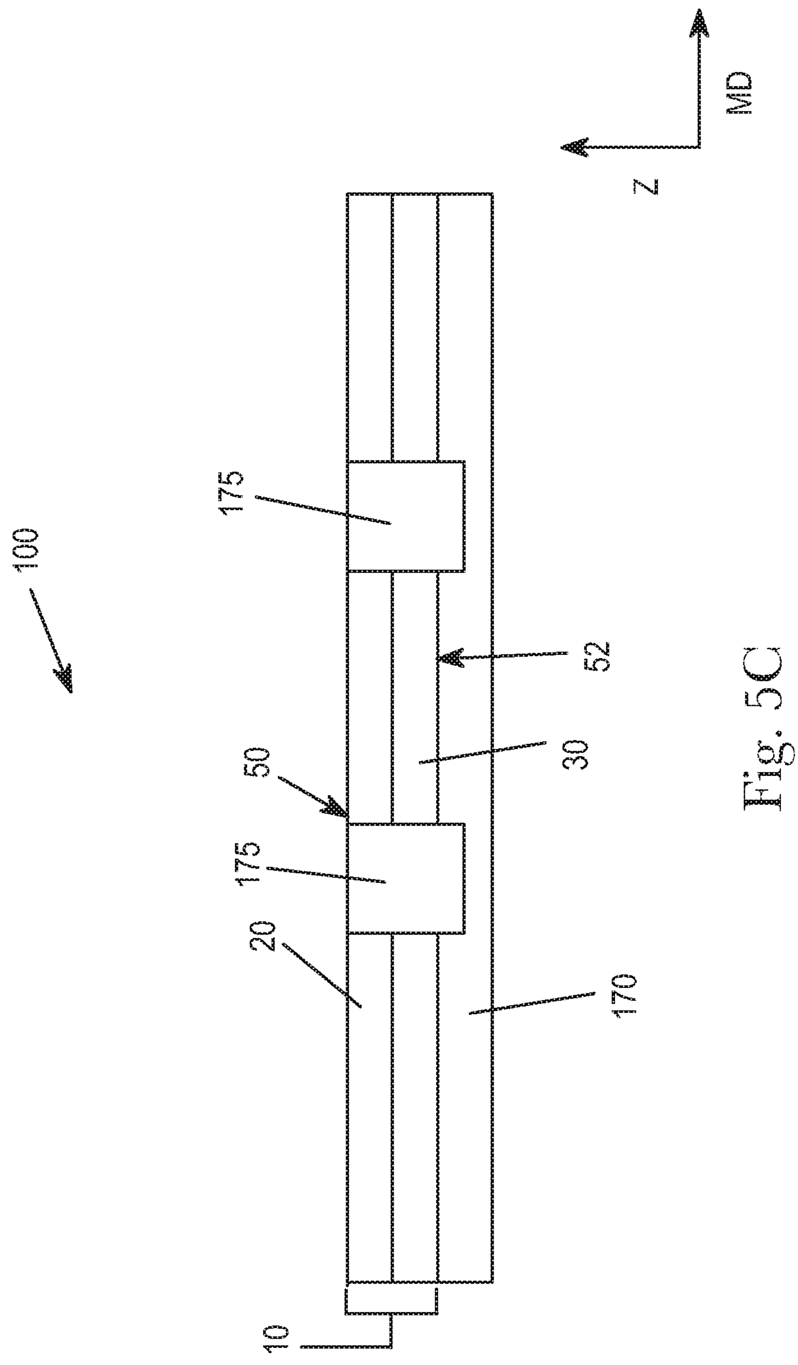


Fig. 5C

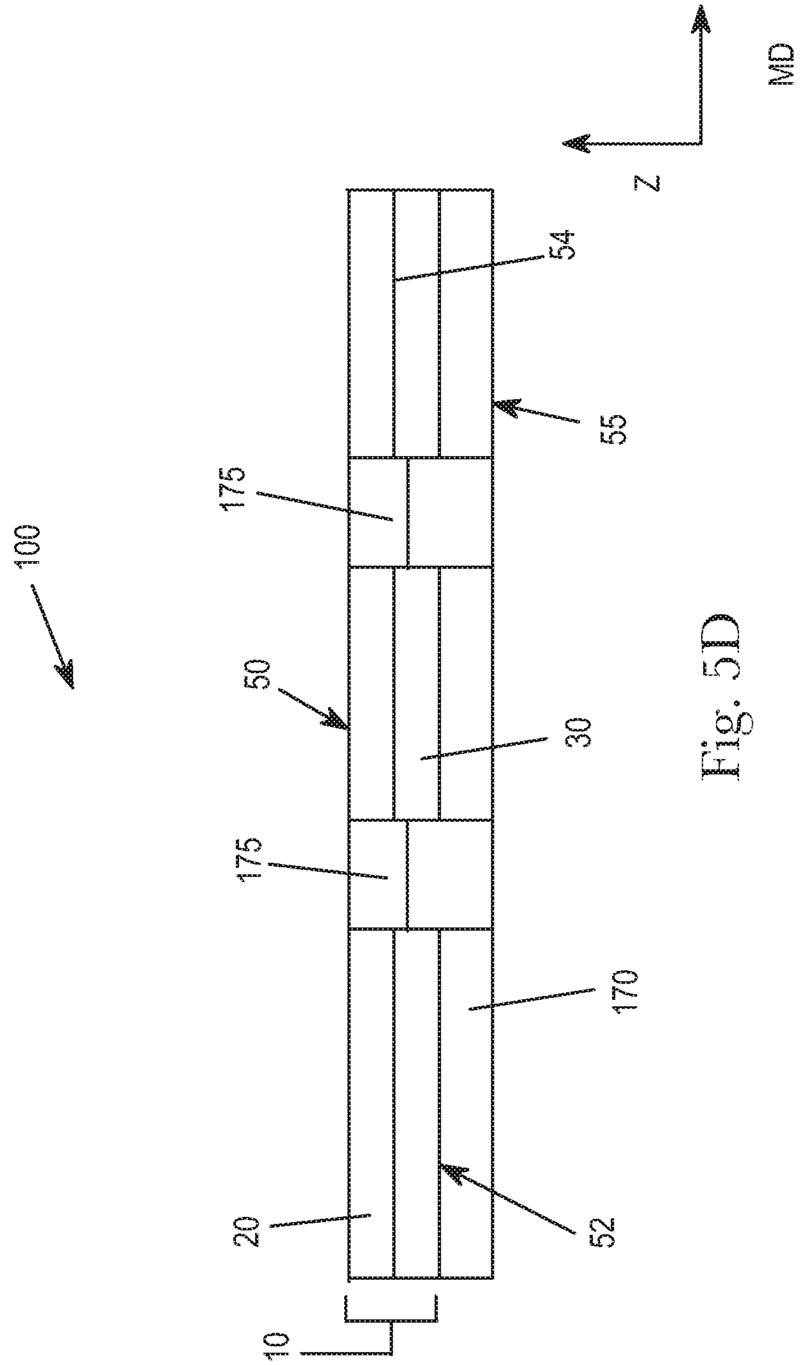


Fig. 5D

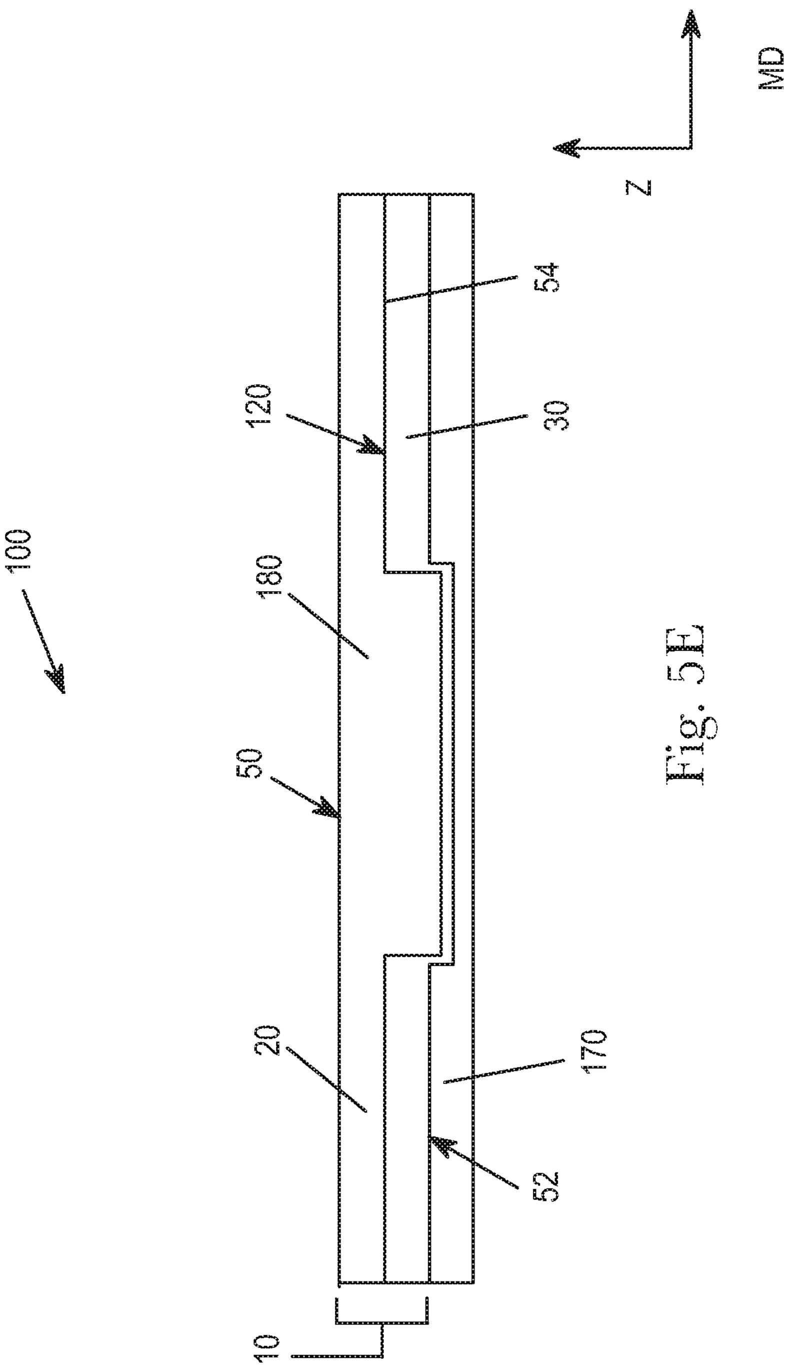


Fig. 5E

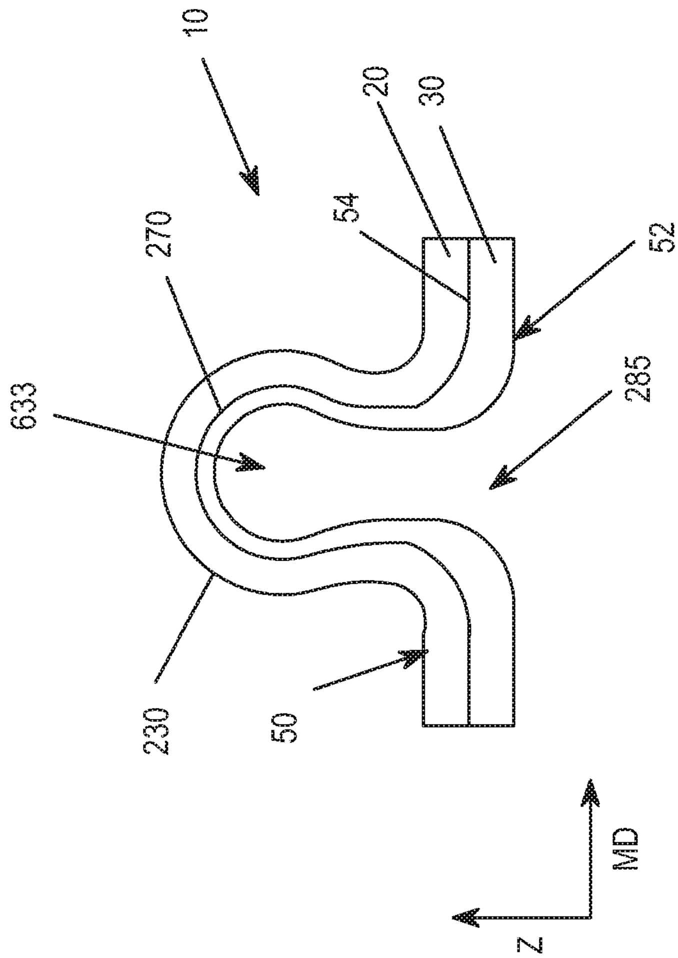
10
20
30
54
52
270
633
285
230
50
MD
Z

Fig. 6B

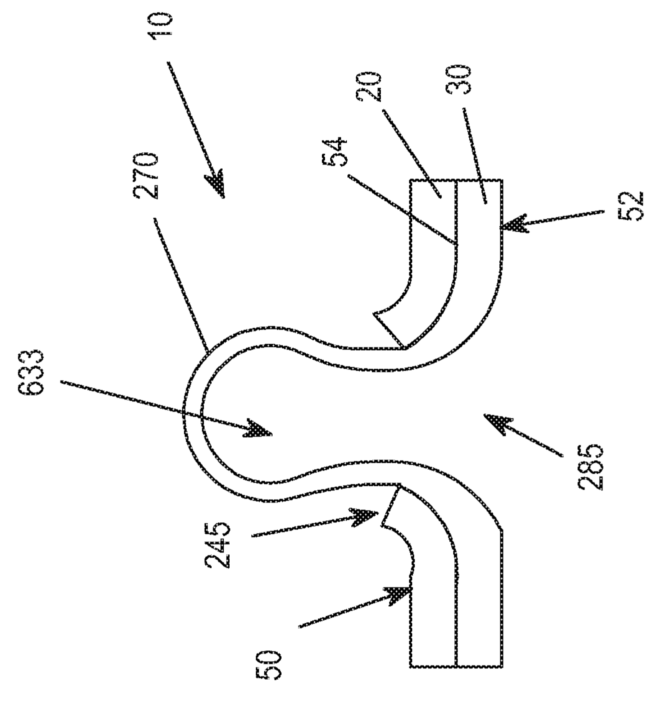
10
20
30
270
54
52
633
285
245
50

Fig. 6A

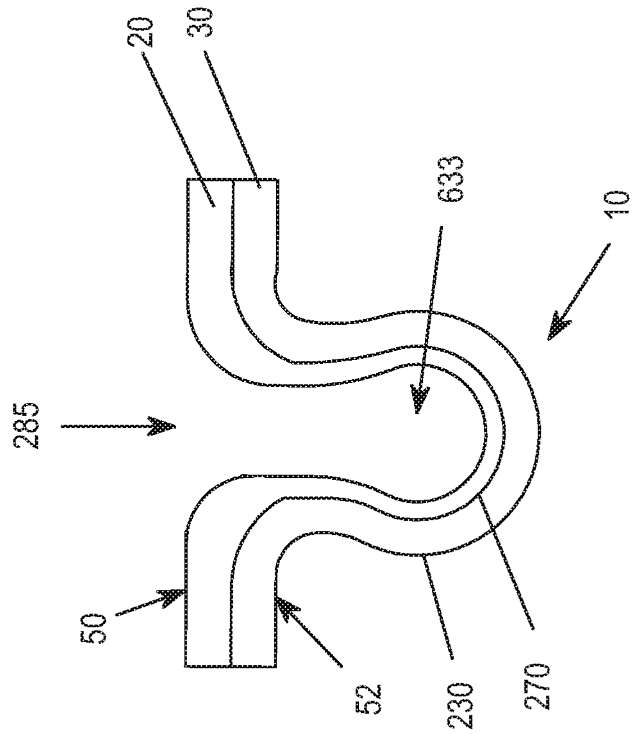


Fig. 6D

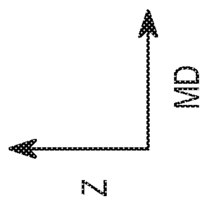


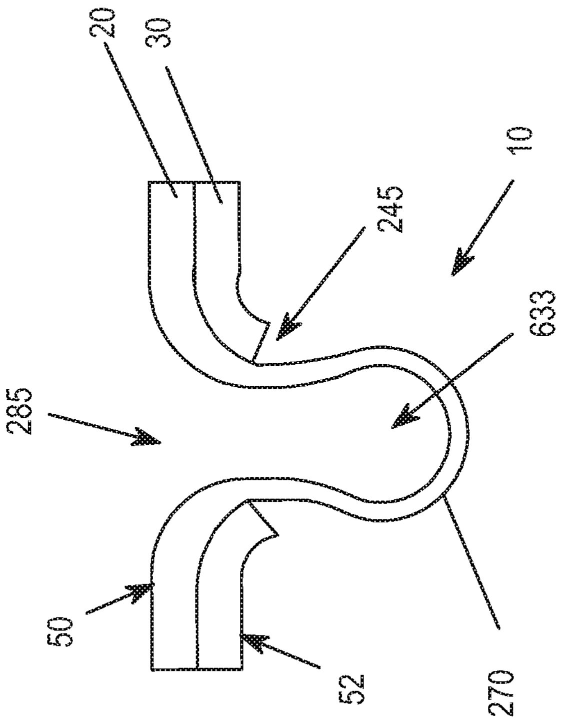


Fig. 6C

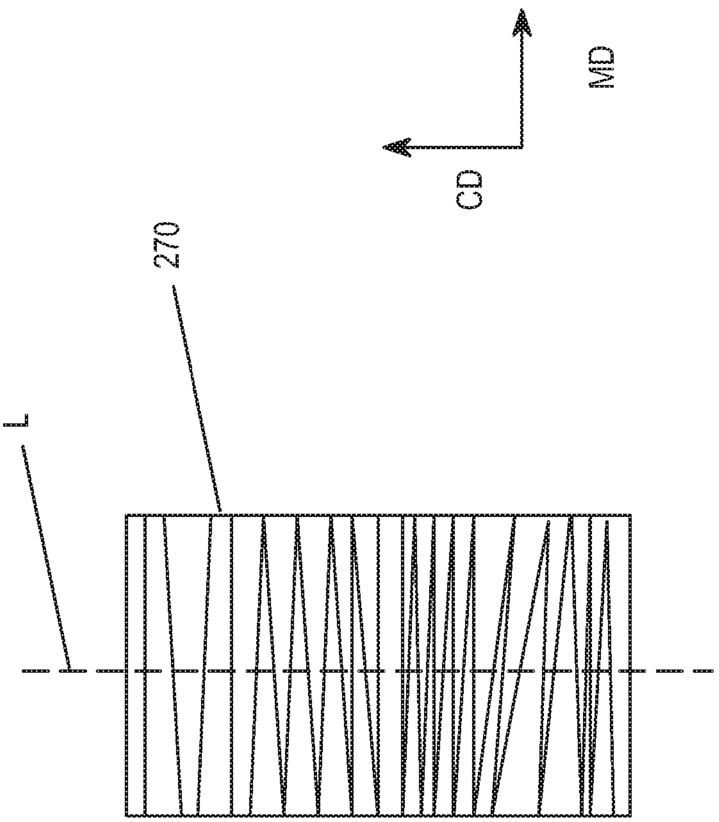


Fig. 6E

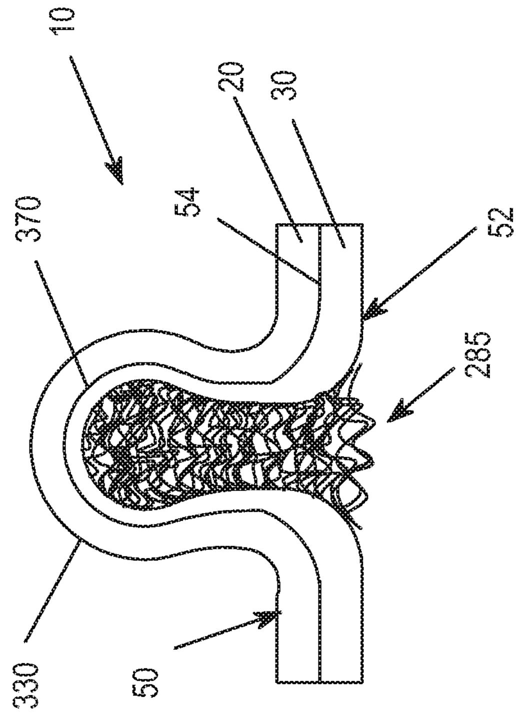


Fig. 7B

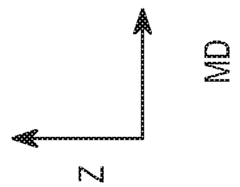


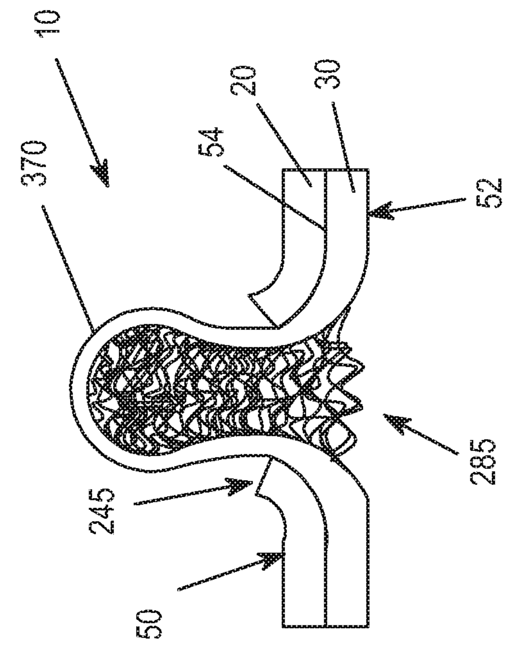


Fig. 7A

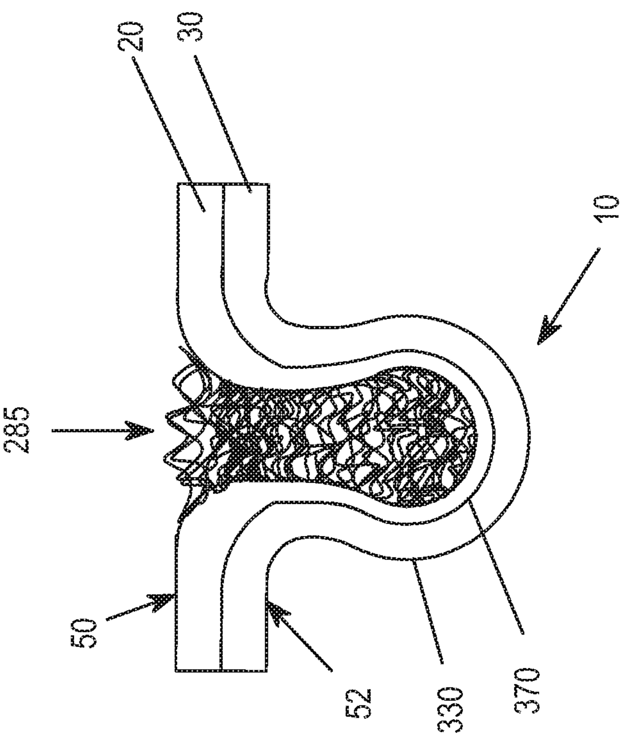


Fig. 7D

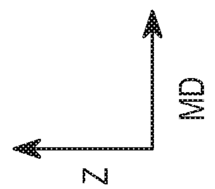


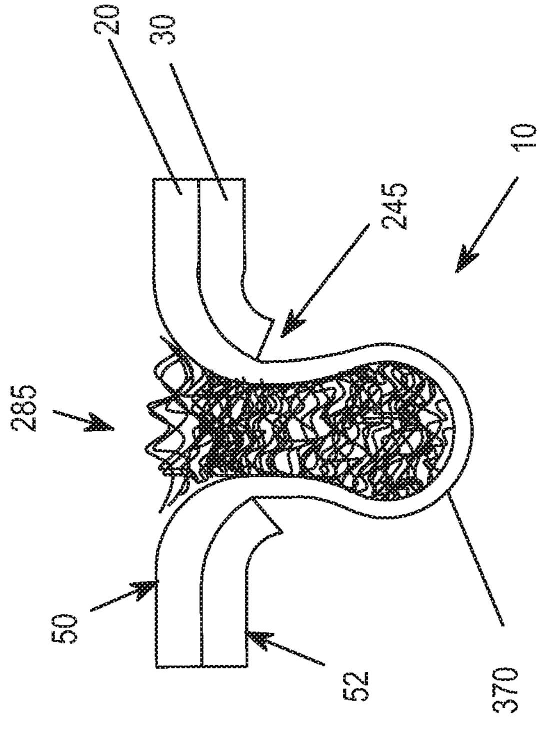


Fig. 7C

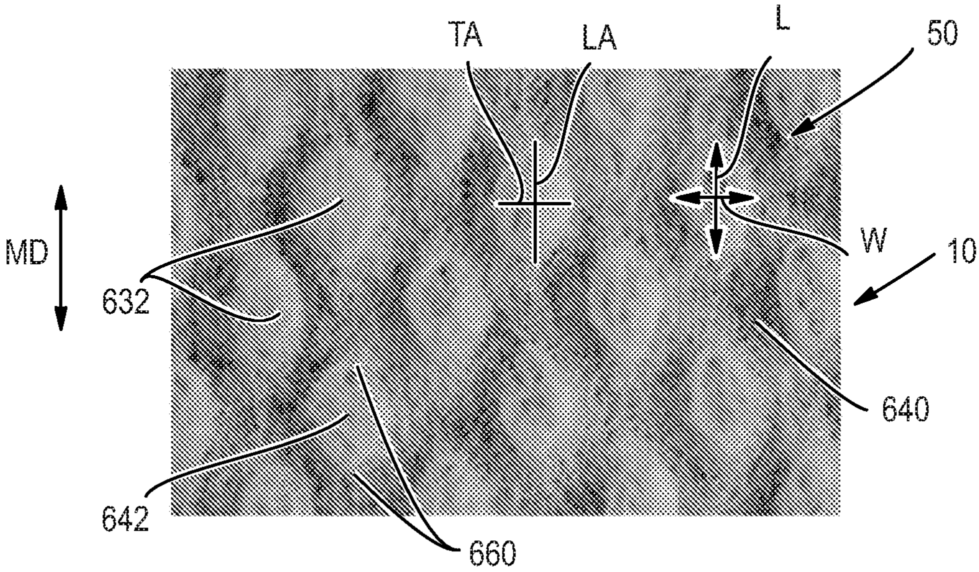


Fig. 8A

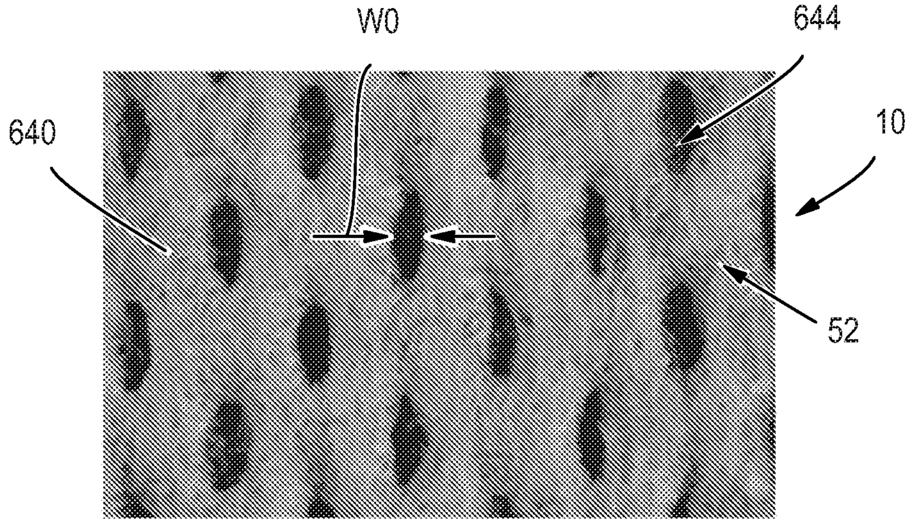


Fig. 8B

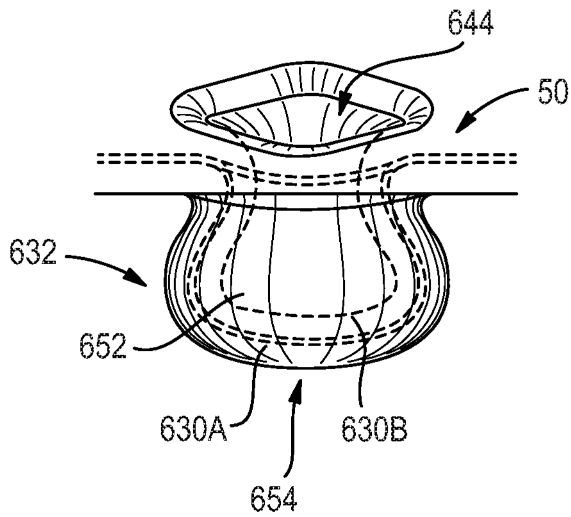
644
50
632
652
630A
630B
654

Fig. 8C

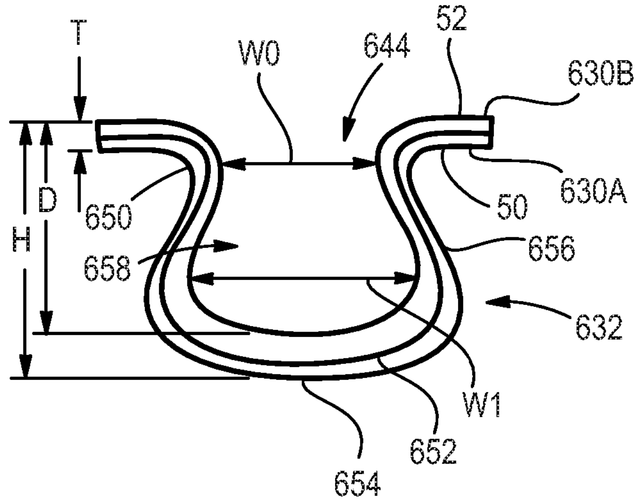
T
W0
644
52
630B
D
650
50
630A
H
658
656
632
W1
652
654

Fig. 8D

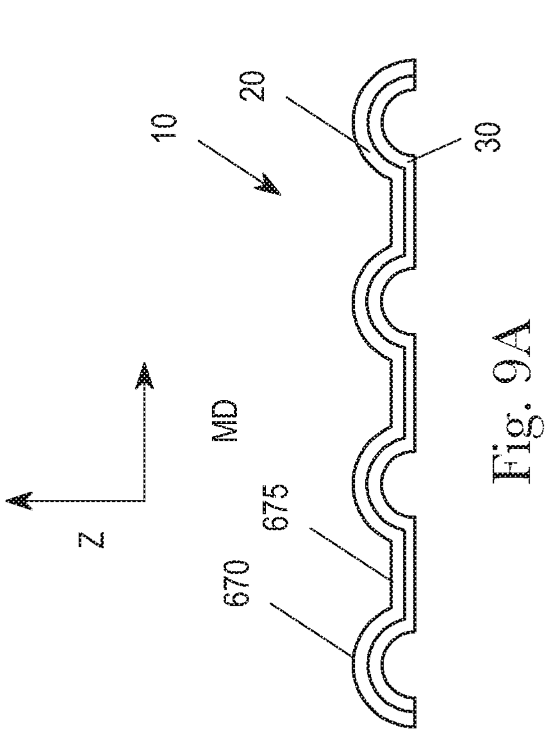


Fig. 9A

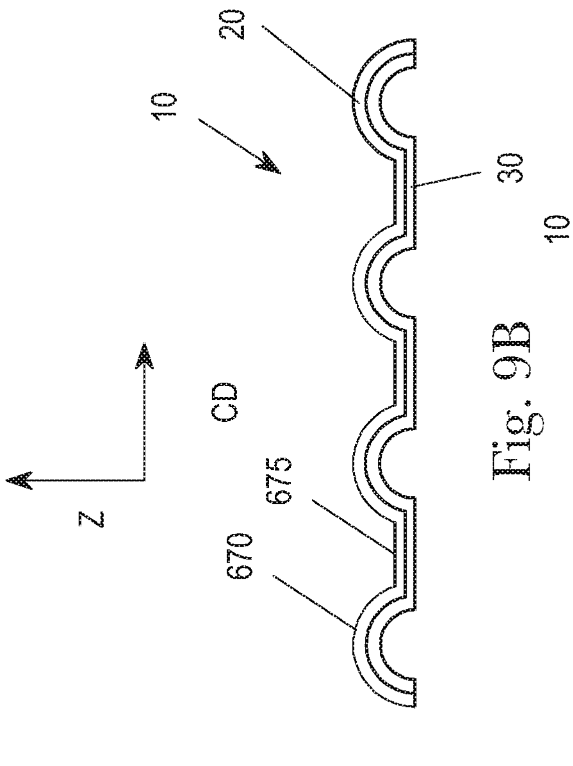


Fig. 9B

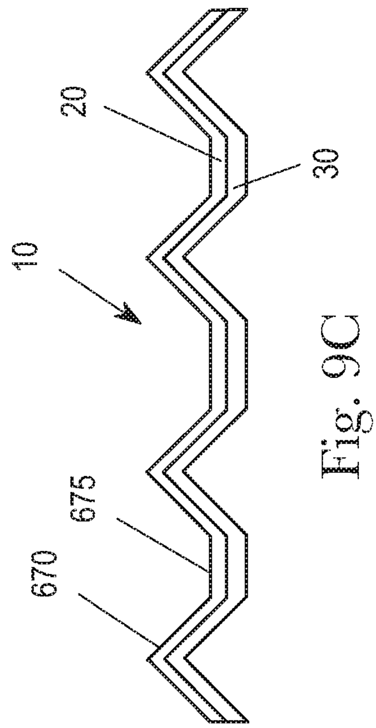


Fig. 9C

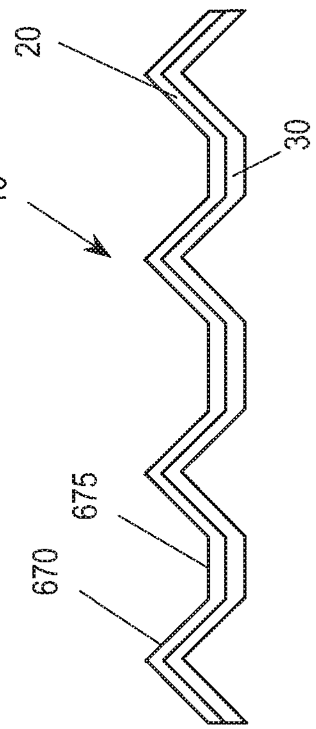


Fig. 9D

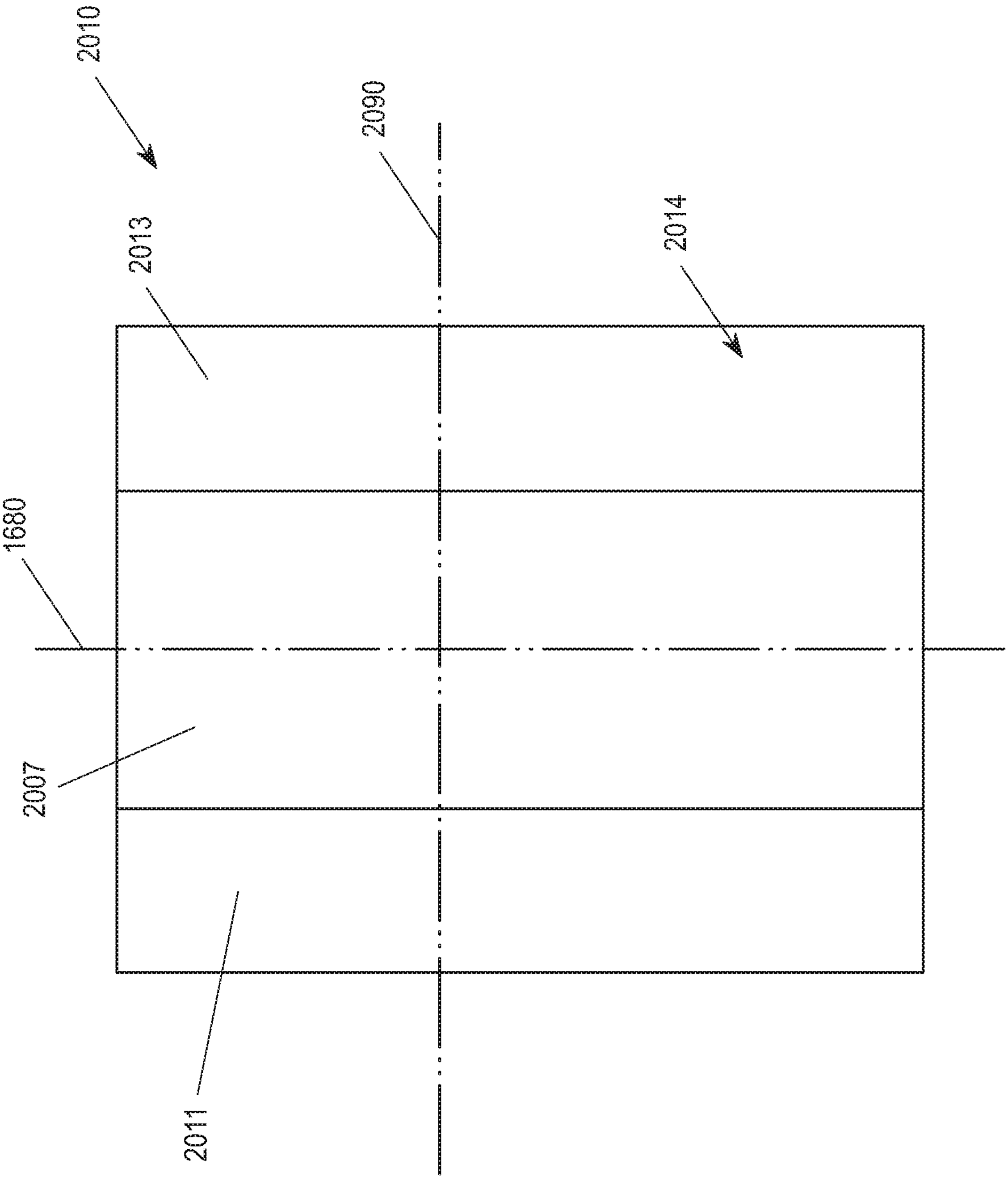


Fig. 10

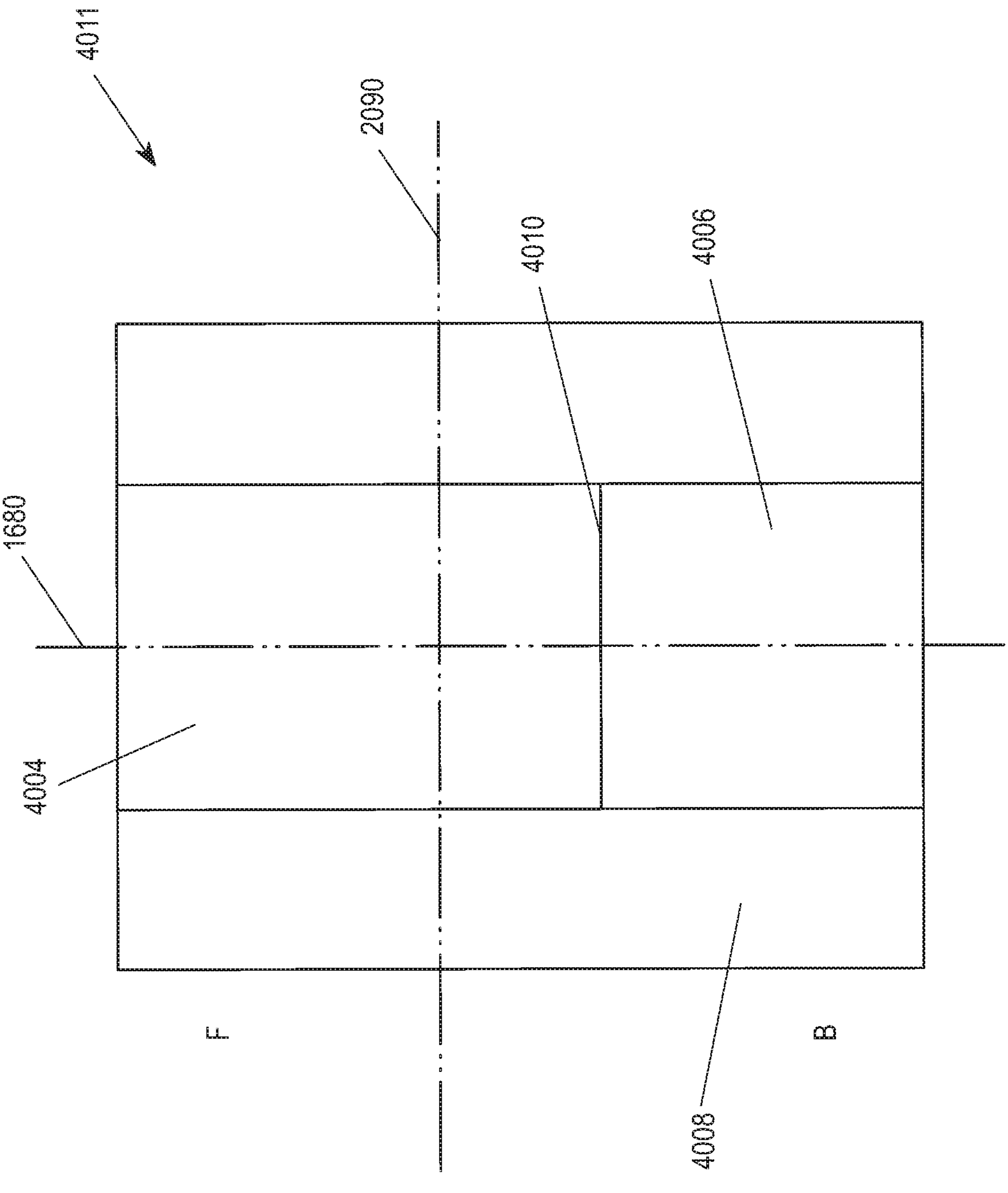


Fig. 11

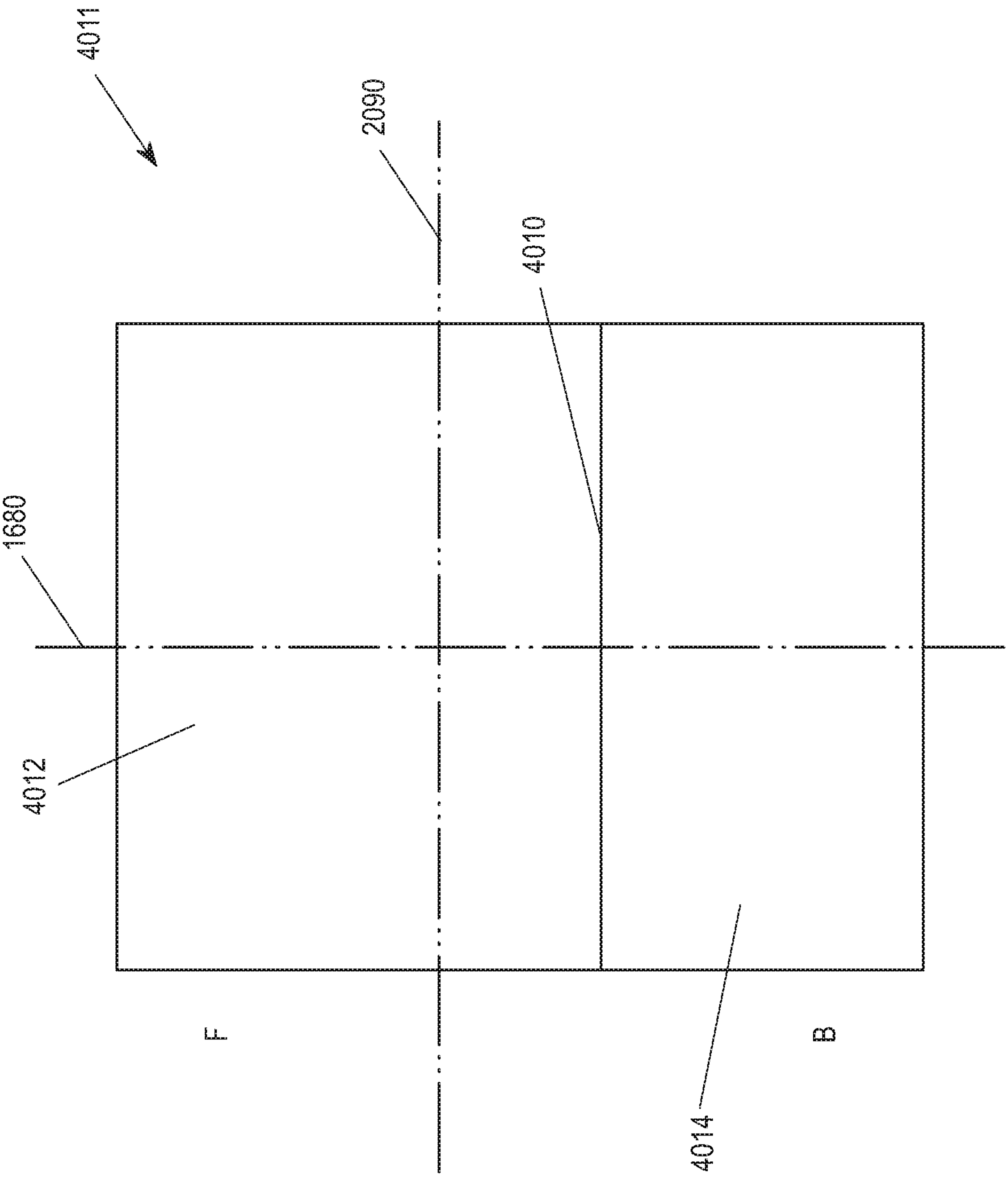


Fig. 12

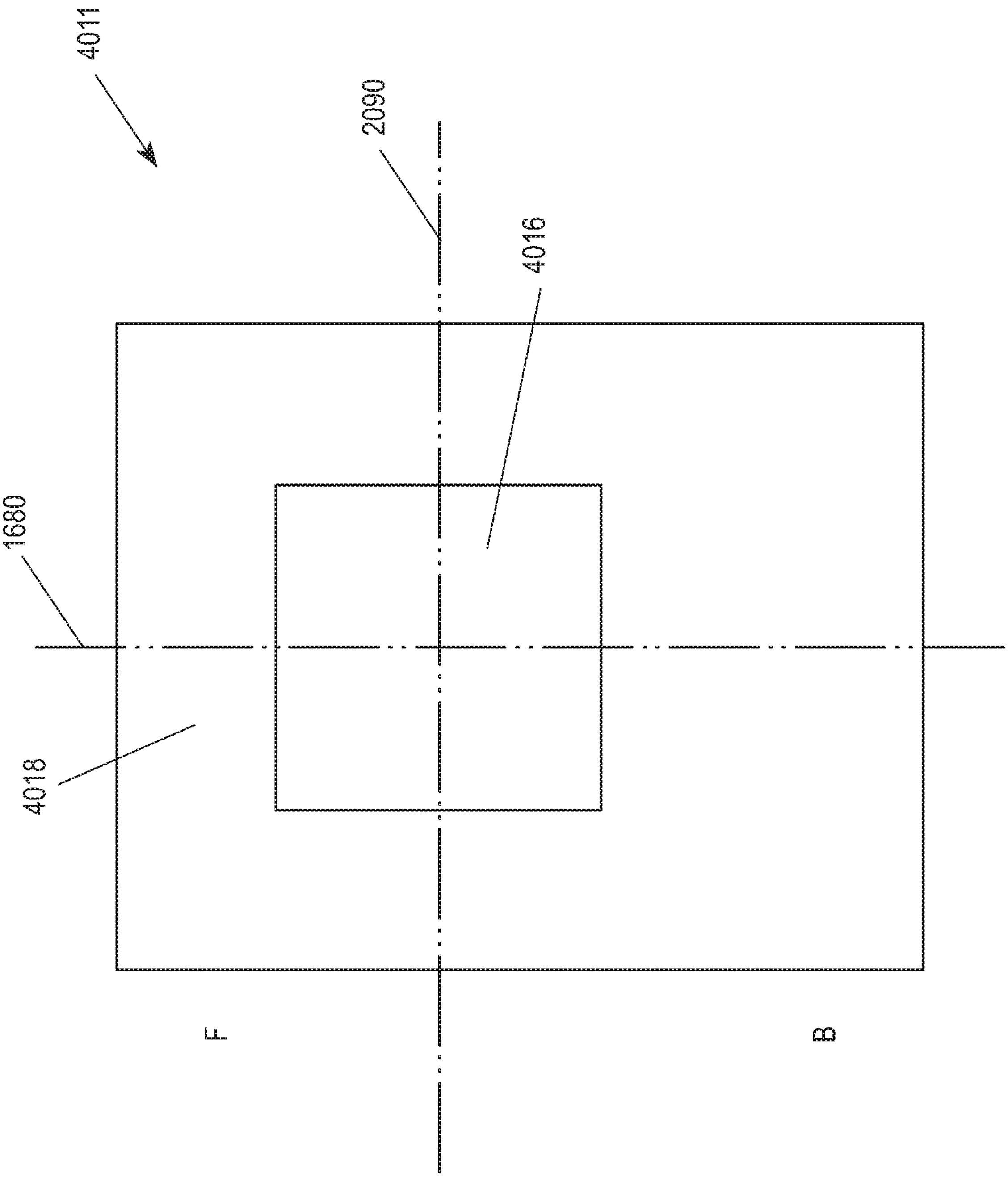


Fig. 13

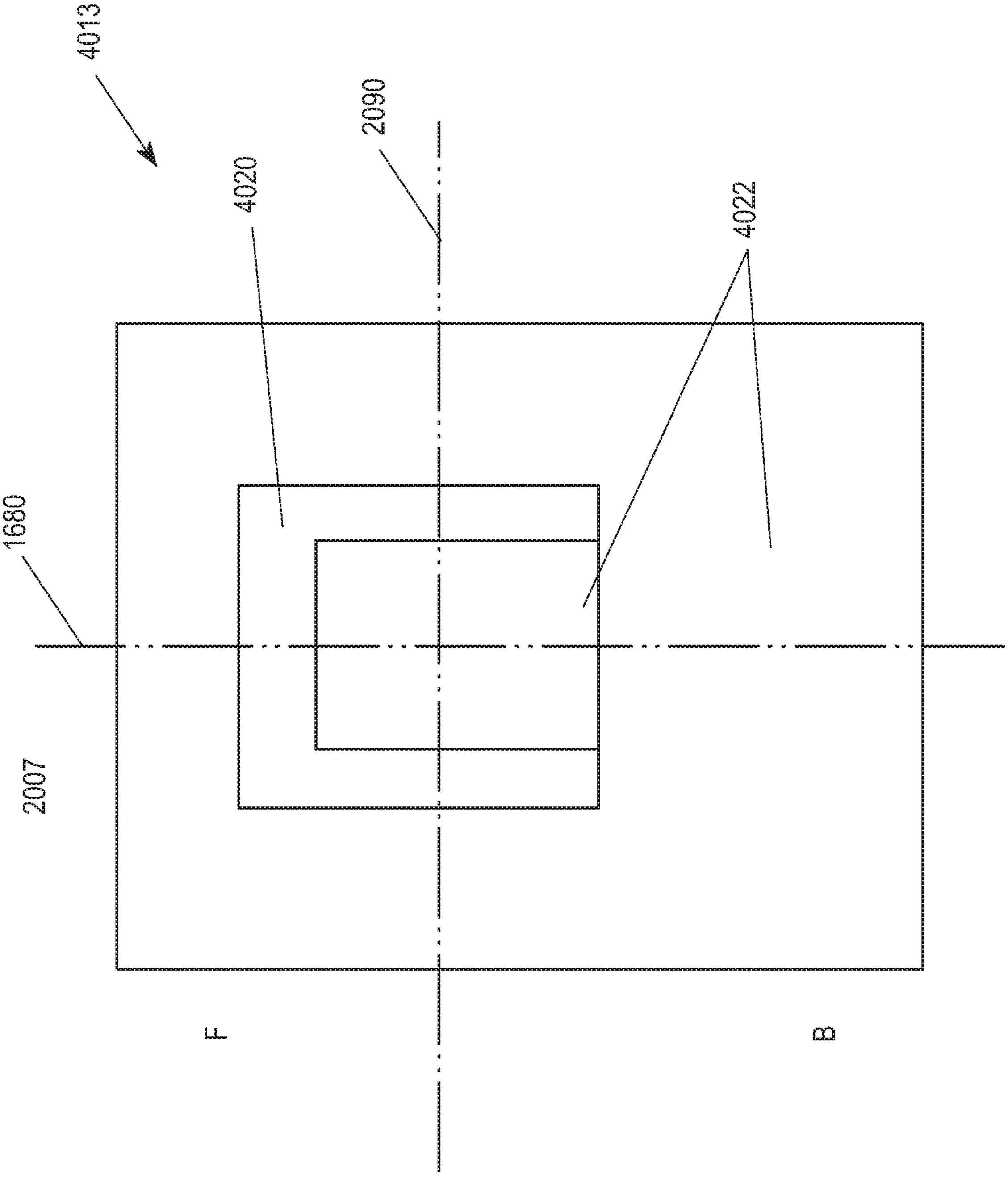


Fig. 14

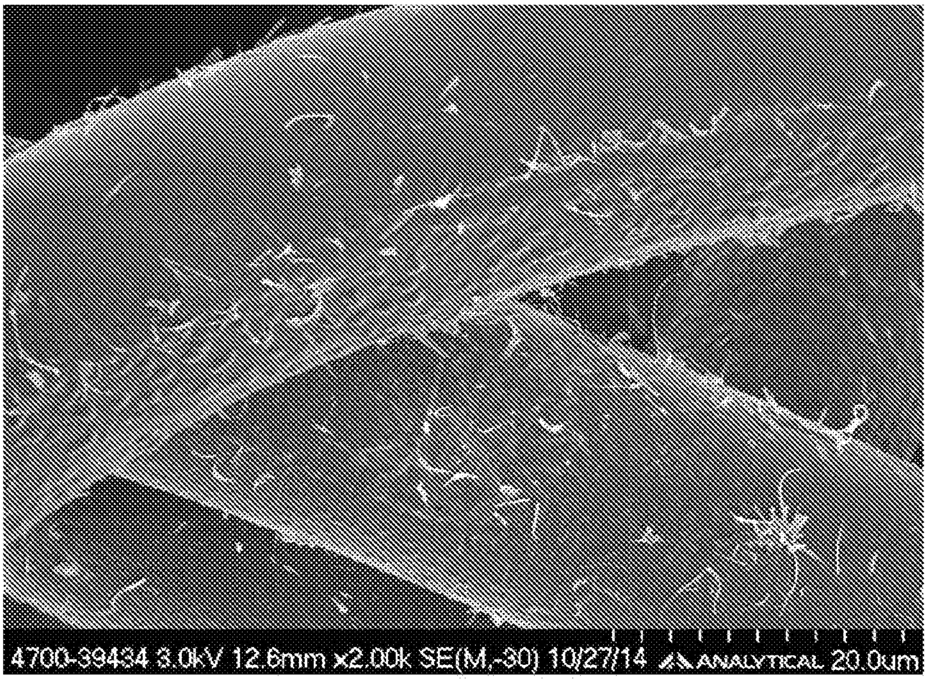


Fig. 15A

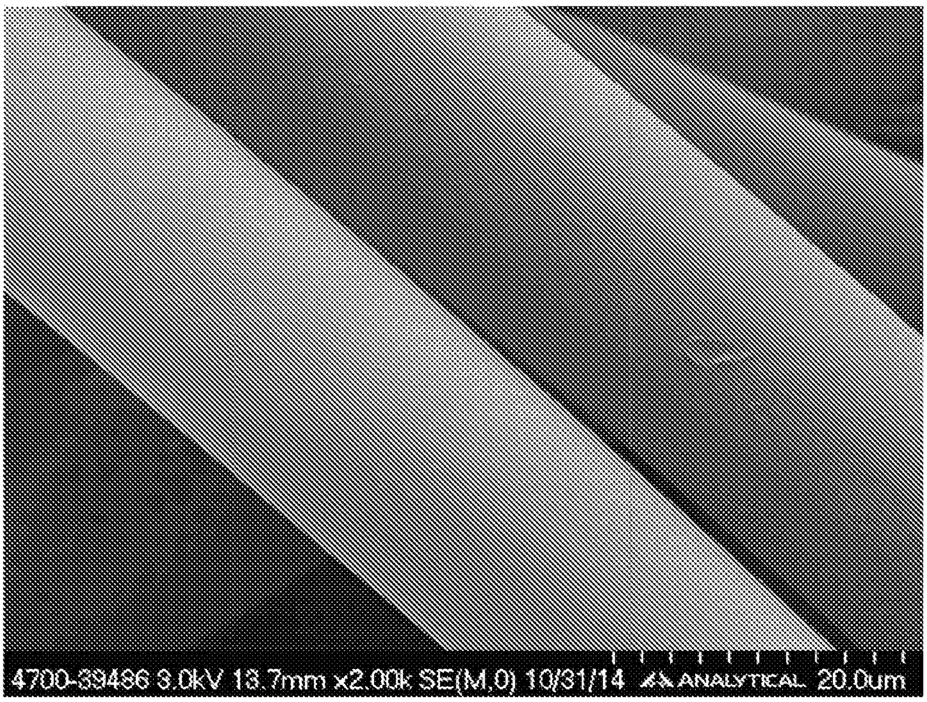


Fig. 15B

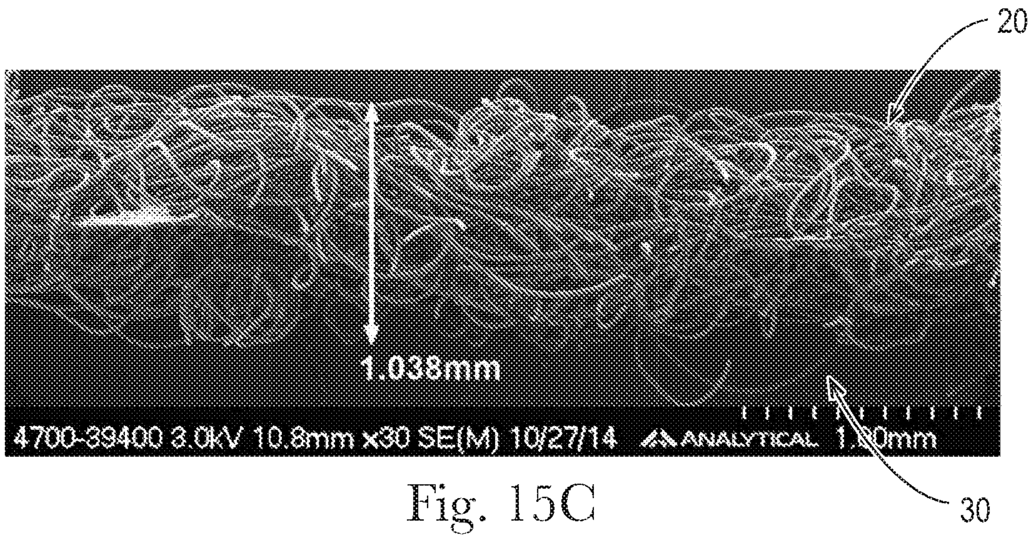


Fig. 15C

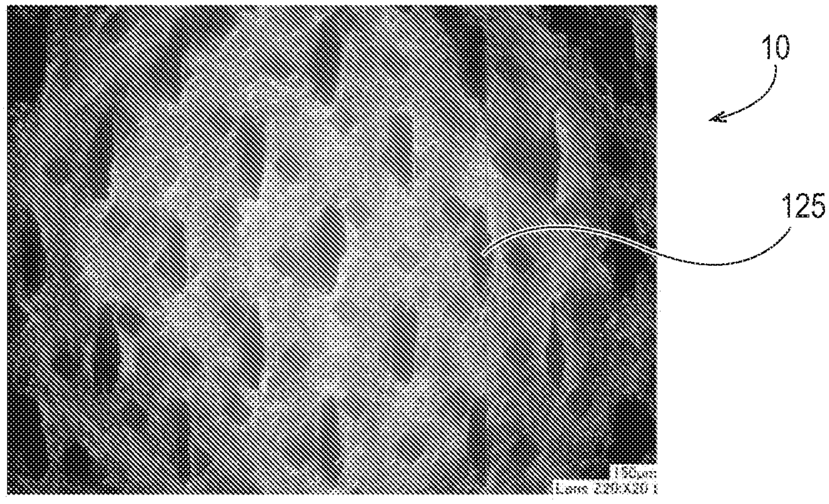


Fig. 16A

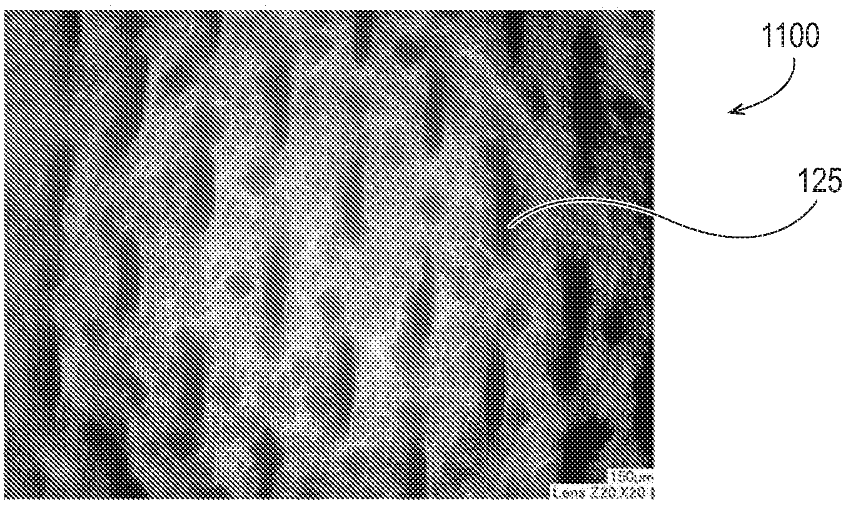


Fig. 16B

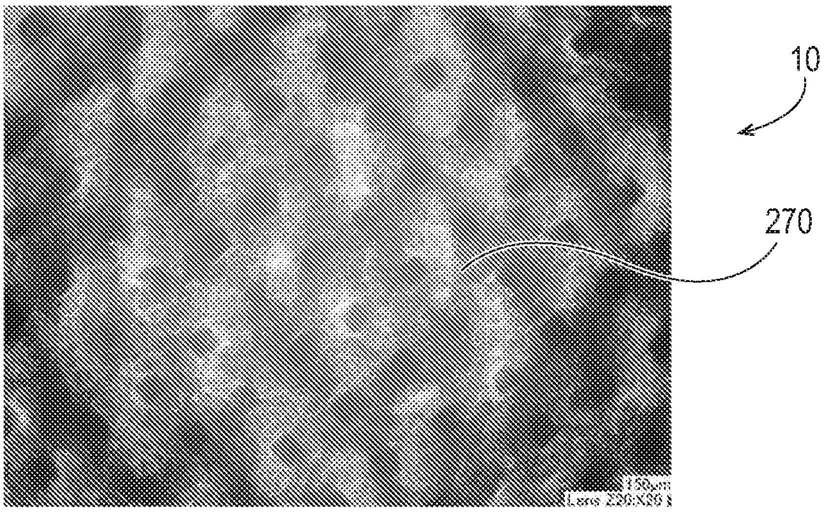


Fig. 17A

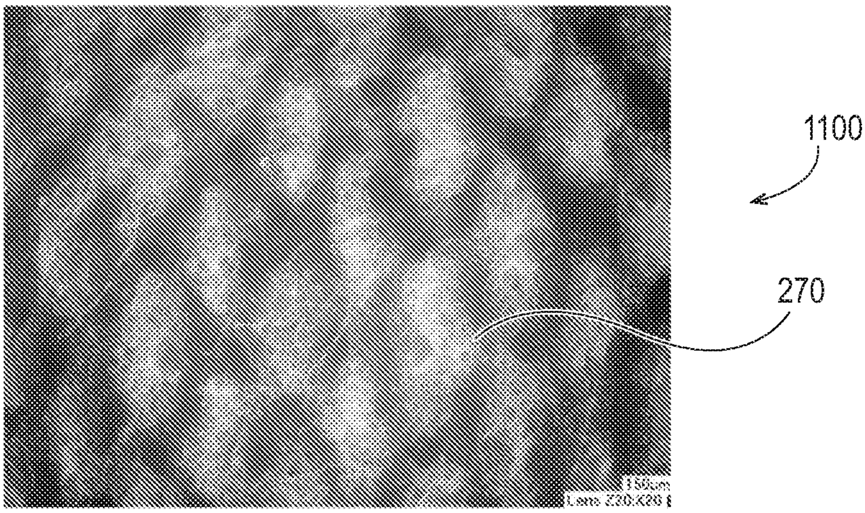


Fig. 17B

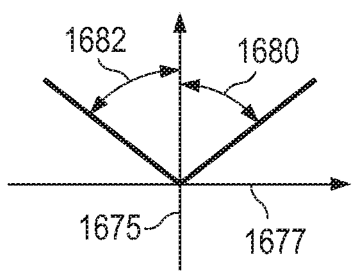


Fig. 18

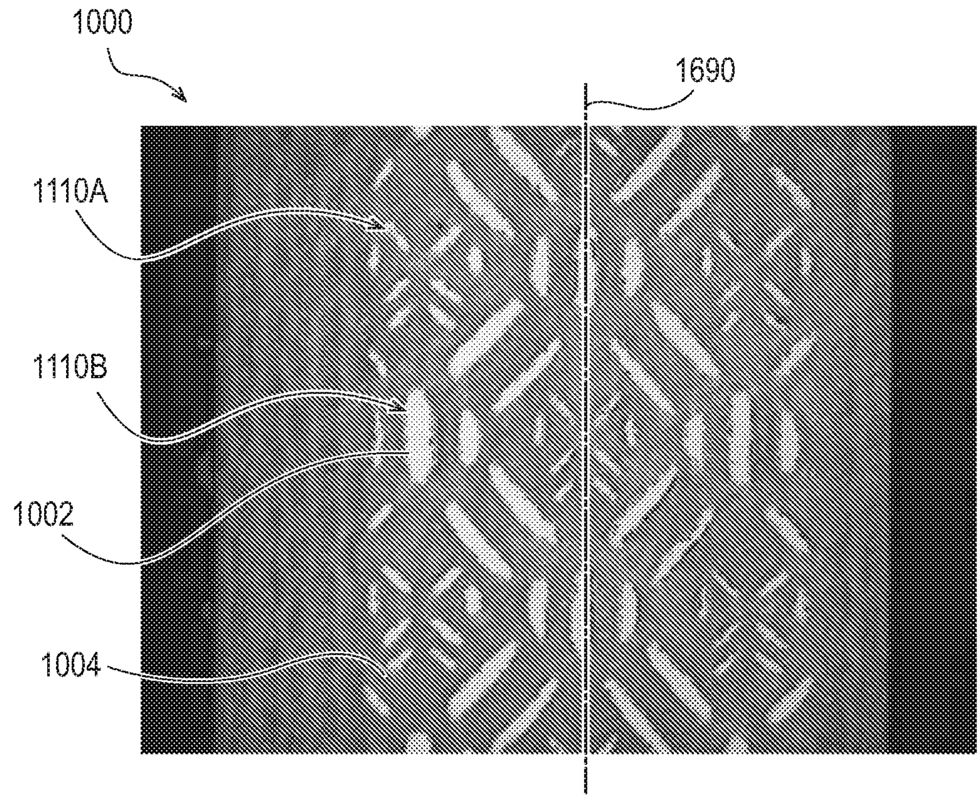


Fig. 19

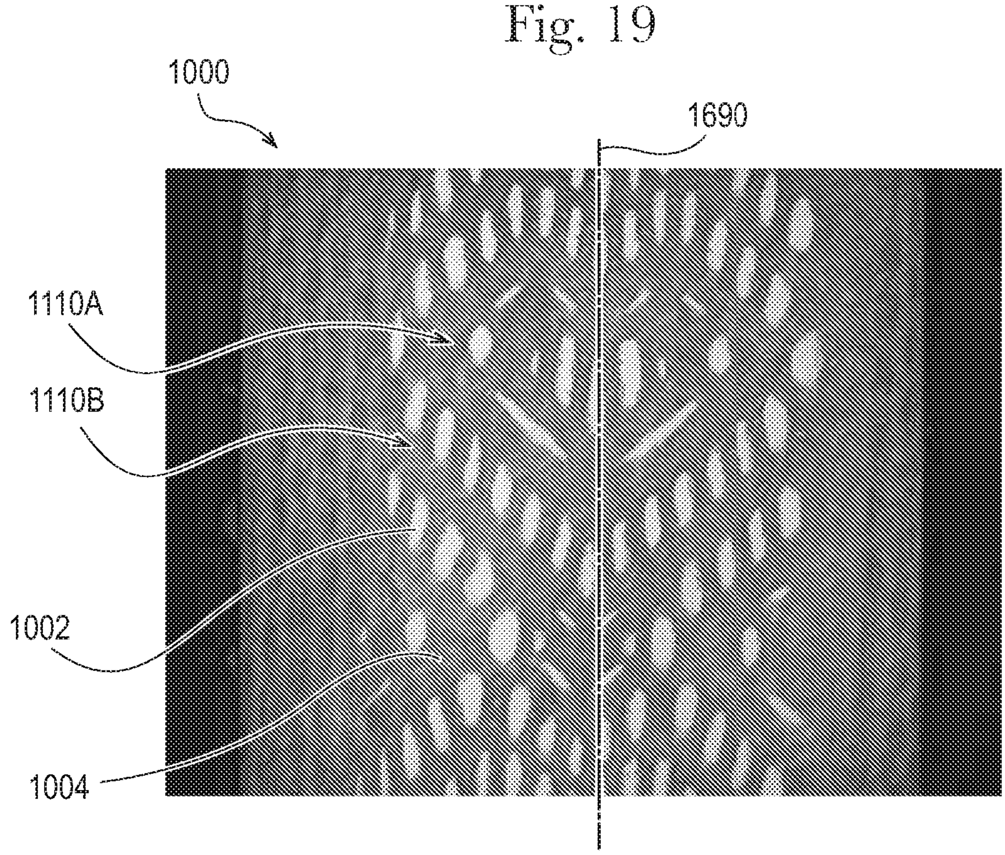


Fig. 20A

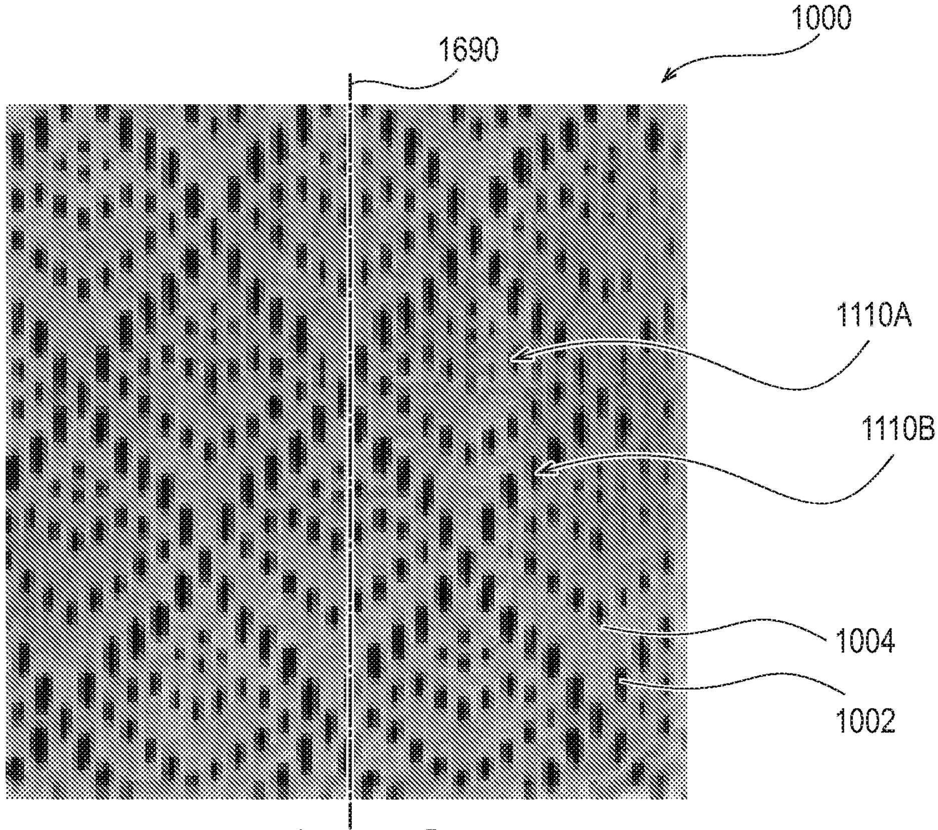


Fig. 20B

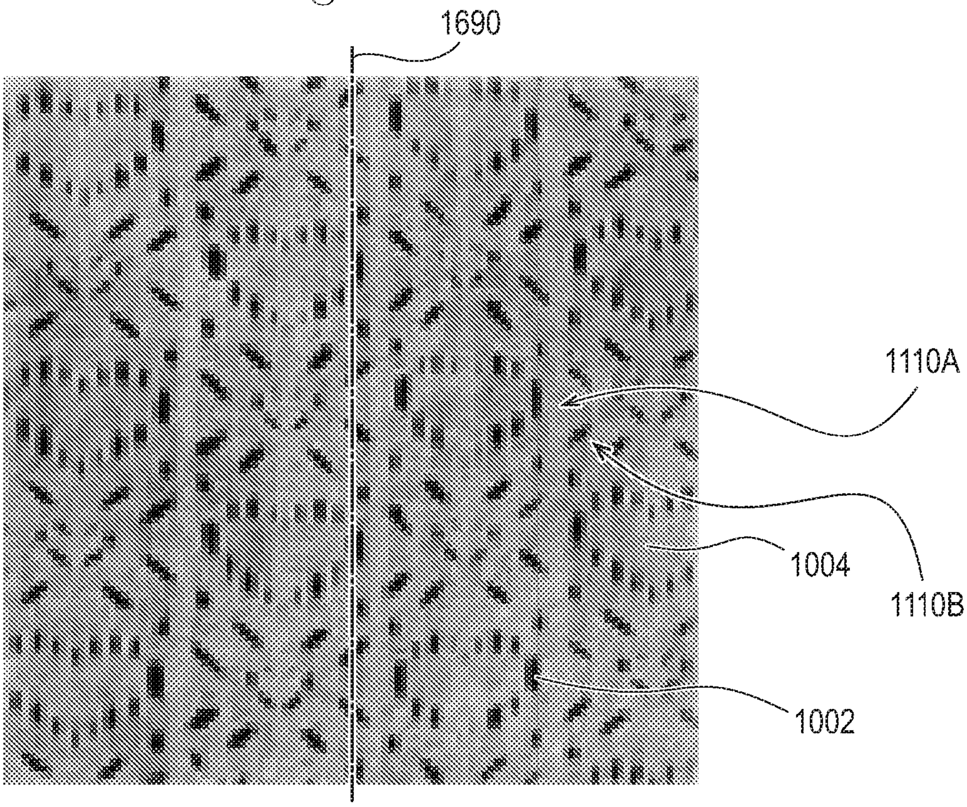


Fig. 20C

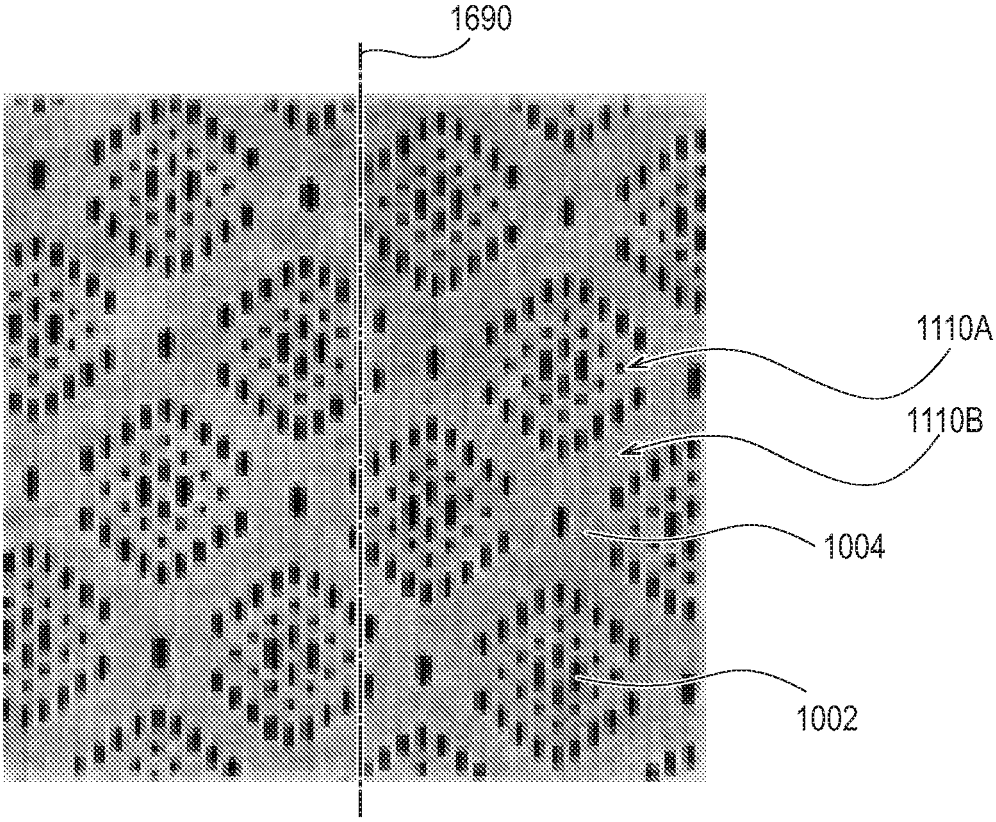


Fig. 20D

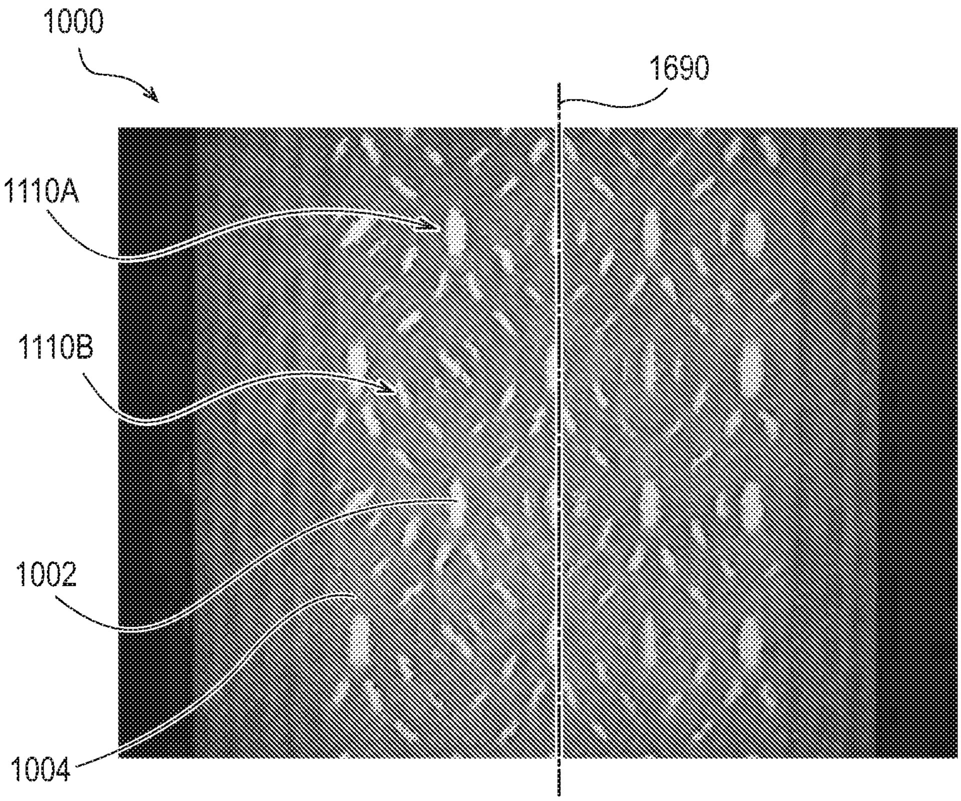
1000
1690
1110A
1110B
1002
1004

Fig. 21

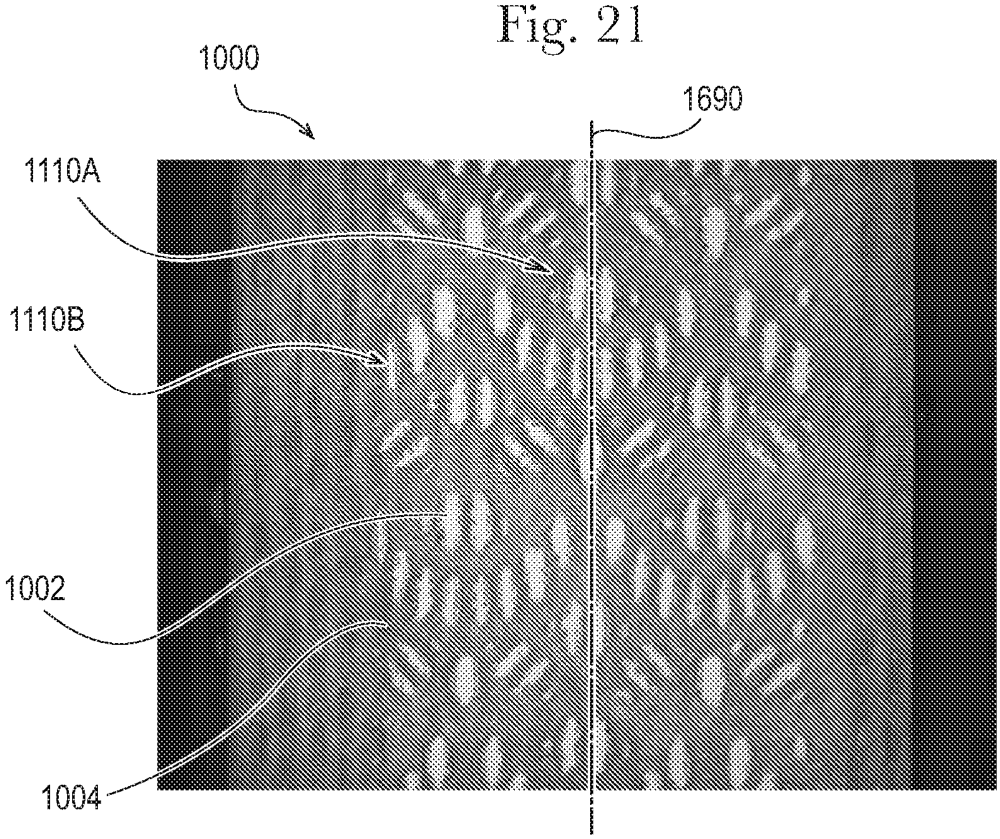
1000
1690
1110A
1110B
1002
1004

Fig. 22

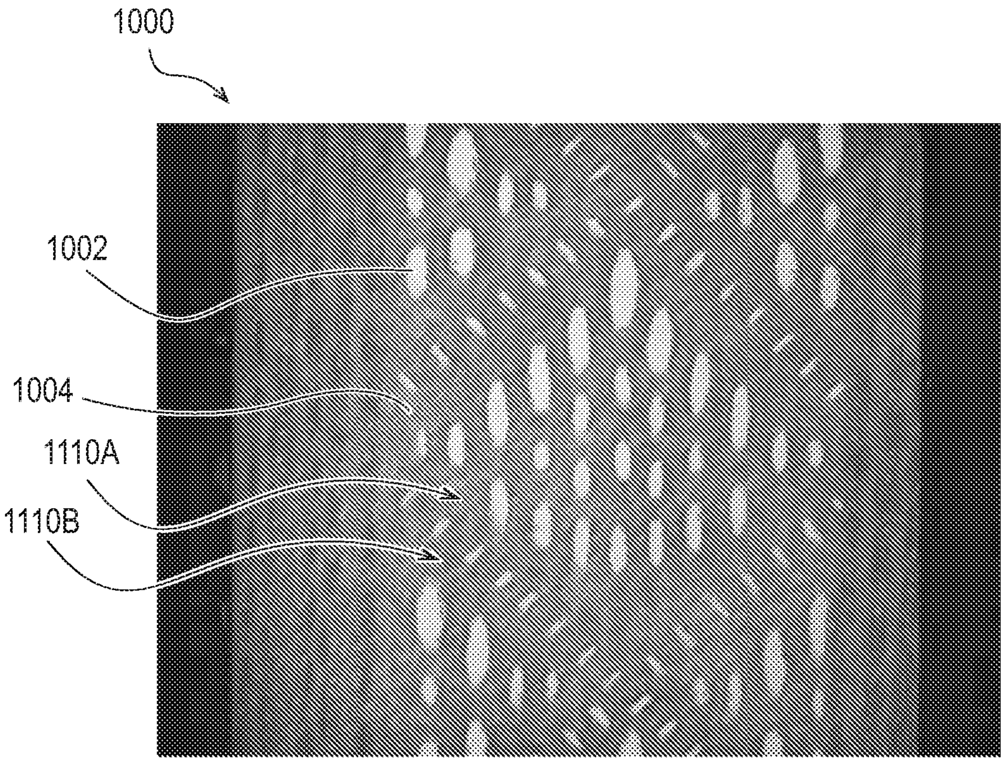


Fig. 23

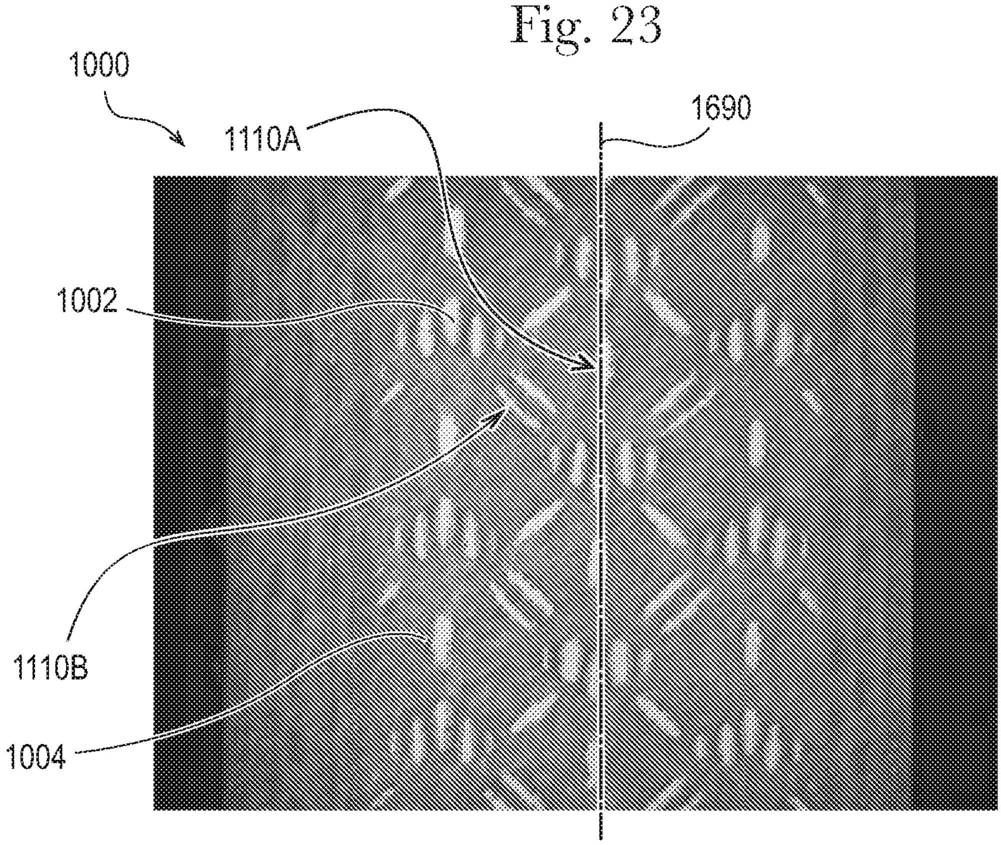


Fig. 24

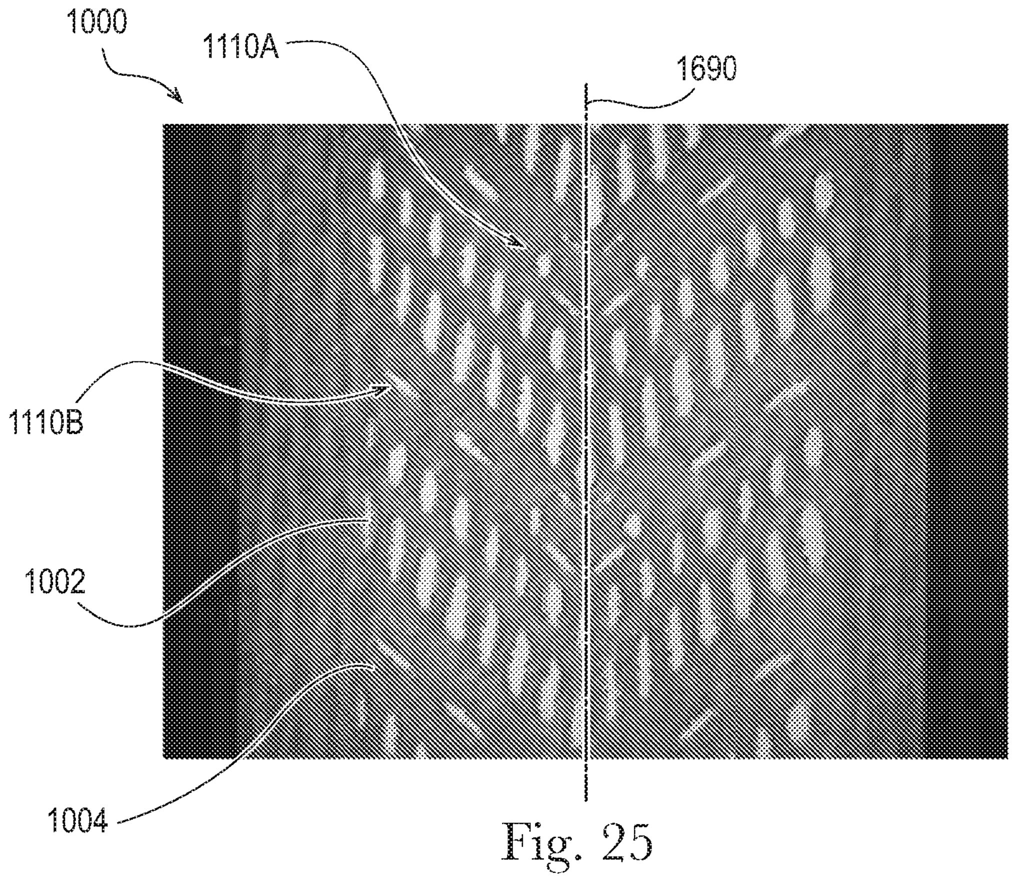
1000
1110A
1690
1110B
1002
1004

Fig. 25

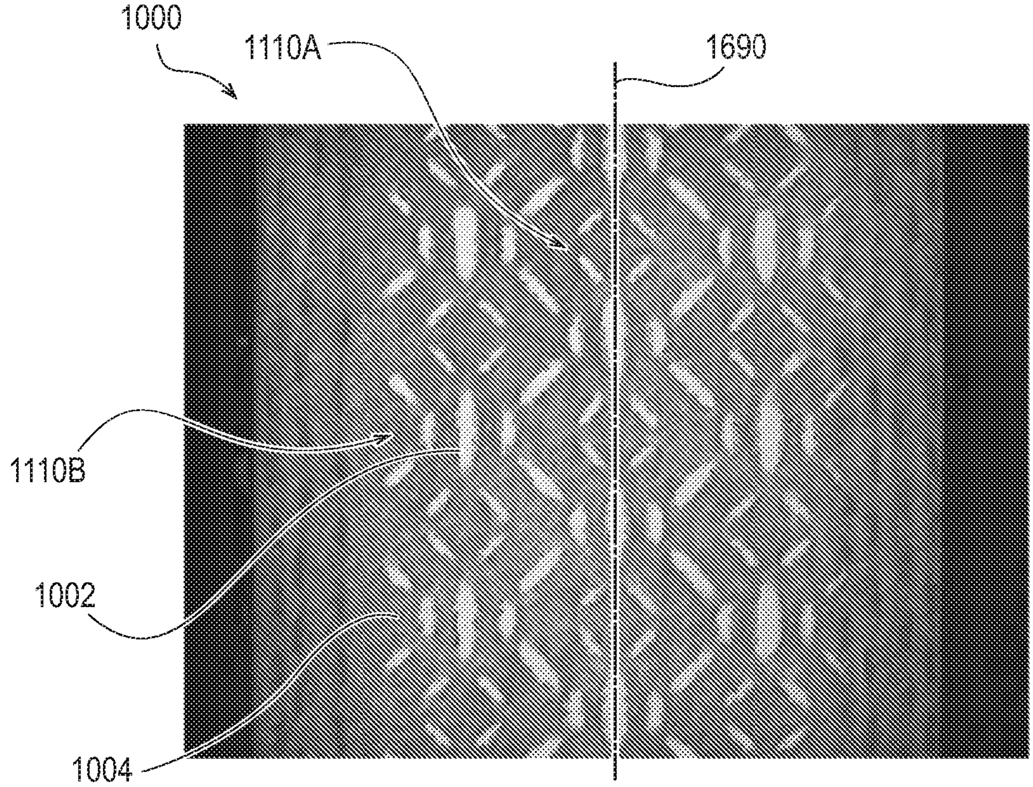
1000
1110A
1690
1110B
1002
1004

Fig. 26

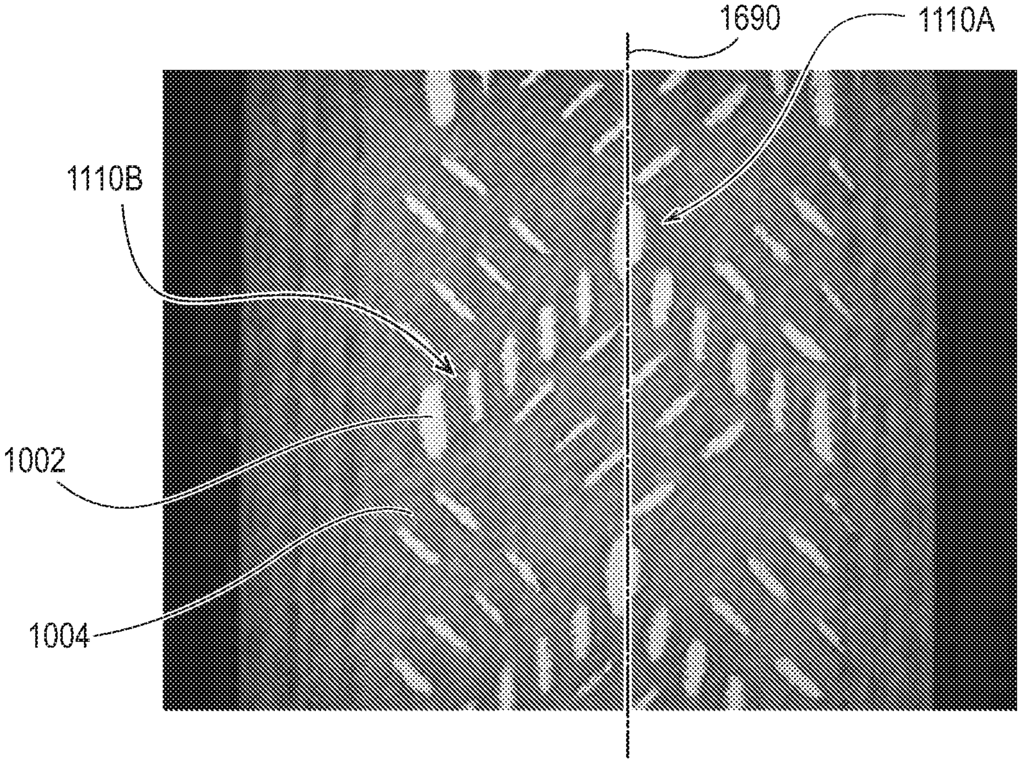


Fig. 27

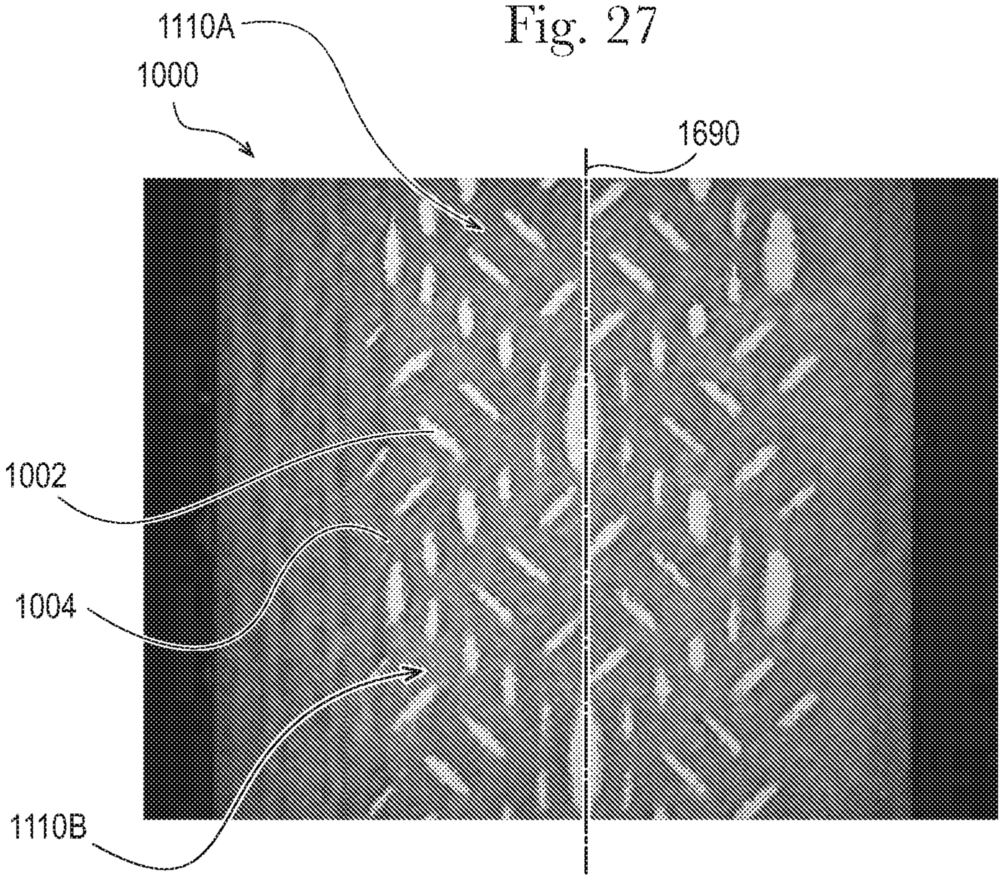


Fig. 28

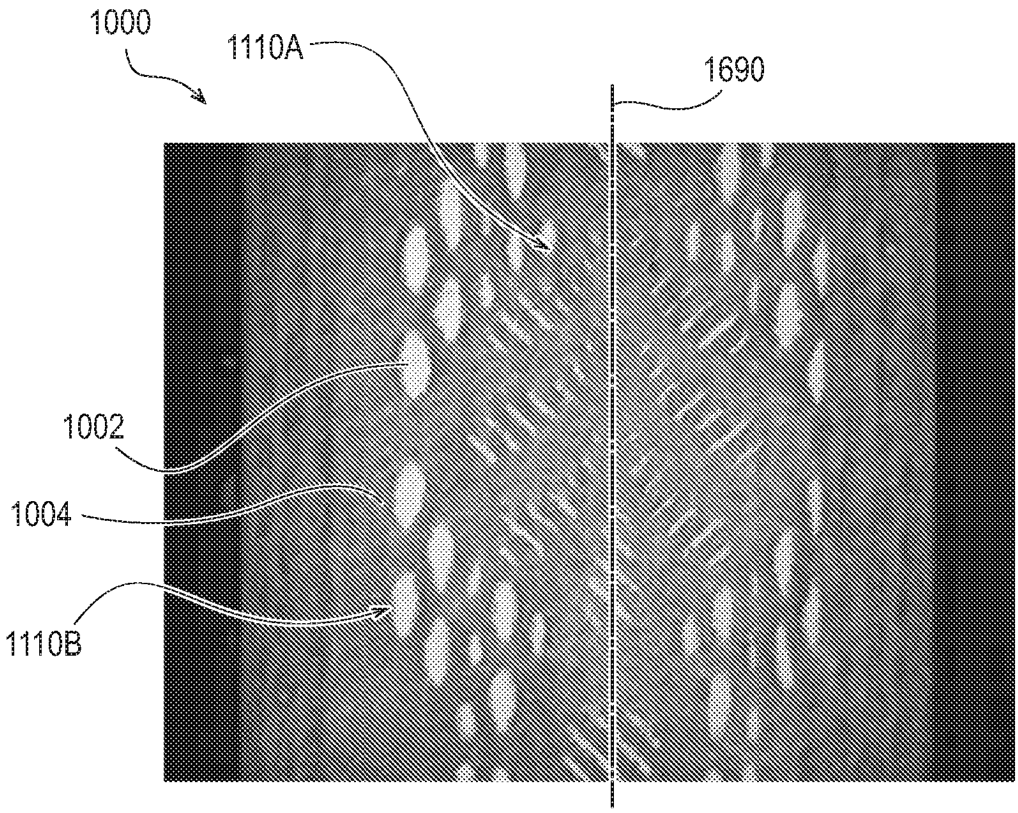
1000
1110A
1690
1002
1004
1110B

Fig. 29

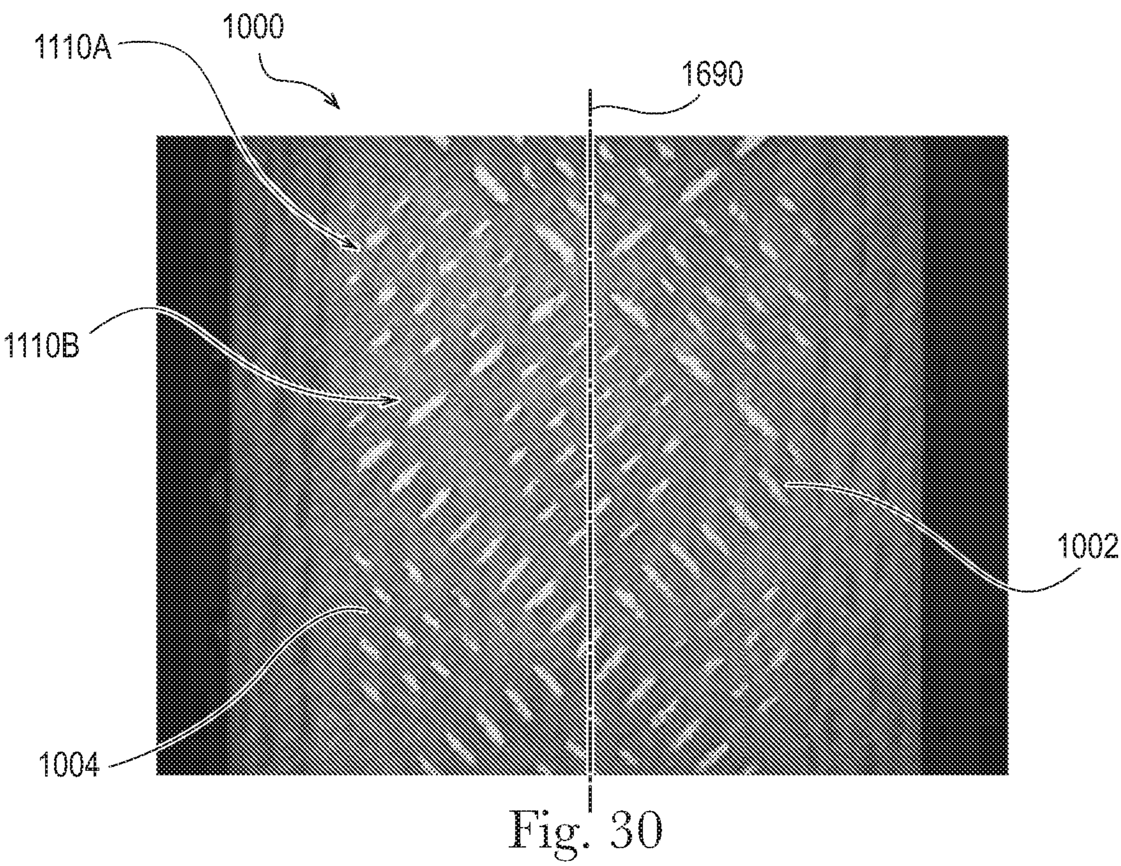
1110A
1000
1690
1110B
1002
1004

Fig. 30

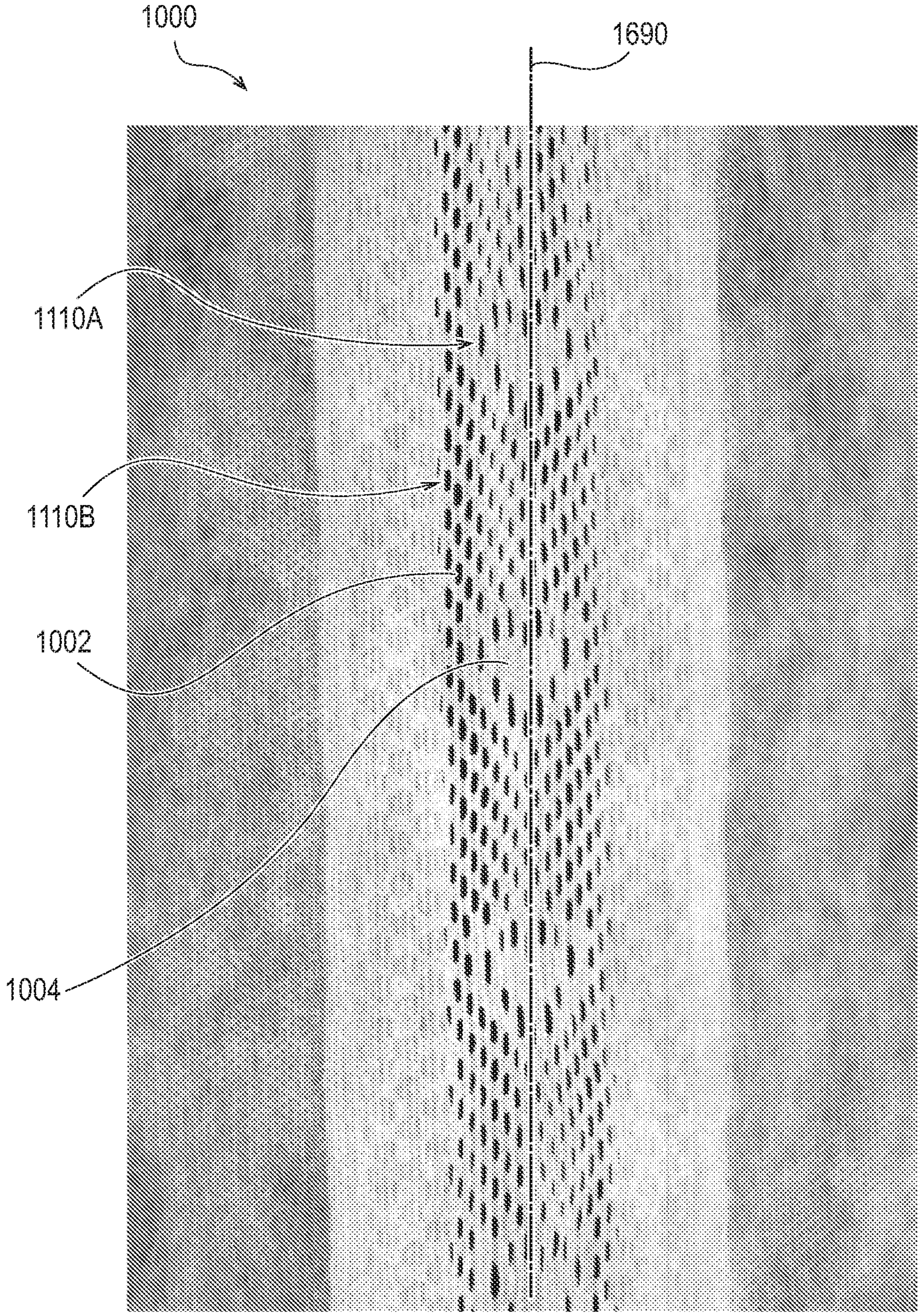


Fig. 31

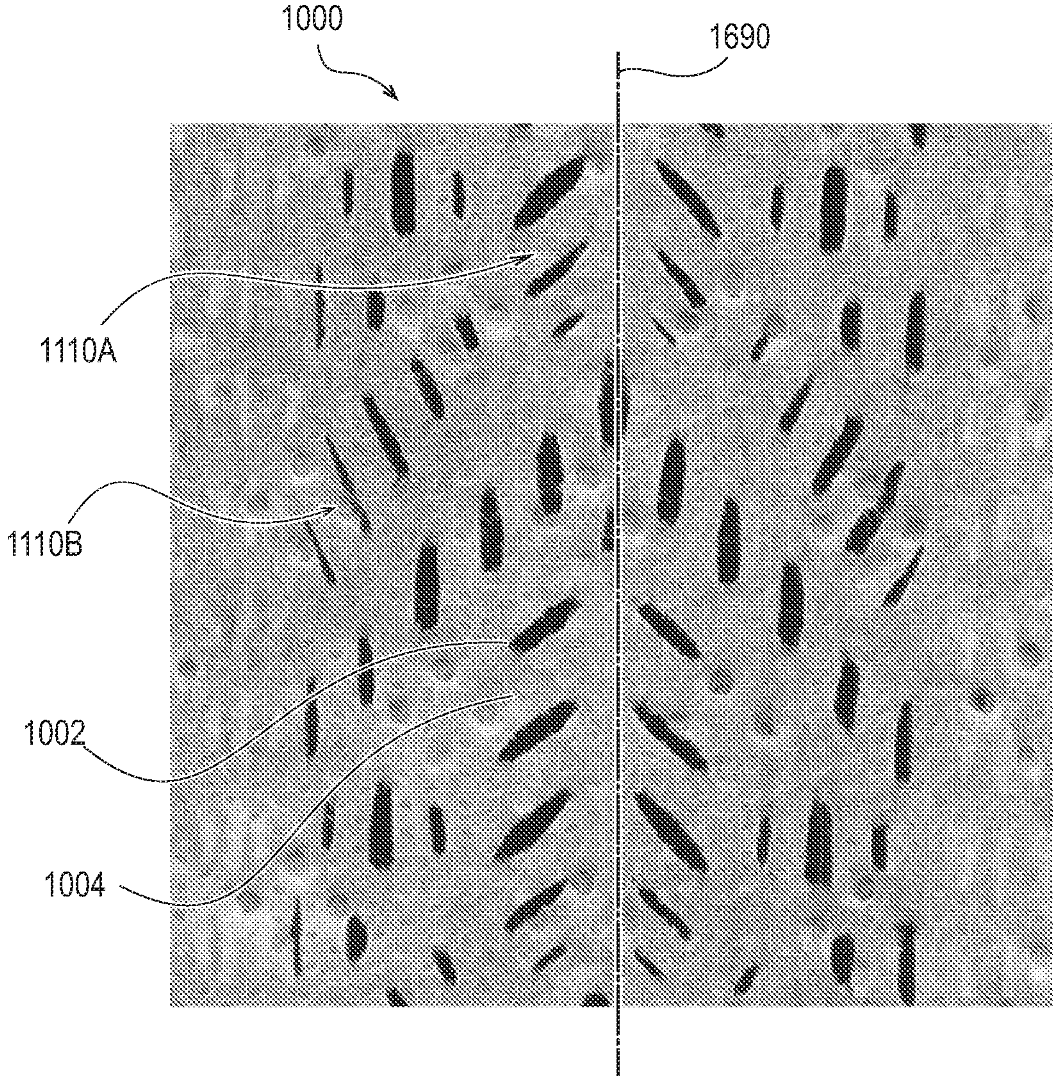


Fig. 32

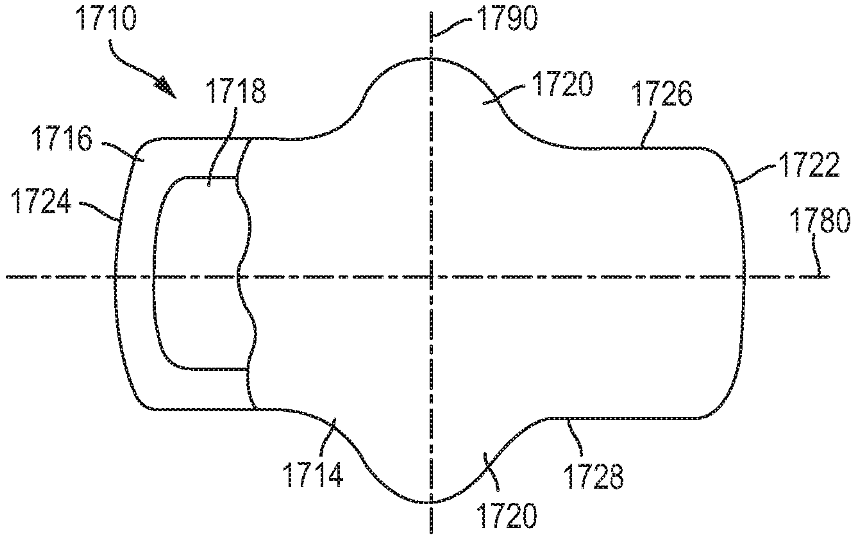


Fig. 33

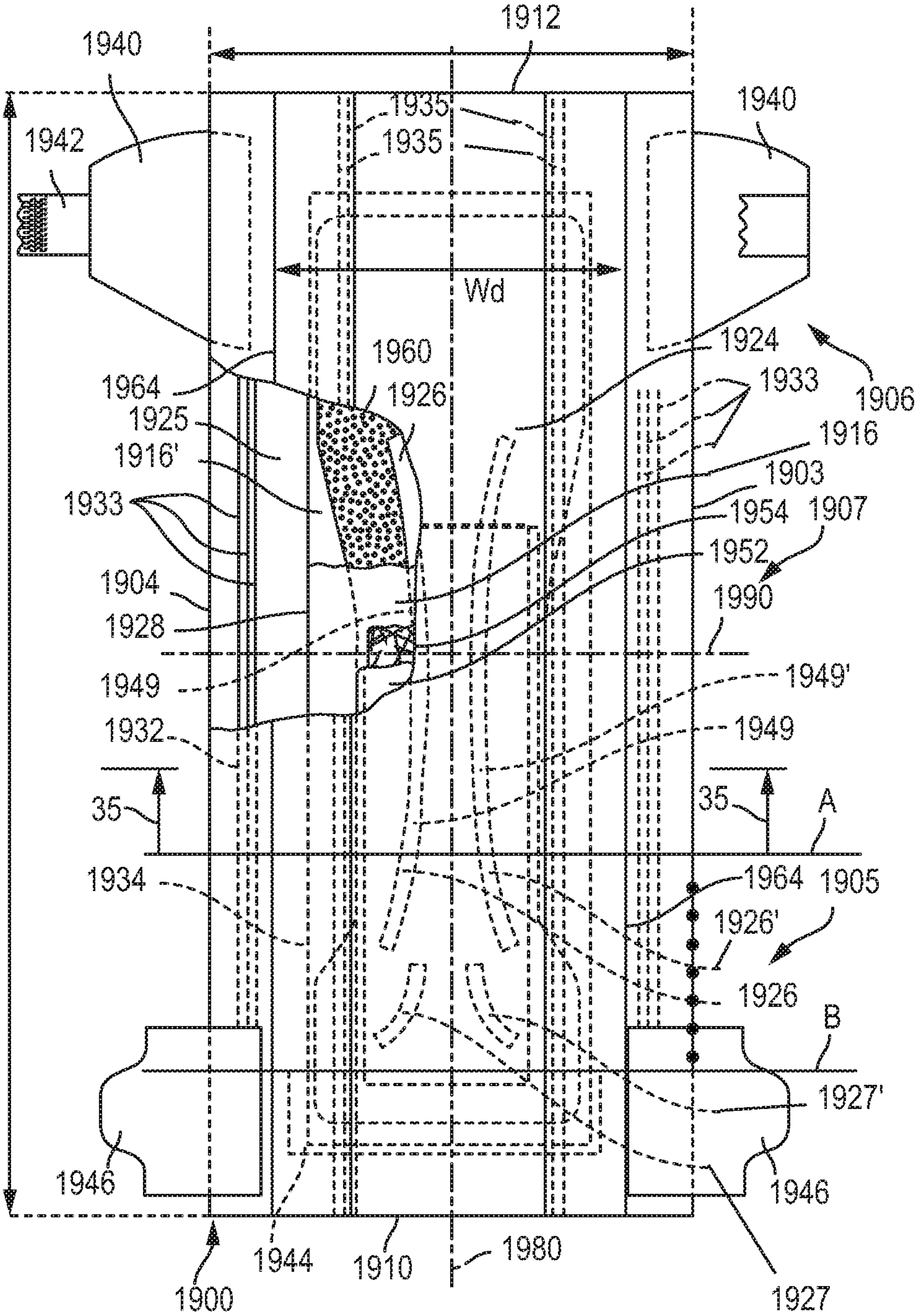
1912
1940
1942
1935
1935
1940
Wd
1924
1960
1933
1906
1964
1926
1925
1916
1916'
1903
1933
1954
1907
1952
1904
1990
1928
1949
1949'
1932
1949
35
35
A
1934
1964
1905
1926'
1926
B
1927'
1946
1946
1944
1910
1980
1900
1927

Fig. 34

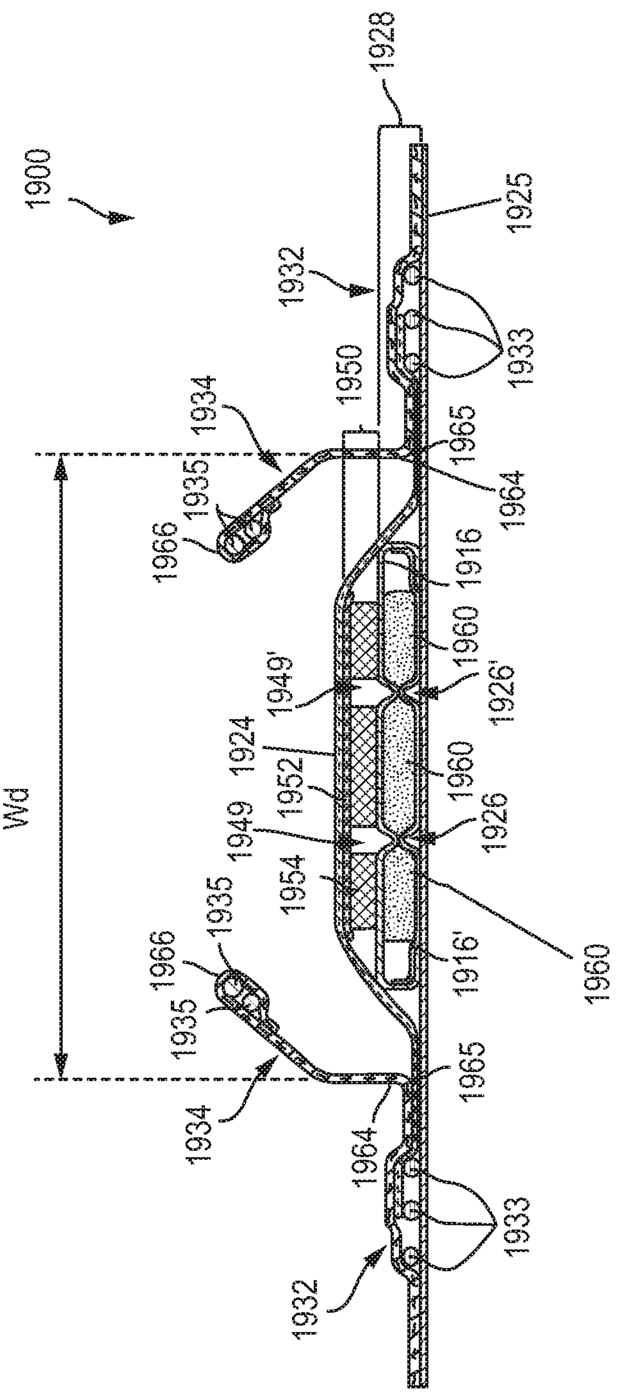
1900
1928
1925
1932
1933
1950
1934
1965
1964
1935
1966
1916
1960
1949'
1926'
1924
1952
Wd
1949
1926
1954
1916'
1960
1935
1966
1935
1934
1965
1964
1933
1932

Fig. 35

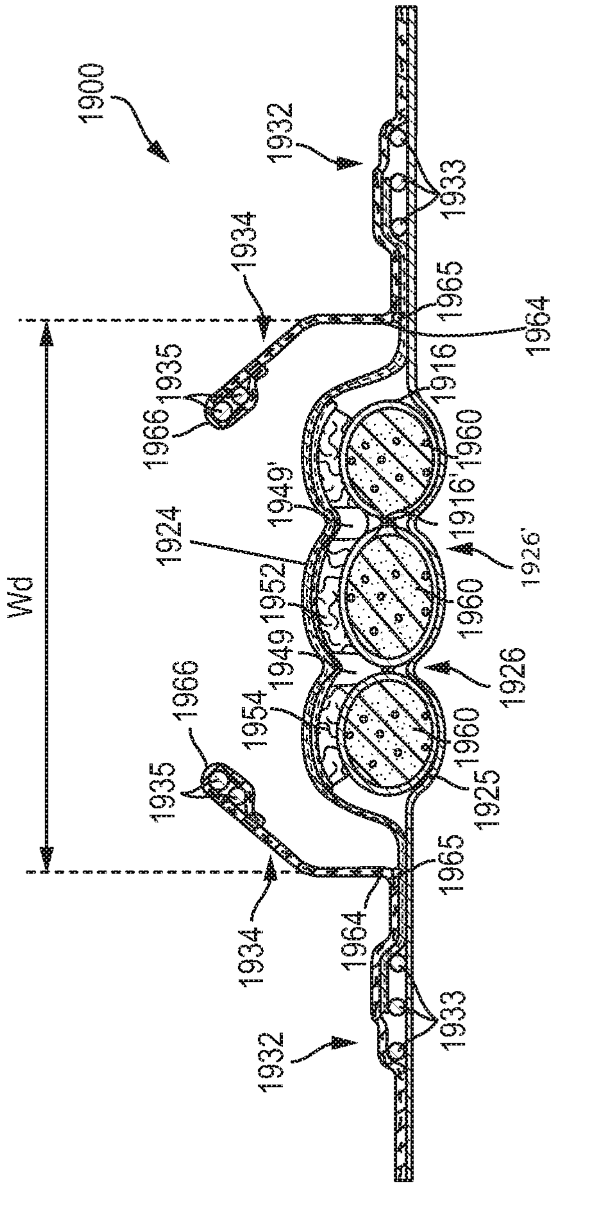
1900
1932
1933
1934
1965
1964
1935
1966
1916
1960
1949'
1916'
1924
1926'
1952
1960
Wd
1949
1926
1954
1960
1966
1935
1925
1965
1934
1964
1933
1932

Fig. 36

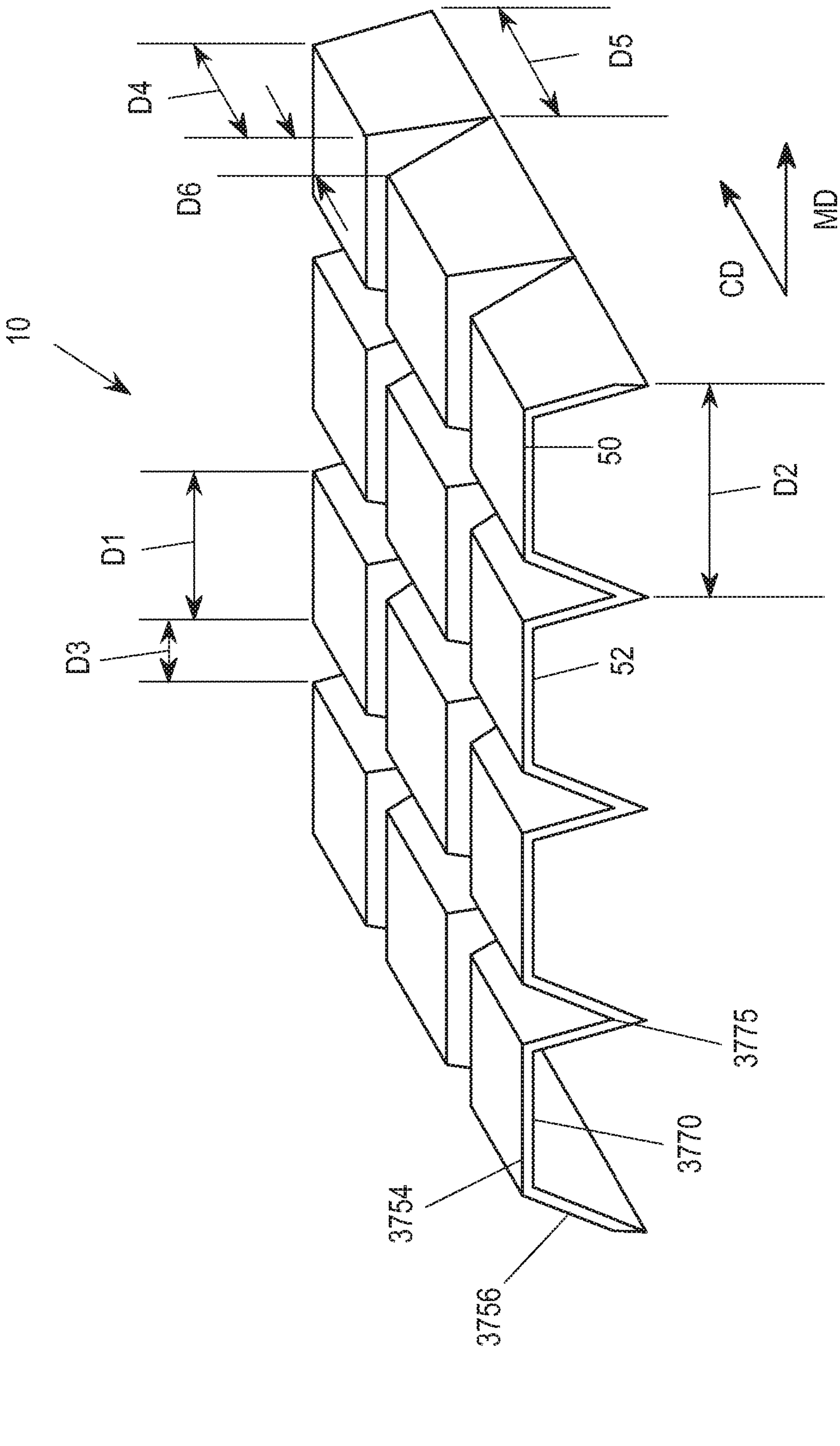


Fig. 37

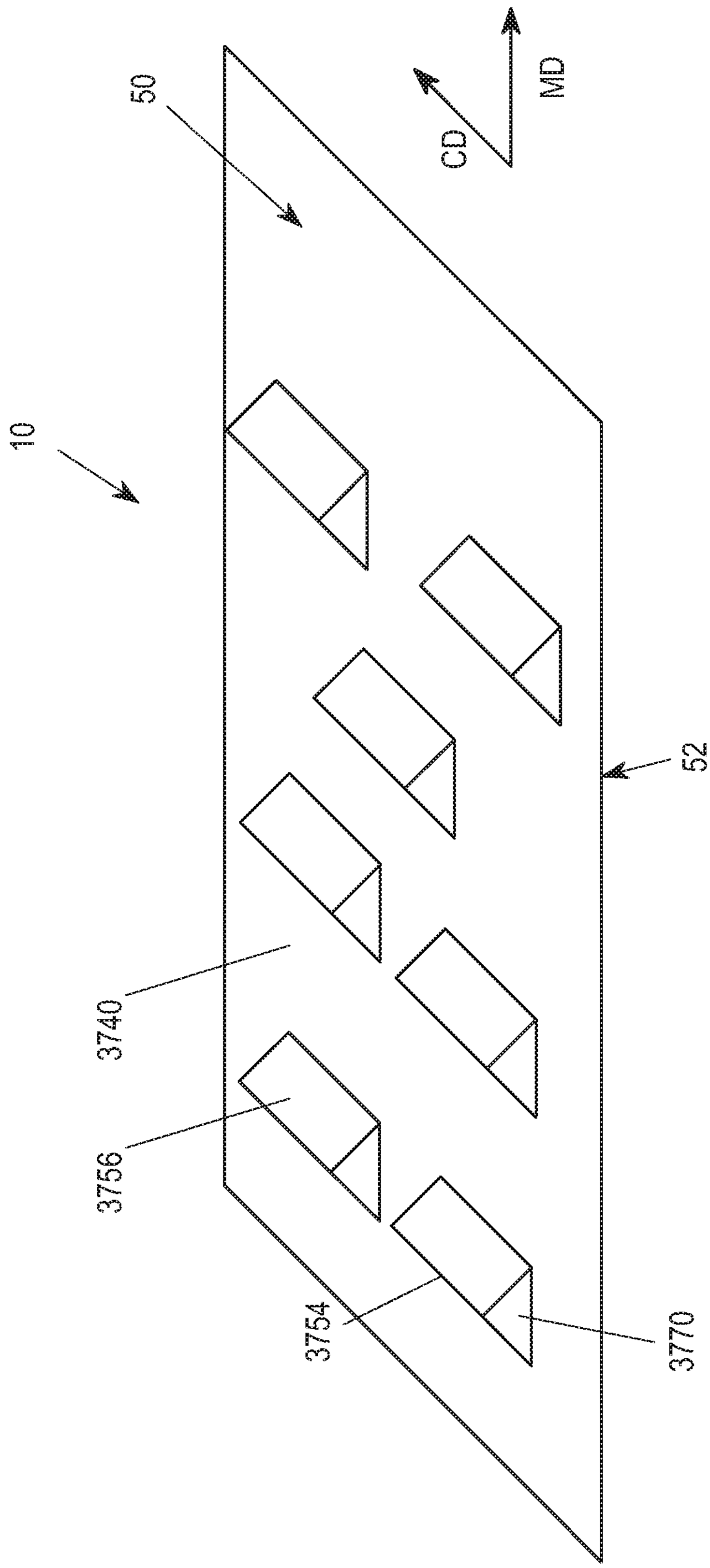


Fig. 38

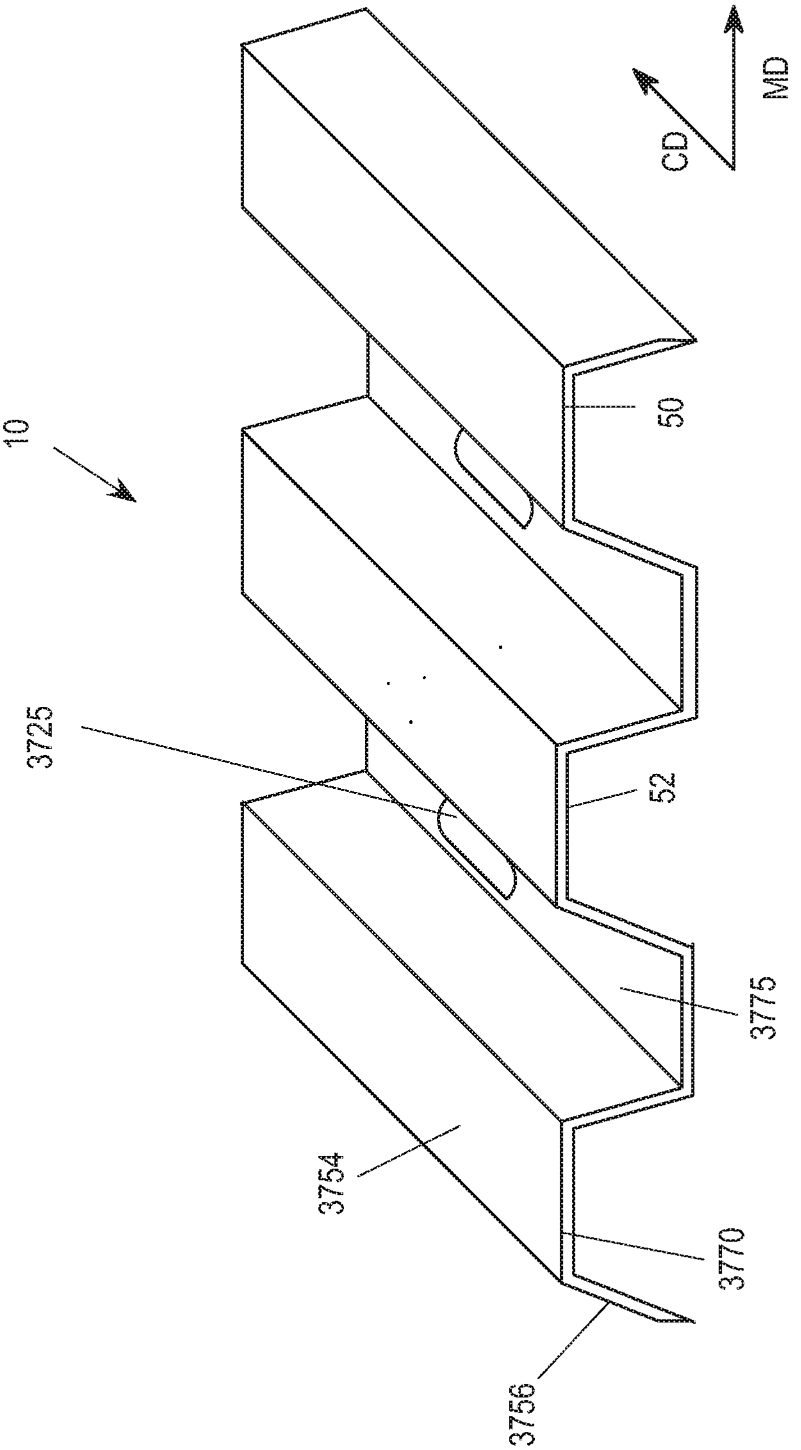


Fig. 39

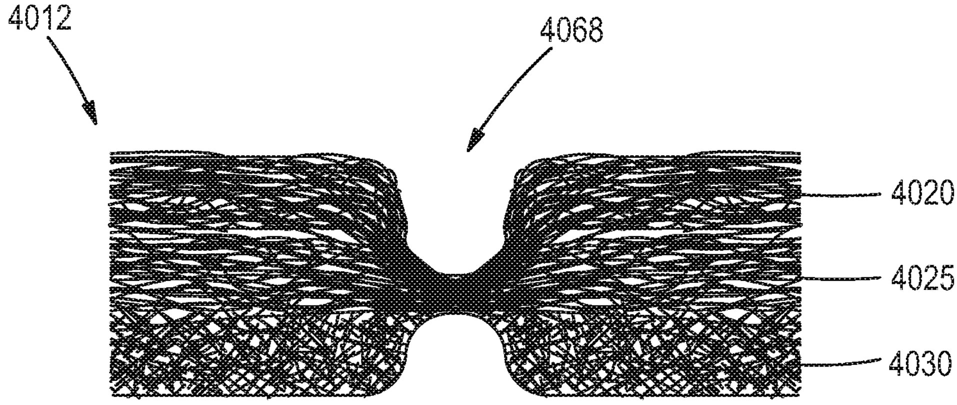


Fig. 40

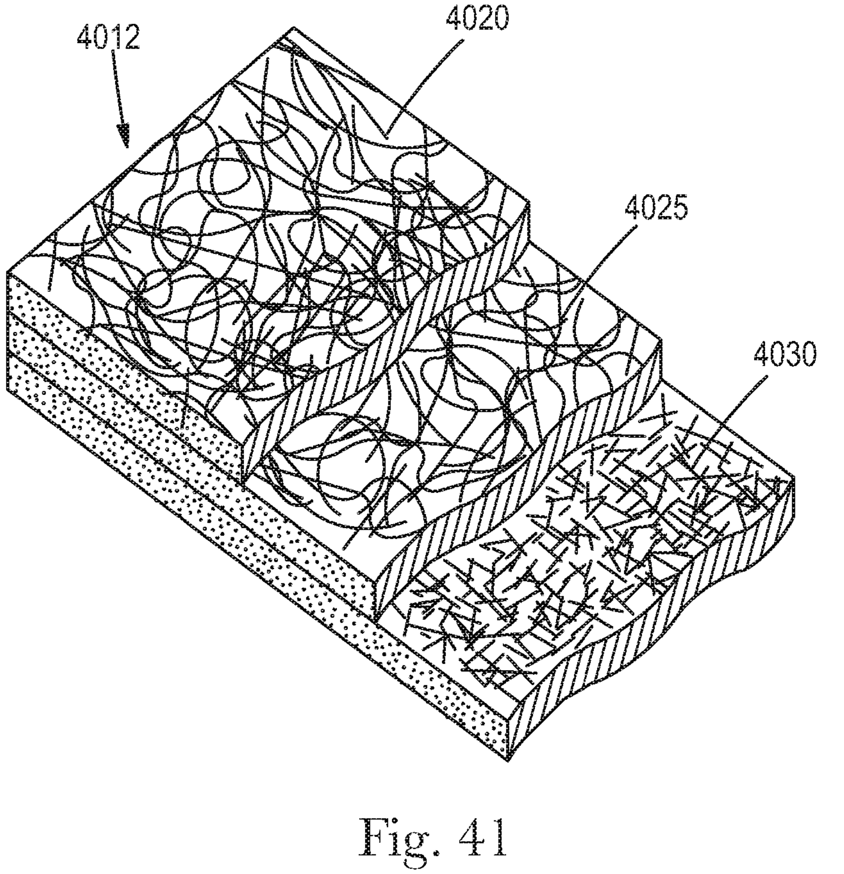


Fig. 41

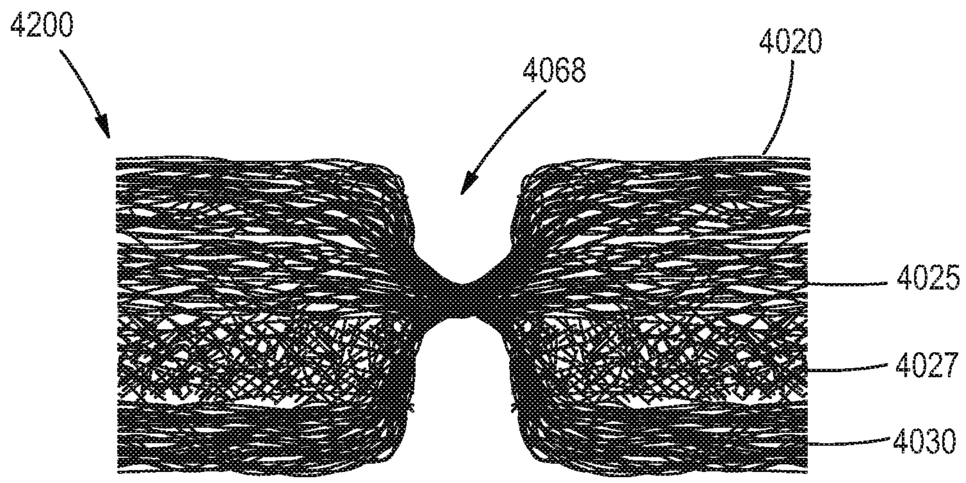


Fig. 42

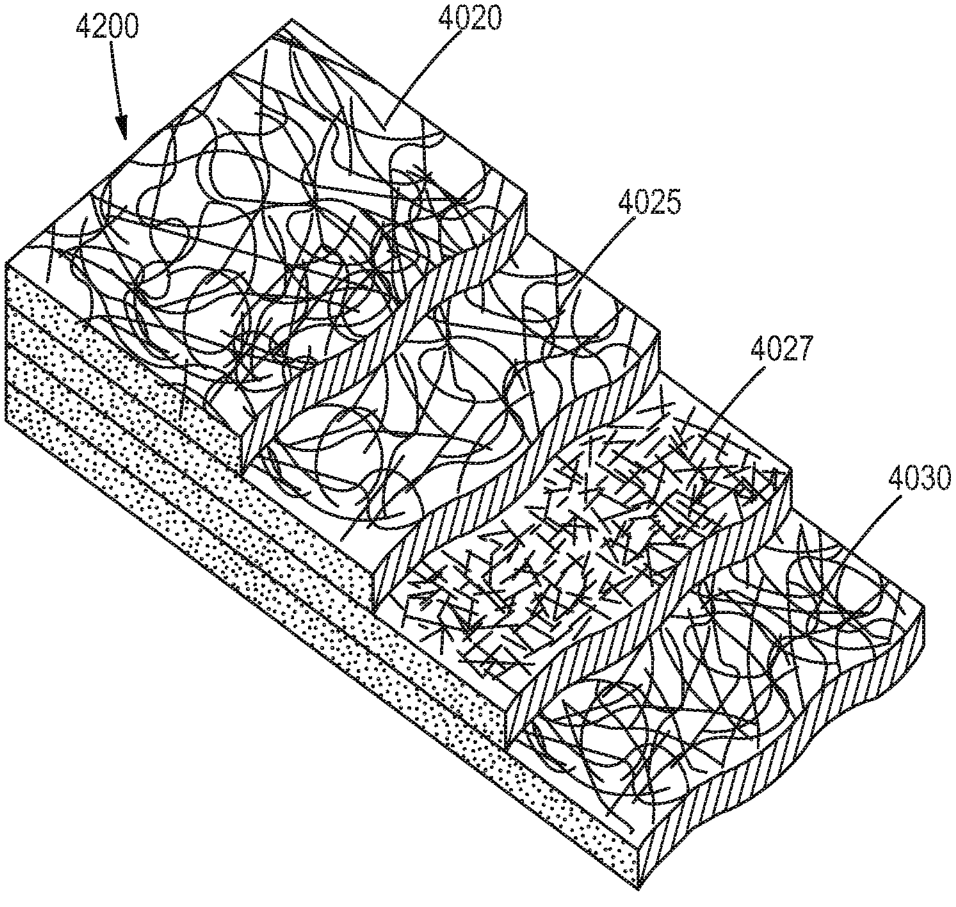


Fig. 43

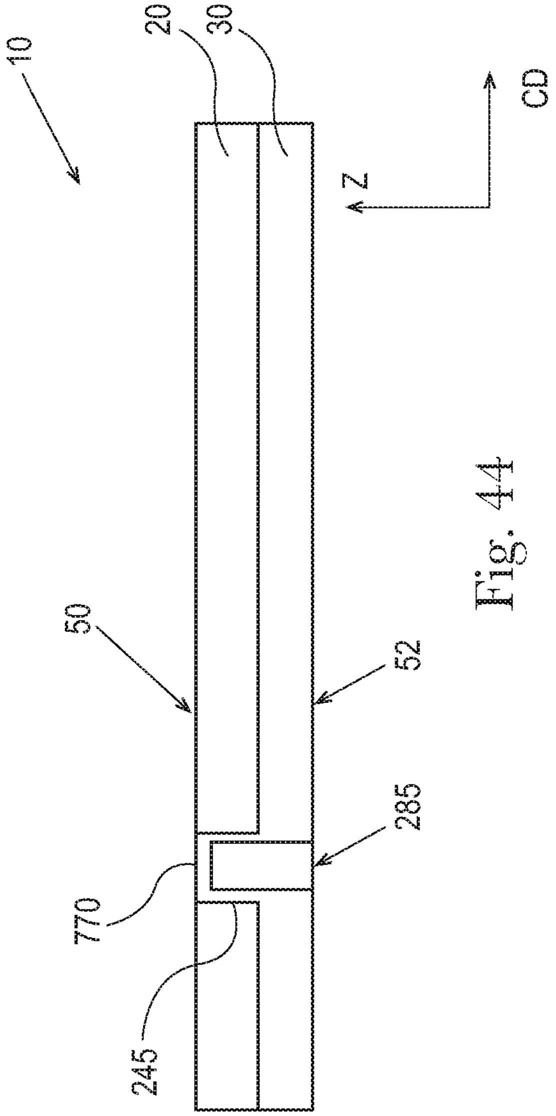


Fig. 44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6315806 B
- US 5183670 A
- US 4536361 A, Torobin
- US 6382526 B
- US 6520425 B
- US 6695992 B, Reneker
- US 8395016 B
- US 8487156 B
- US 7291300 B
- US 7989369 B
- US 7576019 B
- US 20080093778 A, Johnson
- US 7628941 B, Krause
- US 20090295020 A, Krause
- US 849630
- US 933028
- US 20120077886
- US 5969026 A
- US 4578414 A
- US 20090104831 A
- US 8226625 B
- US 8231595 B
- US 8388594 B
- US 8226626 B
- US RE36548 E
- US 5990271 A
- US 3929135 A
- US 4324246 A
- US 4342314 A
- US 4463045 A
- US 7410683 B
- US 8440286 B
- US 8697218 B
- US 14933017 B
- US 14933001 B
- US 5658639 A
- US 5628097 A
- US 5916661 A
- US 7917985 B
- US 20030021951
- US 8679391 B
- US 8158043 B
- US 20010024940
- US 20120282436 A
- US 3566726 A
- US 4634440 A
- US 4780352 A
- US 933017
- US 933001
- US 7172801 B
- US 7838099 B
- US 7754050 B
- US 7682686 B
- US 7507459 B
- US 7553532 B
- US 7718243 B
- US 7648752 B
- US 7732657 B
- US 7789994 B
- US 8728049 B
- US 8153226 B
- US 844459
- US 20160074256
- US 6458447 B
- US 7270861 B
- US 8502013 B
- US 7954213 B
- US 7625363 B
- US 8450557 B
- US 7741235 B
- US 2003018741 A
- US 20090240222 A
- US 20120045620 A
- US 20120141742 A
- US 20120196091 A
- US 20120321839 A
- US 20130022784 A
- US 20130017370 A
- US 2013013732 A
- US 20130165883 A
- US 20130158497 A
- US 20130280481 A
- US 20130184665 A
- US 20130178815 A
- US 20130236700 A
- WO 2008156075 PCT
- WO 2010055699 PCT
- WO 2011125893 PCT
- WO 2012137553 PCT
- WO 2013018846 PCT
- WO 2013047890 PCT
- WO 2013157365 PCT
- US 20090240222
- US 20040137200 A
- US 20120276331
- US 5972806 A
- US 5599335 A, Goldman

- EP 1447066 A, Busam
- WO 9511652 A, Tanzer
- US 20080312622 A1, Hundorf
- WO 2012052172 A, Van Malderen
- US 20140163500
- US 20140163506 A
- US 20140163511 A
- US 4573986 A
- US 3911173 A, Sprague, Jr.
- US 4785996 A, Ziecker
- US 4842666 A, Werenicz
- US 3860003 A
- US 5221274 A
- US 5554145 A
- US 5569234 A
- US 5580411 A
- US 6004306 A
- WO 200059430 A, Daley
- WO 9510996 A, Richards
- US 5700254 A, McDowall
- WO 02067809 A